# EUROPEAN PATENT APPLICATION

(11) **EP 3 037 409 A1**
(43) Date of publication of application: **29.06.2016**
(21) Application number: 14838209.6
(22) Date of filing: 20.08.2014
(51) Int. Cl.: C07C 249/02, C07C 251/08, C08F 10/00, C07C 251/12, C07F 15/04, C08F 110/02, C08F 110/06, C08F 110/14, C08F 210/16, C08F 4/70

(54) **BIMETALLIC CATALYST FOR OLEFIN POLYMERIZATION**

(30) Priority: 20.08.2013 CN 201310365304
(71) Applicant: Shanghai Institute of Organic Chemistry, Chinese Academy of Sciences, Shanghai 200032 (CN)
(72) Inventor: TANG, Yong, Shanghai 200032 (CN); CHEN, Zhou, Shanghai 200032 (CN); JI, Gang, Shanghai 200032 (CN); SUN, Xiuli, Shanghai 200032 (CN); XU, Chongjie, Shanghai 200032 (CN); LI, Junfang, Shanghai 200032 (CN)
(74) Representative: Lenthall, Joseph
(86) International application number: PCT/CN2014/084859
(87) International publication number: WO 2015/024517

(57) **Abstract**

The present invention relates to a bimetallic catalyst for olefin polymerization, a preparation method therefor, and the application thereof in catalysis for olefin polymerization. The catalyst is a transitional bimetallic complex of the VIII group with a type of multidentate ligand, and the structural formula thereof is shown as formula (I), wherein the definition for each radical group is described in the description. The catalyst of the present invention has a high activity and a high catalytic efficiency, suitable for olefin polymerization reactions, particularly suitable for the preparation of an olefin polymer having a bimodal distribution of molecular weights.

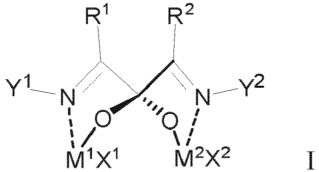

## Description

### FIELD OF THE INVENTION

The present invention relates to a catalyst or catalyst system for olefin polymerization or copolymerization, a preparation method therefor, and the application thereof in catalysis for olefin polymerization. The catalyst relates to a kind of bimetal complexes of new polydentate ligand with Group IV, V and Group VIII transition metal.

### BACKGROUND OF THE INVENTION

After Ziegler-Natta catalyst was found in 1950s, high activity MgCl₂ supported titanium catalyst has shown very good catalytic performance (K. Ziegler, et al., Angew. Chem. 1995, 67, 424; K. Ziegler et al., Angew. Chem. 1995,67, 541; N. Kashiwa et al., USP-3642746, 1968). So far in the industry, this kind of catalyst has been used in the preparation of polymers such as High density polyethylene (HDPE), linear low density polyethylene (LLDPE) and syndiotactic polypropylene (i-pp). However, such solid catalysts which possess multiple active centers cannot be well adjusted in the catalyst structure in order to control the polymer structure and properties. The discovery of the Group IV metallocene catalyst has well solved this problem. Since they have single active center, their structure can be changed according to the requirements so as to obtain a polymer of expected structure (W. Kaminsky et al., Adv. Organomet. Chem. 1980, 18, 99; W. Kaminsky et al., Angew. Chem., Int. Ed. Engl. 1980, 19, 390; H. H. Brintzinger et al., Angew. Chem. Int. Ed. Engl.1995, 34, 1143). In addition, the polyolefins are of low surface energy and chemically inert, thus limiting the material from being stuck, dyed, printed and blended (with additive or polar materials), therefore, it is needed to introduce a small amount of polar functional groups into the polymer chain of polyolefin so as to improve the polarity of polyolefin, improve compatibility of polyolefin materials with polar materials, while maintaining considerable physical and mechanical properties. Therefore, it is necessary to find some modest, high-efficient and controllable way to introduce polar functional groups into the non-polar molecule chain. But the early transition metal catalysts, including the Ziegler-Natta catalysts and metallocene catalysts which have been widely used, are very easy to be spoiled by the polar groups in the polar monomers and then inactivated, thus making the co-polymerization results of ethylene and polar monomers not satisfying.

Over the last decade, the metal complex obtained by coordinating ligands containing coordinating atoms such as N, O, and P (replacing the cyclopentadienyl) and transition materials as olefin polymerization catalyst has been vigorously researched. This type of catalysts is collectively referred to as "post-metallocene catalyst". Since 1995, there are a growing number of new excellent catalysts have been synthesized, wherein the typically transition metal complexes are the following:

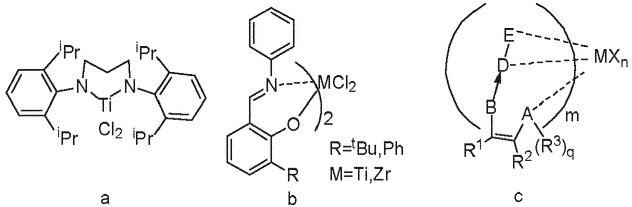

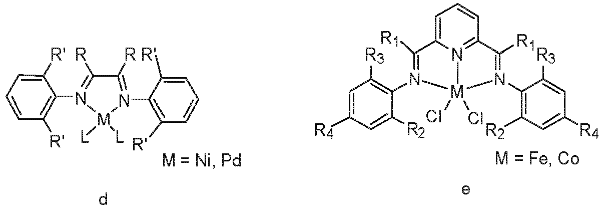

Late transition metal coordinated "post-metallocene catalysts" which have been reported in the literature can achieve copolymerization of olefin and polar monomer, thus producing functionalized polyolefin material with outstanding performance, as the central metals are of week oxygen affinity, however, they are of comparatively lower activity (e.g.: a) Johnson, L. K. et al. J. Am. Chem. Soc., 1996, 118(1), 267; b)Mecking, S. et al. J. Am. Chem. Soc., 1998, 120(5), 888; c) Chien, J. C. W. et al. Po/ym. Int., 2002, 51, 729; d) Connor, E. F. et al. J. Po/ym. Sci.: Part A: Po/ym. Chem, 2002, 40, 2842; e) Britovesk, G. J. P. et al. J. Chem. Soc., Dalton Trans., 2002,1159; f) Chen, G. et al. J. Am. Chem. Soc., 2003, 125(22), 6697).

These problems have become difficulties which troubles the catalyst technology and product applications. To achieve the application, now a step-by-step polymerization or mixed catalyst is needed so as to satisfy the mechanical properties as well as processing performance of the products. In summary, olefin polymerization catalysts of high catalytic activity as well as be able to achieve the co-polymerization of olefin and various polar monomers, and can obtain a polymer of which the molecular weight distribution can be adjusted by simple method are needed in the art.

### SUMMARY OF THE INVENTION

The object of the present invention is to provide a type of catalyst which can efficiently catalyze olefin polymerization, and the preparation method thereof.

Another object of the present invention is to provide a method to adjust the structure of polymer by the catalyst so as to provide polymers of which the molecular weight is unimodal distributed or bimodal distributed.

In the first aspect of the present invention, a compound of formula I is provided: 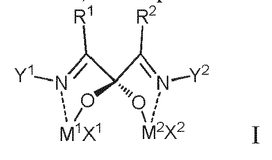 wherein:
------is a coordination bond;
each of R¹ and R² is independently selected from the group consisting of: hydrogen, substituted or unsubstituted C1-C10 alkyl, substituted or unsubstituted aryl, and substituted or unsubstituted C3-C10 cycloalkyl;
wherein the aryl refers to phenyl, naphthyl, fluorenyl or anthryl; wherein the "substituted" means one or more hydrogen atoms on the group are substituted with substituents selected from the group consisting of: OR³, SR⁴, NR⁵R⁶, PR⁷R⁸ or P(O)R⁹R¹⁰, SiR¹², C1-C4 alkyl, halogen, and phenyl;
or R¹ and R² together form substituted or unsubstituted C1-C10 alkylene;
each of M¹ and M² is independently selected from the group consisting of: Fe, Co, Ni, Pd, Pt, and the combinations thereof;
each of X¹ and X² is independently selected from the group consisting of: halogen, ClO₄, substituted or unsubstituted C1-C10 alkyl, substituted or unsubstituted C3-C10 cycloalkyl, substituted or unsubstituted phenyl, and substituted or unsubstituted benzyl; wherein the "substituted" means one or more hydrogen atoms on the group are substituted with substituents selected from the group consisting of: OR³, SR⁴, NR⁵R⁶, PR⁷R⁸ or P(O)R⁹R¹⁰, SiR¹², C₁-C₄ alkyl, halogen, phenyl, and -CF₃;
each of Y¹ and Y² is independently selected from the group consisting of: substituted or unsubstituted C1-C30 alkyl, substituted or unsubstituted aryl, and substituted or unsubstituted heteroaryl; wherein the aryl refers to phenyl, naphthyl, fluorenyl or anthryl; the heteroaryl refers to furyl, thienyl, pyrrolyl, pyridinyl or pyranyl; the "substituted" means one or more hydrogen atoms on the group are substituted with substituents selected from the group consisting of: OR³, SR⁴, NR⁵R⁶, PR⁷R⁸ or P(O)R⁹R¹⁰, SiR¹², C1-C4 alkyl, halogen, phenyl;
wherein R³, R⁴, R⁵, R⁶, R⁷, R⁸, R⁹, R¹⁰, R¹² are independently selected from the group consisting of: C1-C10 alkyl, phenyl, and C5-C16 heteroaryl; wherein 0 to 5 hydrogen groups on phenyl or heteroaryl can be substituted by the following substituent groups: halogen, -CF₃, OR¹³ C1-C4 alkyl; R¹³ is C1-C4 alkyl;
or Y¹, R¹ and the joint N=C together form substituted or unsubstituted 5-12 member heterocyclic ring which comprises 1-3 heteroatoms, preferably 5-7 member heterocyclic ring; wherein the heteroatoms are selected from the group consisting of: N, S, O and P; the "substituted" means one or more hydrogen atoms on the group are substituted with substituents selected from the group consisting of: hydrogen, substituted or unsubstituted C1-C10 alkyl, substituted or unsubstituted aryl, and substituted or unsubstituted C3-C10 cycloalkyl; the aryl refers to phenyl, naphthyl, fluorenyl or anthryl; the heteroaryl refers to furyl, thienyl, pyrrolyl, pyridinyl or pyranyl;
or Y², R² and the joint N=C together form substituted or unsubstituted 5-12 member heterocyclic ring which comprises 1-3 heteroatoms, preferably 5-7 member heterocyclic ring; wherein the heteroatoms are selected from the group consisting of: N, S, O and P; the "substituted" means one or more hydrogen atoms on the group are substituted with substituents selected from the group consisting of: hydrogen, substituted or unsubstituted C1-C10 alkyl, substituted or unsubstituted aryl, and substituted or unsubstituted C3-C10 cycloalkyl; wherein the aryl refers to phenyl, naphthyl, fluorenyl or anthryl; the heteroaryl refers to furyl, thienyl, pyrrolyl, pyridinyl or pyranyl;
and the compound of formula I is charge balanced.

In another preferred embodiment, each of R¹ and R² is independently selected from the group consisting of: substituted or unsubstituted C1-C4 alkyl, and substituted or unsubstituted C3-C6 cycloalkyl; the "substituted" means being substituted with OR³, SR⁴, NR⁵R⁶, PR⁷R⁸ or P(O)R⁹R¹⁰, SiR¹², C1-C4 alkyl, halogen, phenyl, wherein the phenyl refers to unsubstituted phenyl or phenyl of which one or more hydrogen atoms on the phenyl ring are substituted with substituents selected from the group consisting of: OR¹³, C1-C4 alkyl, halogen, and -CF₃.

In another preferred embodiment, each of R¹ and R² is independently selected from the group consisting of: substituted or unsubstituted aryl; the aryl refers to phenyl, naphthyl, fluorenyl or anthryl; the "substituted" refers to being substituted with OR¹³, C1-C4 alkyl, halogen, CF₃.

In another preferred embodiment, R¹ and R² together form substituted or unsubstituted C₁-C₄ alkylene.

In another preferred embodiment, each of X¹ and X² is independently selected from the group consisting of: halogen, and substituted or unsubstituted C1-C4 alkyl;

In another preferred embodiment, each of X¹ and X² is independently selected from the group consisting of: halogen, ClO₄, substituted or unsubstituted phenyl, and substituted or unsubstituted benzyl; wherein the "substituted" means one or more hydrogen atoms on the phenyl ring are substituted with substituents selected from the group consisting of: OR¹³, C1-C4 alkyl, halogen, CF₃, phenyl.

In another preferred embodiment, each of Y¹ and Y² is independently selected from the group consisting of: substituted or unsubstituted C1-C10 alkyl; the "substituted" means being substituted with OR³, SR⁴, NR⁵R⁶, PR⁷R⁸ or P(O)R⁹R¹⁰, SiR¹², C1-C4 alkyl, halogen.

In another preferred embodiment, each of Y¹ and Y² is independently selected from the group consisting of: substituted or unsubstituted aryl, and substituted or unsubstituted heteroaryl; the aryl refers to phenyl, naphthyl, fluorenyl or anthryl; the heteroaryl refers to furyl, thienyl, pyrrolyl, pyridinyl or pyranyl. The "substituted" means being substituted with OR³, SR⁴, NR⁵R⁶, PR⁷R⁸ or P(O)R⁹R¹⁰, SiR¹², C1-C4 alkyl, halogen, or phenyl. Wherein the phenyl refers to substituted or unsubstituted phenyl, the "substituted" means one or more hydrogen atoms on the phenyl ring are substituted with substituents selected from the group consisting of: OR¹³, C1-C4 alkyl, halogen, and CF₃; wherein R³, R⁴, R⁵, R⁶, R⁷, R⁸, R⁹, R¹⁰, R¹¹, R¹² are independently selected from the group consisting of: C1-C10 alkyl, phenyl, C5-C16 heteroaryl; wherein 0 to 5 hydrogen groups on phenyl or heteroaryl can be substituted by the following substituent groups: halogen, -CF₃, OR¹³, C1-C4 alkyl; R13 is C1-C4 alkyl;

The halogen refers to fluorine, chlorine, bromine or iodine.

In another preferred embodiment, each of X¹ and X² is independently selected from the group consisting of: chlorine, bromine.

In another preferred embodiment, the hetero atoms of Y¹ and/or Y² can optionally form coordination bond with M¹.

In another preferred embodiment, the hetero atoms of Y¹ and/or Y² can optionally form coordination bond with M².

In another preferred embodiment, the M¹ and M² are independently selected from the group consisting of: Fe, Co, Ni, Pd, and Pt.

In another preferred embodiment, the M¹ and M² are independently selected from the group consisting of: Fe(II), Co(II), Ni(II), Pd(II), and Pt(II).

In another preferred embodiment, when Y¹, R¹ and the joint N=C together form substituted or unsubstituted 5-12 member heterocyclic ring which comprises 1-3 heteroatoms, then Y², R² and the joint N=C do not together form substituted or unsubstituted 5-12 member heterocyclic ring which comprises 1-3 heteroatoms.

In another preferred embodiment, the substituted or unsubstituted 5-12 member heterocyclic ring which comprises 1-3 heteroatoms are unsubstituted or substituted by 1-3 substituents.

In the second aspect of the present invention, it provides a method for preparing compound of the first aspect of the present invention, which comprises the following steps: 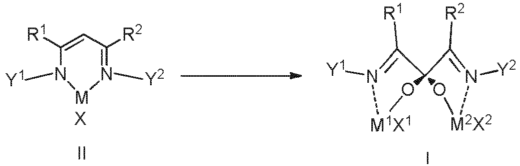 in an inert solvent, reacting compound of formula II or a complex thereof with an oxidizing reagent, thereby obtaining compound of formula I;
wherein M is selected from the group consisting of: Fe, Co, Ni, Pd, Pt, and the combinations thereof;
X is selected from the group consisting of: halogen, C1-C10 alkyl, substituted or unsubstituted C3-C10 cycloalkyl, substituted or unsubstituted phenyl, substituted or unsubstituted benzyl;
while other groups are defined as in the first aspect of the present invention.

Preferably, in the method, each of R¹ and R² is independently selected from the group consisting of: hydrogen, substituted or unsubstituted C1-C10 alkyl, substituted or unsubstituted aryl, and substituted or unsubstituted C3-C10 cycloalkyl; or R¹ and R² together form substituted or unsubstituted C1-C10 alkylene;
each of Y¹ and Y² is independently selected from the group consisting of: substituted or unsubstituted C1-C30 alkyl, substituted or unsubstituted aryl, and substituted or unsubstituted heteroaryl.

In another preferred embodiment, the hetero atoms of Y¹, Y² can optionally form coordination bond with M.

In another preferred embodiment, the complexes of compound II are the complexes formed by compound of formula II and a ligand selected from the group consisting of: triphenylphosphine, acetonitrile, tetrahydrofuran, and C4-C16 heteroarene; or a ligand which comprises groups selected from the group: OR³, SR⁴, NR⁵R⁶, PR⁷R⁸, and the combinations thereof.

In another preferred embodiment, the C4-C16 hetroarene is pyridine.

In another preferred embodiment, the complexes of compound of formula II are formed by atom M coordinating to the hetero atom of the ligands.

In another preferred embodiment, M is selected from the group consisting of: Fe, Co, Ni, Pd, and Pt; preferably selected from the group consisting of: Fe(II), Co(II), Ni(II), Pd(II), and Pt(II).

In another preferred embodiment, the oxidizing reagent is selected from the group: benzoyl peroxide, ozone, air, oxygen, hydrogen peroxide, and the combinations thereof.

In another preferred embodiment, the inert solvent is a low polar solvent.

In another preferred embodiment, the low polar solvent is selected from the group consisting of: tetrahydrofuran, diethyl ether, toluene, hexane, acetonitrile, methylene chloride, and the combinations thereof.

In another preferred embodiment, the reaction is conducted under oxygen or air.

In another preferred embodiment, the reaction is conducted at -78°C-150°C.

In another preferred embodiment, the reaction is conducted at 0°C-100°C.

In another preferred embodiment, the reaction is conducted at 0°C-75°C.

In another preferred embodiment, the reaction is conducted at 20°C-80°C.

In another preferred embodiment, the reaction time is 2h-15 days.

In another preferred embodiment, the reaction time is 3-10 days.

In another preferred embodiment, the reaction is conducted under 0.01-10MPa.

In another preferred embodiment, the reaction is conducted under 0.1-5MPa.

Or the method comprise the following steps: 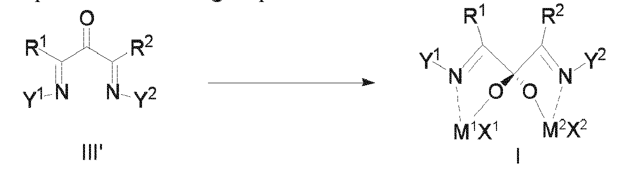
in an inert solvent, reacting a compound of formula III' with a reagent selected from the group consisting of: M¹X¹, M¹X¹·B, M²X², M²X²·B, and the combinations thereof, or the hydrates thereof, thereby obtaining the compound of formula I;
wherein the M₁X₁·B or M₂X₂·B are respectively a metallic compound formed by M₁X₁ or M₂X₂ and a coordinating solvent B; preferably, the coordinating solvent B is selected from the group consisting of: DME(dimethoxyethane), THF(tetrahydrofuran), acetonitrile, ethanol, ethylene glycol, methanol, AcAc(acetylacetonyl), DMF, and the combinations thereof;
while the other groups are defined as above.

Preferably, in the method, Y¹, R¹ and the joint N=C together form substituted or unsubstituted 5-12 member heterocyclic ring which comprises 1-3 heteroatoms, preferably 5-7 member heterocyclic ring;
and/or Y², R² and the joint N=C together form substituted or unsubstituted 5--12 member heterocyclic ring which comprises 1-3 heteroatoms, preferably 5 -7 member heterocyclic ring.

In another preferred embodiment, the step comprises: reacting a compound of formula III' firstly with M₁X₁ or M¹X¹·B, and then with M²X² or M²X²·B.

In the third aspect of the present invention, a compound of formula II, or the complexes thereof is provided: 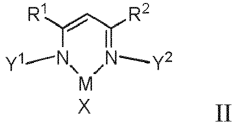 wherein the groups are defined as in the second aspect of the present invention; and when R¹ and R² are both -CH₃ or -CF₃, then Y¹ and Y² are not simultaneously 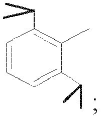 the complexes are complex formed by compound of formula II and a ligand selected from the group consisting of: triphenylphosphine, acetonitrile, tetrahydrofuran, and C4-C16 heteroarene; or a ligand which comprises a substituent selected from the group consisting of: OR³, SR⁴, NR⁵R⁶, PR⁷R⁸, and the combinations thereof.

In another preferred embodiment, the C4-C16 hetroarene is pyridine.

In another preferred embodiment, the complexes of compound of formula II are formed by atom M coordinating to the hetero atom of the ligands.

In another preferred embodiment, in the compound of formula II, the hetero atoms of Y¹ and Y² can optionally form coordination bond with M.

In the fourth aspect of the present invention, the method for preparing the compound of the third aspect of the present invention is provided, which comprises the following steps: 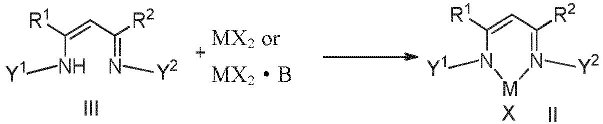
in an inert solvent, reacting compound of formula III with MX₂ or MX₂·B, thereby obtaining compound of formula II or a complex thereof;
wherein MX₂·B is a metallic compound formed by MX₂ and a coordinating solvent B;
B is a coordinating solvent, such as DME (ethylene glycol dimethyl ether), THF (tetrahydrofuran), acetonitrile, ethanol, ethylene glycol, methanol, AcAc (acetylacetonyl), DMF;
the complex of compound of formula II is a complex formed by compound of formula II and a ligand selected from the group consisting of: triphenylphosphine, acetonitrile, tetrahydrofuran, and C4-C16 heteroarene; or a ligand which comprises groups selected from: OR³, SR⁴, NR⁵R⁶, PR⁷R⁸, or the combinations thereof;
wherein the groups are defined as in the second aspect of the present invention.

In another preferred embodiment, in the compound of formula II, the hetero atoms of Y¹ and Y² can optionally form coordination bond with M.

In another preferred embodiment, the ligand is selected from the group: pyridine, triphenylphosphine, tetrahydrofuran, acetonitrile, AcAc (acetylacetonyl).

In another preferred embodiment, the coordinating solvent is selected from the group consisting of: ethanol, methanol, ethylene glycol dimethyl ether, pyridine, acetonitrile, tetrahydrofuran, ethylene glycol, methanol, and DMF.

In another preferred embodiment, the MX₂·B is NiX₂·DME

In another preferred embodiment, the inert solvent is a low polar solvent.

In another preferred embodiment, the low polar solvent is selected from the group consisting of: tetrahydrofuran, diethyl ether, toluene, hexane, acetonitrile, methylene chloride, and the combinations thereof.

In another preferred embodiment, the reaction is conducted with the existence of alkali.

In another preferred embodiment, the alkali is selected from the group consisting of: KH, NaH, BuLi, Et₃N, pyridine, and the combinations thereof.

In the fourth aspect of the present invention, a compound of formula III' is provided: 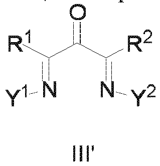 wherein Y¹, R¹ and the joint N=C together form substituted or unsubstituted 5-12 member heterocyclic ring which comprises 1-3 heteroatoms, preferably 5-7 member heterocyclic ring; and/or Y², R² and the joint N=C together form substituted or unsubstituted 5-12 member heterocyclic ring which comprises 1-3 heteroatoms, preferably 5-7 member heterocyclic ring.

In the fifth aspect of the present invention, a method for preparing compound of formula III' is provided, wherein it comprises the following steps: 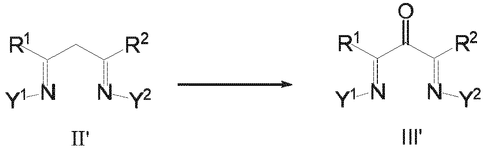 in an inert solvent, reacting a compound of formula II' with an oxidizing reagent in presence of an alkali, thereby obtaining compound of formula III'; wherein the groups are described as in the fourth aspect of the present invention.

In another preferred embodiment, the oxidizing reagent is selected from the group: oxygen, benzoyl peroxide, ozone, air, hydrogen peroxide, and the combinations thereof.

In another preferred embodiment, the alkali is selected from the group consisting of: n-butyl lithium, lithium diisopropylamide, potassium diisopropylamide, sodium bis (trimethylsilyl) amine, potassium bis (trimethylsilyl) amine, lithium bis (trimethylsilyl) amine, Et₃N, pyridine, and the combinations thereof.

In the sixth aspect of the present invention, a compound of formula II' is provided: 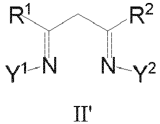 wherein Y¹, R¹ and the joint N=C together form substituted or unsubstituted 5-12 member heterocyclic ring which comprises 1-3 heteroatoms, preferably 5-7 member heterocyclic ring; and/or Y², R² and the joint N=C together form substituted or unsubstituted 5-12 member heterocyclic ring which comprises 1-3 heteroatoms, preferably 5-7 member heterocyclic ring.

In the seventh aspect of the present invention, a method for preparing compound of formula II' in the sixth aspect of the present invention is provided, which comprises the following steps:
(a1) in an inert solvent, reacting a compound of formula II'c with a compound of formula II'b, thereby obtaining a compound of formula II'a; 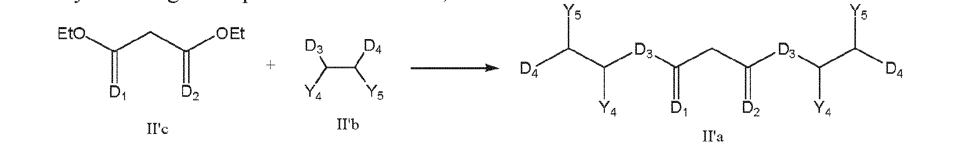
(a2) in an inert solvent, reacting a compound of formula II'a, thereby obtaining a compound of formula II'f; 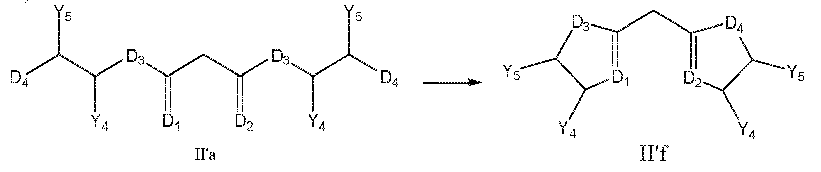
and step (a3) preparing a compound of formula II from a compound of formula II'f;
   or the method comprise the following steps:
   (b1) in an inert solvent, reacting a compound of formula II'd with a compound of formula II'b, thereby obtaining a compound of formula II'e; 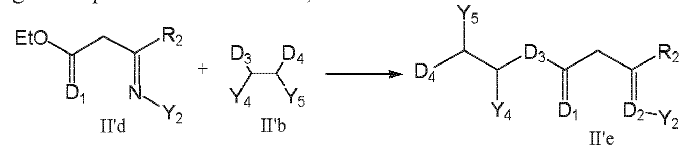
   (b2) reacting a compound of formula II'e, thereby obtaining a compound of formula II'g; 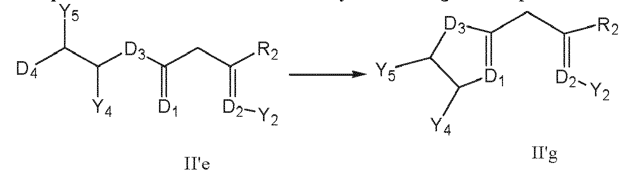
   and step (b3) preparing a compound of formula II from compound of formula II'g;
      wherein D₁, D₂, D₃, D₄ are independently selected from the group consisting of: N, S, O, P, NH, and PH;
      Y₄, Y₅ are independently selected from the group consisting of: substituted or unsubstituted C1-C30 alkyl, substituted or unsubstituted aryl, and substituted or unsubstituted heteroaryl; wherein the aryl refers to phenyl, naphthyl, fluorenyl or anthryl; the heteroaryl refers to furyl, thienyl, pyrrolyl, pyridinyl or pyranyl; the "substituted" means one or more hydrogen atoms on the group are substituted with substituents selected from the group consisting of: OR³, SR⁴, NR⁵R⁶, PR⁷R⁸ or P(O)R⁹R¹⁰, SiR¹², C1-C4 alkyl, halogen, and phenyl;
      or Y₄, Y₅ and the joint two carbon atoms together form substituted or unsubstituted phenyl; or together form substituted or unsubstituted 5-7 member hetero aromatic ring; wherein the "substituted" means one or more hydrogen atoms on the group are substituted with substituents selected from the group consisting of: hydrogen, substituted or unsubstituted C1-C10 alkyl, substituted or unsubstituted aryl, and substituted or unsubstituted C3-C10 cycloalkyl;
      while the other groups are defined as above.

In another preferred embodiment, a method for preparing the unsymmetrical di-substituted compound precursor II'-h is provided as follows: 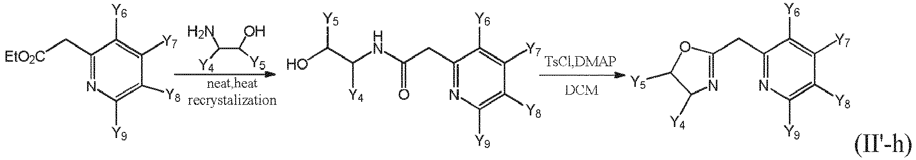 multi-substituted pyridine and the substituted β- amino alcohol are condensed to give an intermediate, while the intermediate is condensed and form a ring by using TsCl, DMAP and in methylene chloride to give the final product.

In another preferred embodiment, the preparation method of the precursor of unsymmetrical di-substituted compound II'-i is provided by the following method:

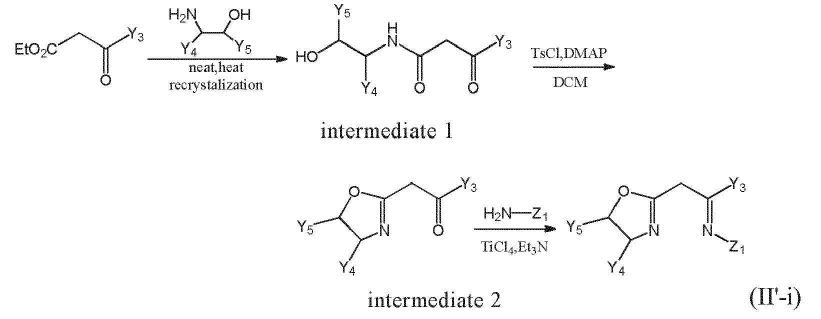

β-carboxylic ester substituted ketone and the substituted β- amino alcohol are condensed to give the intermediate 1, while the intermediate 1 is condensed and form a ring by using TsCl, DMAP and in methylene chloride so as to give the intermediate 2, and the intermediate 2 is condensed with substituted amine with the catalyze of Lewis acid so as to give the final product.

In another preferred embodiment, the step (a1) comprises: condensation reacting the diethyl malonate and the substituted β- amino alcohol, and recrystalizing to give the intermediate II'a;

In another preferred embodiment, the step (a2) comprises: the intermediate II'a is condensed and form a ring with TsCl, DMAP and in dichloromethane to provide the final product.

In another preferred embodiment, the step (b1) comprises: the multi-substituted pyridyl and the substituted β- amino alcohol are condensed to give the intermediate II'e;

In another preferred embodiment, the step (b2) comprises: the intermediate is condensed and form a ring with TsCl, DMAP and in dichloromethane to provide the final product.

In another preferred embodiment, the step (b1) comprises: the β-carboxylic ester substituted ketone and the substituted β- amino alcohol are condensed to give the intermediate II'e.

The intermediate 1 is condensed and form a ring with TsCl, DMAP and in dichloromethane to give intermediate 2, while the intermediate 2 is condensed with substituted amine with the catalyze of Lewis acid so as to give the final product.

In the eighth aspect of the present invention, a use of the compound of any of the third, fourth or sixth aspect of the present or the complexes thereof is provided, wherein, the compound is used (a) as an olefin polymerization catalyst; (b) in preparation of olefin polymerization catalyst composition; or (c) in preparation of compound of formula I in the first aspect of the present invention.

In another preferred embodiment, the catalyst composition further comprises an effective amount of cocatalyst.

In another preferred embodiment, the cocatalyst is selected from the group consisting of: alkyl aluminum compounds, alkyl aluminoxane, weakly coordinating anion, and the combinations thereof.

In another preferred embodiment, the molar ratio of the catalyst and the cocatalyst is 1:1-10000.

In another preferred embodiment, the alkyl aluminum compounds is selected from the group consisting of: AlEt₃, AlEt₂Cl, AlEtCl₂, AlMe₂Cl, AlMe₃, Al(i-Bu)₃, and the combinations thereof.

In another preferred embodiment, the alkyl aluminoxane is methylaluminoxane.

In another preferred embodiment, the weakly coordinating anion is selected from the group consisting of: [B(3,5-(CF₃)₂C₆H₃)₄]⁻, ⁻OSO₂CF₃, ((3,5-(CF₃)₂)C₆H₃)₄B⁻, and the combinations thereof.

In the ninth aspect of the present invention, a use of the compound of the first aspect of the present invention is provided, wherein the compound is used (a) as a catalyst in catalyzing olefin polymerization reaction; or (b) in the preparation of olefin polymerization catalyst composition.

In another preferred embodiment, the catalyst composition further comprises an effective amount of cocatalyst.

In another preferred embodiment, the cocatalyst is selected from the group consisting of: alkyl aluminum compounds, alkyl aluminoxane, low coordinating anion, and the combinations thereof.

In another preferred embodiment, the molar ratio of the catalyst and the cocatalyst is 1:1-10000.

In another preferred embodiment, the alkyl aluminum compounds is selected from the group consisting of: AlEt₃, AlEt₂Cl, AlEtCl₂, AlMe₂Cl, AlMe₃, Al(i-Bu)₃, and the combinations thereof.

In another preferred embodiment, the alkyl aluminoxane is methylaluminoxane.

In another preferred embodiment, the weakly coordinating anion is selected from the group consisting of: [B(3,5-(CF₃)₂C₆H₃)₄]⁻, ⁻OSO₂CF₃, ((3,5-(CF₃)₂)C₆H₃)₄B⁻, and the combinations thereof.

In the tenth aspect of the present invention, a catalyst composition for olefin polymerization is provided, wherein, it comprises a catalytically effective amount of compound of formula I in the first aspect of the present invention, compound of formula II in the third aspect of the present invention or the complexes thereof.

In another preferred embodiment, the catalyst composition further comprises an effective amount of cocatalyst.

In another preferred embodiment, the cocatalyst is selected from the group consisting of: alkyl aluminum compounds, alkyl aluminoxane, weakly coordinating anion, and the combinations thereof.

In another preferred embodiment, the molar ratio of the catalyst and the cocatalyst is 1:1-10000.

In another preferred embodiment, the alkyl aluminum compounds is selected from the group consisting of: AlEt₃, AlEt₂Cl, AlEtCl₂, AlMe₂Cl, AlMe₃, Al(i-Bu)₃, and the combinations thereof.

In another preferred embodiment, the alkyl aluminoxane is methylaluminoxane.

In another preferred embodiment, the weakly coordinating anion is selected from the group consisting of: [B(3,5-(CF₃)₂C₆H₃)₄]⁻, ⁻OSO₂CF₃, ((3,5-(CF₃)₂)C₆H₃)₄B⁻, and the combinations thereof.

In the eleventh aspect of the present invention, an olefin polymerization reaction is provided, wherein in the reaction, the compound of the first or the third aspect of the present invention, or the catalyst composition of the tenth aspect of the present invention is used for catalysis.

In another preferred embodiment, the polymerization pressure of the polymerization reaction is 0.01-10MPa, and preferably 0.1-10MPa.

In another preferred embodiment, the polymerization temperature of the polymerization reaction is -50-150°C.

In another preferred embodiment, the olefin polymerization reaction is the copolymerization reaction of ethylene and the olefins selected from the group consisting of: substituted or unsubstituted C3-C30 α-olefin, substituted or unsubstituted C6-C30 α-olefin, and substituted or unsubstituted C5-C30 cycloolefin.

In another preferred embodiment, the olefin polymerization reaction is the copolymerization reaction of ethylene and the olefins selected from the group consisting of: substituted or unsubstituted C3-C30 α-olefin, substituted or unsubstituted C6-C30 α-olefin, and substituted or unsubstituted C5-C30 cycloolefin, wherein the substituted means substituted by alkyl or polar functional group.

In another preferred embodiment, the olefin polymerization reaction is the copolymerization reaction of ethylene and the olefins selected from the group consisting of: bridged cycloolefins.

The polar functional group substituted olefins, i.e., polar monomers, include derivatives of C3-C50 1-olefin and cycloolefin comprising polar groups, etc., as shown in the following formulas: 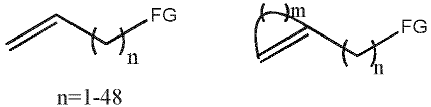 wherein in the 1-olefin derivatives, n=1-48; the cycloolefin derivatives can be single ring or bridged ring structure.

FG (polar group) refers to organic functional groups comprising oxygen, nitrogen, sulfur or selenium, which comprises carbonyl (C=O), hydroxy (OH), carboxyl (COOH), ester group (COOR³²), alkoxy (OR³³), amido (NR³⁴R³⁵), acylamino (CONR³⁴R³⁵), thioether (SR³⁶), selenide (SeR³⁷), etc.; wherein R³² or R³³ is C₁₋₁₀ hydrocarbyl; R³⁴, R³⁵, R³⁶ or R³⁷ is hydrogen or C₁₋₁₀ hydrocarbyl;

In another preferred embodiment, the polar monomer is selected from the group consisting of:

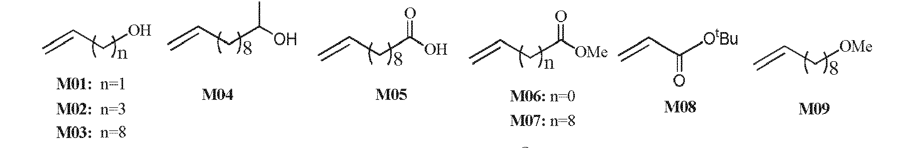

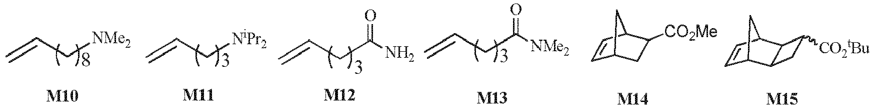

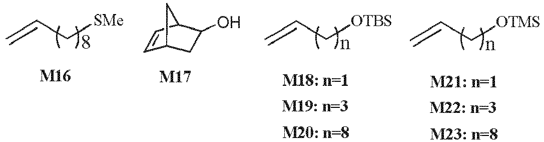

In the ninth aspect of the present invention, a method for preparing olefin polymer is provided, wherein it comprises: polymerizing the olefin in presence of a catalytic effective amount of catalyst thereby obtaining olefin polymers;
wherein the catalyst is selected from the group consisting of: compound of the first aspect of the present invention, compound of the third aspect of the present invention or the complexes thereof, the catalyst composition of the seventh aspect of the present invention, and the combinations thereof.

In another preferred embodiment, the olefin is substituted or unsubstituted C2-C30 olefin.

In another preferred embodiment, the olefin is substituted or unsubstituted C3-C30 α-olefin.

In another preferred embodiment, the olefin is substituted or unsubstituted C6-C30 α-olefin.

In another preferred embodiment, the olefin is substituted or unsubstituted C5-C30 cycloolefin.

In another preferred embodiment, the olefin is derivatives of C₃-C₅₀ 1-olefin or cycloolefin comprising polar groups.

In another preferred embodiment, the olefin is derivatives of C₃-C₃₀ 1-olefin or cycloolefin comprising polar groups.

In another preferred embodiment, the olefin polymer further comprises one or more characters selected from the group consisting of:
(a) a molecular weight is 1000 to 5,000,000 g/mol;
(b) a molecular weight distribution of the olefin polymers is unimodal, bimodal or multimodal.

In another preferred embodiment, when preparing olefin polymers of which the molar weight of the olefin polymers is bimodal, the compound of formula I is used as catalyst.

In the tenth aspect of the present invention, an olefin polymer prepared by the method of the ninth aspect of the present invention is provided.

In another preferred embodiment, the olefin polymer further comprises one or more characters selected from the group consisting of:
(a) a molar weight is 1000 to 5,000,000 g/mol;
(b) a molar weight of the olefin polymers is unimodal, bimodal or multimodal distributed.

In another preferred embodiment, the olefin polymer is the polymer of substituted or unsubstituted C3-C30 α-olefin.

In another preferred embodiment, the olefin polymer is the polymer of substituted or unsubstituted C6-C30 α-olefin.

In another preferred embodiment, the olefin polymer is the polymer of substituted or unsubstituted C5-C30 cycloolefin.

In another preferred embodiment, the olefin is derivatives of C₃-C₃₀ 1-olefin or cycloolefin comprising polar groups.

In another preferred embodiment, the polymerization reaction is the copolymerization reaction of C2-C4 olefin and polar monomer.

In another preferred embodiment, the polar monomers are C₃-C₅₀ 1-olefin derivatives comprising polar groups or cycloolefin derivatives comprising polar groups , the polar group is selected from the group consisting of: carbonyl, hydroxyl, carboxyl, ester of COOR³², alkoxy of OR³³, amido of NR³⁴R³⁵, acylamino group of CONR³⁴R³⁵, thioether of SR³⁶ and selenide of SeR³⁷; wherein R³² or R³³ are C₁₋₁₀ hydrocarbyl; R³⁴, R³⁵, R³⁶ or R³⁷ are hydrogen or C₁₋₁₀ hydrocarbyl.

In another preferred embodiment, the polar monomer is shown as the following formula A: 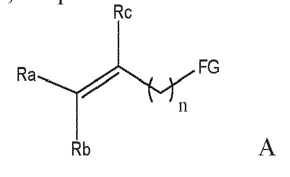
wherein n=1-48;
Ra, Rb, Rc are independently selected from the group consisting of: H, or two or three of Ra, Rb, Rc and the adjacent double bond together form unsaturated C3-C49 monocyclic, polycyclic or bridged ring structures;
FG (polar group) refers to organic functional groups comprising oxygen, nitrogen, sulfur or selenium, which comprises carbonyl (C=O), hydroxy (OH), carboxyl (COOH), ester group (COOR³²), alkoxy (OR³³), amido (NR³⁴R³⁵), acylamino (CONR³⁴R³⁵), thioether (SR³⁶), selenide (SeR³⁷), etc.; wherein R³² or R³³ is C₁₋₁₀ hydrocarbyl; R³⁴, R³⁵, R³⁶ or R³⁷ is hydrogen or C₁₋₁₀ hydrocarbyl;
or one, two or three of Ra, Rb and Rc, -(CH₂)ₙ-FG and the adjacent double bond together form unsaturated C3-C50 monocyclic, polycyclic or bridged ring structures.

In another preferred embodiment, the polar monomer is a polar monomer with protected functional groups, and the functional group protecting reagent is selected from the group consisting of: TBS, TES, TBDPS, TMS, Et₂Al, *i*-Bu₃Al, methyl aluminoxane, ethyl aluminoxane, butyl alumoinxane, modified methylaluminoxane, and the combinations thereof.

It should be understood that, in the present invention, each of the technical features specifically described above and below (such as those in the Examples) can be combined with each other, thereby constituting new or preferred technical solutions which need not be specified again herein..

### DESCRIPTION OF THE FIGURES

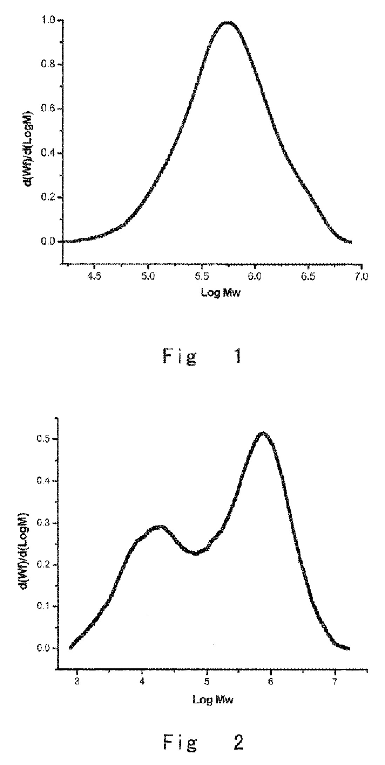

Figure 1 is the molecular weight distribution of ethylene polymers polymerized by catalyst **I-1**;
Figure 2 is the molecular weight distribution of ethylene polymers polymerized by catalyst **I-12**;
Figure 3 is the single-crystal structure of catalyst **I-1**;
Figure 4 is the single-crystal structure of catalyst **I-2**;
Figure 5 is the single-crystal structure of complex **II-4**;
Figure 6 is the single-crystal structure of complex **II-7**;
Figure 7 is the single-crystal structure of complex **II-8**;
Figure 8 is the single-crystal structure of complex **II-11**;
Figure 9 is the single-crystal structure of dipolymer (**I-11**·H₂O)₂;
Figure 10 is the single-crystal structure of dipolymer (**I-1**·(CH₃CN)₂)₂;
Figure 11 is the single-crystal structure of dipolymer (**I-2**·(THF)₂)₂.

### EMBODIMENTS FOR CARRYING OUT THE INVENTION

Through long-term and deep research, the inventor has unexpectedly discovered that a type of compounds having bimetal active center may be used as catalyst for olefin polymerization. The catalytic efficiency can be significantly improved when such catalyst is used for olefin polymerization, and the catalyst is especially suitable for synthesis of olefin polymers of which the molecular weight is bimodal distributed. The present invention is completed based on this discovery.

### Terms

As used herein, the terms "olefin polymer" and "polyolefin" are used interchangeably to refer to an olefin polymer.

As used herein, the term "catalytic system" refers to the catalyst systems formed by catalyst (I), or catalyst (I) and cocatalyst W.

As used herein, "cocatalyst" refers to substances able to catalyze polymerization reaction of olefins together with the catalyst of the present invention, and can improve the reaction. The preferred cocatalyst in the present invention can be neutral Lewis acid.

The term "weakly coordinating anion" refers to relatively non-coordinating anions of which the coordinating situation can be found in the references (W. Beck., et al., Chem. Rev., vol. 88, p 1405-1421 (1988), and S. H. Stares, Chem. Rev., vol. 93, p927-942(1993)) and the references thereof, such as (R¹⁴)₃AlX⁻, (R¹⁴)₂AlX₂⁻, (R¹⁴)AlX₃⁻, SbF₆⁻, PF₆⁻, BF₄⁻, (C₆F₅)₄B⁻, (R_{f}SO₂)₂,N⁻, CF₃SO₃⁻, ((3,5-(CF₃)₂)C₆H₃)₄B⁻.

The term "substituted" means one or more hydrogen atoms are substituted by the following substituents: C1-C30 alkyl, C3-C30 cycloalkyl, C1-C30 alkoxy, halogen, hydroxyl, C1-C10 aldehyde group, C2-C10 acyl group, C2-C10 ester group, amino, C6-C30 aryl, C5-C30 heteroaryl; OR³, SR⁴, NR⁵R⁶, PR⁷R⁸ or P(O)R⁹R¹⁰, or SeR¹¹, SiR¹².

Wherein R³, R⁴, R⁵, R⁶, R⁷, R⁸, R⁹, R¹⁰, R¹¹, R¹² are independently selected from the group consisting of: C1-C10 alkyl, phenyl, C5-C16 heteroaryl; wherein 0 to 5 hydrogen groups on phenyl or heteroaryl can be substituted by the following substituent groups: halogen, -CF₃, OR¹³, C1-C4 alkyl; R¹³ is C1-C4 alkyl;

The aryl and heteroaryl comprises unsubstituted or substituted aryl or heteroaryl, or substituted aryl or heteroaryl with 1-3 substituents. The substituents are selected from: halogen, C1-C10 alkyl, cyano, OH, nitro, C3-C10 cycloalkyl, C1-C10 alkoxy, amino.

Particularly, in the present invention, unless otherwise specified, the substituents are inert substituents, i.e. the substituents are those difficult to coordinate with metal. Preferable substituents of the present invention are selected from the group consisting of: C1-C30 alkyl, C3-C30 cycloalkyl, C6-C30 aryl, C5-C30 heteroaryl.

As used herein, the term "C1-C30 alkyl" refers to linear or branched alkyls with 1 to 30 carbon atoms, such as methyl, ethyl, propyl, isopropyl, butyl, isobutyl, sec-butyl, tert-butyl, or the like.

The term "C3-C30 cycloalkyl" refers to cycloalkyls with 3 to 30 carbon atoms, such as cyclopropyl, cyclobutyl, cyclopentyl, cycloheptyl, or the like.

The term "C6-C30 aryl" refers to aryls with 6 to 30 carbon atoms, including monocyclic or bicyclic aryl, such as phenyl, naphthyl, or the like.

The term "C5-C30 heteroaryl" refers to heteroaryls with 5 to 30 carbon atoms, such as pyrrolyl, pyridyl, furyl, or the like.

The term "C1-C10 acyl group" refers to groups which process "-CO-alkyl" structure, preferably "-CO-C1-C10 alkyl", such as formyl, acetyl, acryl, isoacryl, butyryl, isobutyryl, sec-butyryl, tert-butyryl, or the like.

The term "halogen" refers to F, Cl, Br and I.

As used herein, the term "inert functional group" refers to carbon-containing functional groups other than hydrocarbon or substituted hydrocarbon, the functional group does not substantially interference with any reaction the substituted compound may be involved, and when the inert functional group is close to a metal atom, the coordinate ability with metal is not stronger than the oxygen, nitrogen, Z groups which containing coordinating atoms, i.e. these functional groups should not replace the desired coordinating group.

In the structure of each compound, "→" means coordination bond.

The term "coordinating solvent" refers to solvents which can form coordination bond with Group VIII metal M, in the present invention, the preferred coordinating solvent are solvents selected from the group consisting of: ethanol, methanol, ethylene glycol dimethyl ethers, pyridine, acetonitrile, tetrahydrofuran, water.

The "ligands comprising a group selected from the following: OR³, SR⁴, NR⁵R⁶, PR⁷R⁸, and the combinations thereof" of the present invention can be selected from the group consisting of: methanol, ethanol, ethylamine.

As used herein, "Y¹" and "Y₁", "Y²" and "Y₂", "Y³" and "Y₃" each represents different groups.

### Compound of Formula I and the Preparation Thereof

The present invention provides a compound of formula I: 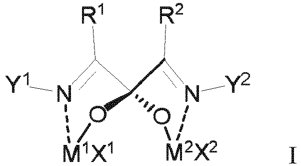 wherein:
------ is coordination bond;
each of R¹ and R² is independently selected from the group consisting of: hydrogen, substituted or unsubstituted C1-C10 alkyl, substituted or unsubstituted aryl, substituted or unsubstituted C3-C10 cycloalkyl; wherein the aryl means phenyl, naphthyl, fluorenyl or anthracenyl group;
wherein the "substituted" means one or more hydrogen atoms on the group are substituted with substituents selected from the group consisting of: OR³, SR⁴, NR⁵R⁶, PR⁷R⁸ or P(O)R⁹R¹⁰, SiR¹², C1-C4 alkyl, halogen, and phenyl;
or R¹ and R² together form substituted or unsubstituted C1-C10 alkylene;
each of M¹ and M² is independently selected from the group consisting of: Fe, Co, Ni, Pd, Pt, and the combinations thereof;
each of X¹ and X² is independently selected from the group consisting of: halogen, C1-C10 alkyl, substituted or unsubstituted C3-C10 cycloalkyl, substituted or unsubstituted phenyl, substituted or unsubstituted benzyl; wherein the "substituted" means one or more hydrogen atoms on the group are substituted with substituents selected from the group consisting of: OR³, SR⁴, NR⁵R⁶, PR⁷R⁸ or P(O)R⁹R¹⁰, SiR¹², C1-C4 alkyl, halogen, phenyl, CF₃;
each of Y¹ and Y² is independently selected from the group consisting of: substituted or unsubstituted C1-C30 alkyl, substituted or unsubstituted aryl, substituted or unsubstituted heteroaryl; wherein the aryl refers to phenyl, naphthyl, fluorenyl or anthryl; the heteroaryl refers to furyl, thienyl, pyrrolyl, pyridinyl or pyranyl; the "substituted" means one or more hydrogen atoms on the group are substituted with substituents selected from the group consisting of: OR³, SR⁴, NR⁵R⁶, PR⁷R⁸ or P(O)R⁹R¹⁰, SiR¹², C1-C4 alkyl, halogen, phenyl;
wherein R³, R⁴, R⁵, R⁶, R⁷, R⁸, R⁹, R¹⁰, R¹², R¹³ are independently selected from the group consisting of: C1-C10 alkyl, phenyl, C5-C16 heteroaryl; wherein 0 to 5 hydrogen groups on phenyl or heteroaryl can be substituted by the following substituent groups: halogen, -CF₃, OR¹³, C1-C4 alkyl; R¹³ is C1-C4 alkyl;
and the compound of formula I is charge balanced.

In another preferred embodiment, each of R¹ and R² is independently selected from the group consisting of: substituted or unsubstituted C1-C4 alkyl, substituted or unsubstituted C3-C6 cycloalkyl; the "substituted" means being substituted by OR³, SR⁴, NR⁵R⁶, PR⁷R⁸ or P(O)R⁹R¹⁰, SiR¹², C1-C4 alkyl, halogen, and phenyl. Wherein the phenyl means unsubstituted phenyl or phenyl of which one or more hydrogen atoms on the phenyl ring are substituted with substituents selected from the group consisting of: OR¹³, C1-C4 alkyl, halogen, CF₃.

In another preferred embodiment, each of R¹ and R² is independently selected from the group consisting of: substituted or unsubstituted aryl; the aryl refers to phenyl, naphthyl, fluorenyl or anthryl; the "substituted" refers to being substituted by OR¹³, C1-C4 alkyl, halogen, CF₃.

In another preferred embodiment, each of X¹ and X² is independently selected from the group consisting of: halogen, substituted or unsubstituted C₁-C₄ alkyl;

In another preferred embodiment, each of X¹ and X² is independently selected from the group consisting of: halogen, substituted or unsubstituted phenyl, substituted or unsubstituted benzyl; wherein the "substituted" means one or more hydrogen atoms on the phenyl ring are substituted with substituents selected from the group consisting of: OR¹³, C1-C4 alkyl, halogen, CF₃, phenyl.

In another preferred embodiment, each of Y¹ and Y² is independently selected from the group consisting of: substituted or unsubstituted C1-C10 alkyl; the "substituted" means being substituted by OR³, SR⁴, NR⁵R⁶, PR⁷R⁸ or P(O)R⁹R¹⁰, SiR¹², C1-C4 alkyl, halogen.

In another preferred embodiment, each of Y¹ and Y² is independently selected from the group consisting of: substituted or unsubstituted aryl, substituted or unsubstituted heteroaryl; the aryl refers to phenyl, naphthyl, fluorenyl or anthryl; the heteroaryl refers to furyl, thienyl, pyrrolyl, pyridinyl or pyranyl. The "substituted" means being substituted by OR³, SR⁴, NR⁵R⁶, PR⁷R⁸ or P(O)R⁹R¹⁰, SiR¹², C1-C4 alkyl, halogen, phenyl. Wherein the phenyl comprises unsubstituted or unsubstituted phenyl, and the "substituted" means one or more hydrogen atoms on the phenyl ring are substituted with substituents selected from the group consisting of: OR¹³, C1-C4 alkyl, halogen, CF₃;
wherein R³, R⁴, R⁵, R⁶, R⁷, R⁸, R⁹, R¹⁰, R¹¹, R¹² are independently selected from the group consisting of: C1-C10 alkyl, phenyl, C5-C16 heteroaryl; wherein 0 to 5 hydrogen groups on phenyl
or heteroaryl can be substituted by the following substituent groups: halogen, -CF₃, OR¹³, C1-C4 alkyl; R¹³ is C1-C4 alkyl;
and the halogen refers to fluorine, chlorine, bromine or iodine.

In another preferred embodiment, each of X¹ and X² is independently selected from the group consisting of: chlorine, bromine.

In another preferred embodiment, the hetero atoms of Y¹, Y² can optionally form coordination bond with M¹, M².

In another preferred embodiment, the M¹ and M² are independently selected from the group consisting of: Fe, Co, Ni, Pd, Pt, and the combinations thereof;

In another preferred embodiment, the M¹ and M² are independently selected from the group consisting of: Fe(II), Co(II), Ni(II), Pd(II), Pt(II), and the combinations thereof.

The compound of formula I can be prepared by the following method: 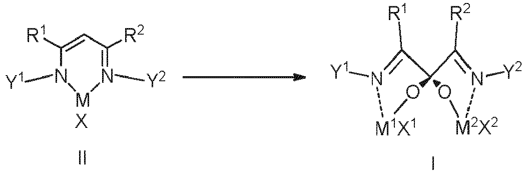 in an inert solvent, reacting compound of formula II with an oxidizing reagent, thereby obtaining compound of formula I;
wherein M is selected from the group consisting of: Fe, Co, Ni, Pd, Pt, and the combinations thereof;
X is selected from the group consisting of: halogen, C1-C10 alkyl, substituted or unsubstituted C3-C10 cycloalkyl, substituted or unsubstituted phenyl, substituted or unsubstituted benzyl;
while other groups are defined as above.

In another preferred embodiment, the hetero atoms of Y¹, Y² can optionally form coordination bond with M.

In another preferred embodiment, the complexes of compound of formula II are the complexes formed by compound of formula II and ligands selected from the group consisting of: triphenylphosphine, acetonitrile, tetrahydrofuran, C4-C16 heteroarene; or ligands which comprises groups selected from the group: OR³, SR⁴, NR⁵R⁶, PR⁷R⁸, and the combinations thereof.

In another preferred embodiment, the C4 -to C16 hetroarene is pyridine.

In another preferred embodiment, the complexes of compound of formula II are formed by M atom coordinating to the hetero atom of the ligands.

In another preferred embodiment, the M is selected from the group consisting of: Fe, Co, Ni, Pd, and Pt; preferably selected from the group consisting of: Fe(II), Co(II), Ni(II), Pd(II), and Pt(II).

In another preferred embodiment, the oxidizing reagent is selected from the group: benzoyl peroxide, air, ozone, oxygen, hydrogen peroxide, and the combinations thereof. More preferably, using oxygen or air as the oxidizing reagent can achieve very good effect.

In another preferred embodiment, the inert solvent is low polar solvent.

In another preferred embodiment, the low polar solvent is selected from the group consisting of: tetrahydrofuran, diethyl ether, toluene, hexane, acetonitrile, methylene chloride, and the combinations thereof.

In another preferred embodiment, the reaction is conducted in oxygen or air.

The reaction temperature is not specifically limited, for example, in some of the preferred embodiments, the reaction can be conducted at -78°C-150°C, 0°C-100°C, 0°C-75°C, 20°C-80°C.

The reaction time is not specifically limited, for example, in some of the preferred embodiments, the reaction time is 2h-15 days, 3-10 days.

In another preferred embodiment, the reaction is conducted under 0.01-10MPa, preferably under 0.1-5MPa.

Wherein the compound II can be prepared by any conventional methods in the art, such as be prepared by the method of the present invention, or be purchased from commercially available way.

### Compound of Formula I'

The present invention provides a compound of formula I': 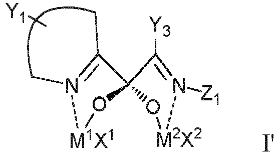 wherein:
- ----- is coordination bond;
wherein the Y₃ and Z₁ can be uncyclized so as to form the structure of compound of formula I';
Y₃ and Z₁ can also be cyclized like the ring structure of 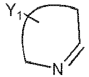 so as to form the double-cycle structure compound as 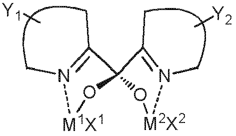 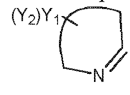 is substituted or unsubstituted five- or six-membered ring, the five- or six-membered ring contains 1-3 ofN, S, O orP atoms;
Y₁, Y₂, Y₃ are independently selected from the group consisting of: hydrogen, substituted or unsubstituted C1-C10 alkyl, substituted or unsubstituted aryl, substituted or unsubstituted C3-C10 cycloalkyl; wherein the aryl means phenyl, naphthyl, fluorenyl or anthracenyl;
wherein the "substituted" means one or more hydrogen atoms on the group are substituted with substituents selected from the group consisting of: OR³, SR⁴, NR⁵R⁶, PR⁷R⁸ or P(O)R⁹R¹⁰, SiR¹², C1-C4 alkyl, halogen, and phenyl;
Z₁ is selected from the group consisting of: substituted or unsubstituted C1-C30 alkyl, substituted or unsubstituted aryl, substituted or unsubstituted heteroaryl; wherein the aryl refers to phenyl, naphthyl, fluorenyl or anthryl; the heteroaryl refers to furyl, thienyl, pyrrolyl, pyridinyl or pyranyl; the "substituted" means one or more hydrogen atoms on the group are substituted with substituents selected from the group consisting of: OR³, SR⁴, NR⁵R⁶, PR⁷R⁸ or P(O)R⁹R¹⁰, SiR¹², C1-C4 alkyl, halogen, phenyl;
wherein the R³, R⁴, R⁵ R⁶, R⁷, R⁸, R⁸, R⁹, R¹⁰, R¹⁰, R¹² are independently selected from the group consisting of: C1-C10 alkyl, phenyl, C5-C16 heteroaryl; wherein 0 to 5 hydrogen groups on phenyl or heteroaryl can be substituted by the following substituent groups: halogen, -CF₃, OR¹³, C1-C4 alkyl; R¹³ is C1-C4 alkyl;
each of M¹ and M² is independently selected from the group consisting of: Fe, Co, Ni, Pd, Pt, and the combinations thereof;
each of X¹ and X² is independently selected from the group consisting of: halogen, ClO₄, C1-C10 alkyl, substituted or unsubstituted C3-C10 cycloalkyl, substituted or unsubstituted phenyl, substituted or unsubstituted benzyl; wherein the "substituted" means one or more hydrogen atoms on the group are substituted with substituents selected from the group consisting of: OR³, SR⁴, NR⁵R⁶, PR⁷R⁸ or P(O)R⁹R¹⁰, SiR¹², C1-C4 alkyl, halogen, phenyl, CF₃;
and the compound of formula I' is charge balanced.

In another preferred embodiment, Y₁, Y₂, Y₃ are independently selected from the group consisting of: substituted or unsubstituted C1-C4 alkyl, substituted or unsubstituted C3-C6 cycloalkyl; the "substituted" means being substituted by OR³, SR⁴, NR⁵R⁶, PR⁷R⁸ or P(O)R⁹R¹⁰, SiR¹², C1-C4 alkyl, halogen, and phenyl. Wherein the phenyl means unsubstituted phenyl or phenyl of which one or more hydrogen atoms on the phenyl ring are substituted with substituents selected from the group consisting of: OR¹³, C1-C4 alkyl, halogen, CF₃.

In another preferred embodiment, Y₁, Y₂, Y₃ are independently selected from the group consisting of: substituted or unsubstituted aryl; the aryl refers to phenyl, naphthyl, fluorenyl or anthryl; the "substituted" refers to being substituted by OR¹³, C1-C4 alkyl, halogen, CF₃.

In another preferred embodiment, Y₁, Y₂, Y₃ are independently selected from the group consisting of: halogen, substituted or unsubstituted C1-C4 alkyl;

In another preferred embodiment, each of X¹ and X² is independently selected from the group consisting of: halogen, substituted or unsubstituted C1-C4 alkyl;

In another preferred embodiment, each of X¹ and X² is independently selected from the group consisting of: halogen, substituted or unsubstituted phenyl, substituted or unsubstituted benzyl; wherein the "substituted" means one or more hydrogen atoms on the phenyl ring are substituted with substituents selected from the group consisting of: OR¹³, C1-C4 alkyl, halogen, CF₃, phenyl.

In another preferred embodiment, each of X¹ and X² is independently selected from the group consisting of: chlorine, bromine.

In another preferred embodiment, Z₁ is selected from the group consisting of: substituted or unsubstituted C1-C10 alkyl; the "substituted" means being substituted by OR³, SR⁴, NR⁵R⁶, PR⁷R⁸ or P(O)R⁹R¹⁰, SiR¹², C1-C4 alkyl, halogen.

In another preferred embodiment, Z₁ is selected from the group consisting of: substituted or unsubstituted aryl, substituted or unsubstituted heteroaryl; the aryl refers to phenyl, naphthyl, fluorenyl or anthryl; the heteroaryl refers to furyl, thienyl, pyrrolyl, pyridinyl or pyranyl. The "substituted" means being substituted by OR³, SR⁴, NR⁵R⁶, PR⁷R⁸ or P(O)R⁹R¹⁰, SiR¹², C1-C4 alkyl, halogen, phenyl. Wherein the phenyl refers to substituted or unsubstituted phenyl, the "substituted" means one or more hydrogen atoms on the phenyl ring are substituted with substituents selected from the group consisting of: OR¹³, C1-C4 alkyl, halogen, CF₃; wherein the R³, R⁴, R⁵, R⁶, R⁷, R⁸, R⁹, R¹⁰, R¹¹, R¹² are independently selected from the group consisting of: C1-C10 alkyl, phenyl, C5-C16 heteroaryl; wherein 0 to 5 hydrogen groups on phenyl or heteroaryl can be substituted by the following substituents: halogen, -CF₃, OR¹³, C1-C4 alkyl; R¹³ is C1-C4 alkyl.

The halogen refers to fluorine, chlorine, bromine or iodine.

In another preferred embodiment, the hetero atoms of Z₁ can optionally form coordination bonds with M².

In another preferred embodiment, the M¹ and M² are independently selected from the group consisting of: Fe, Co, Ni, Pd, Pt, and the combinations thereof;

In another preferred embodiment, the M¹ and M² are independently selected from the group consisting of: Fe(II), Co(II), Ni(II), Pd(II), Pt(II), and the combinations thereof.

The compound of formula I' can be prepared by the following method: 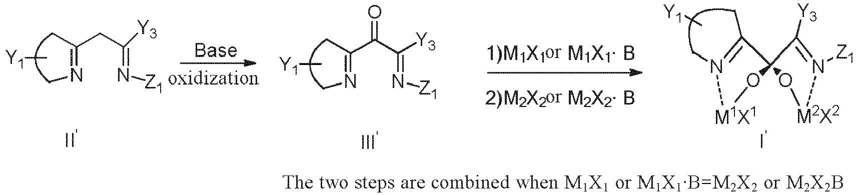 in an inert solvent, reacting compound of formula II' with oxygen in strong alkali, thereby obtaining compound of formula III'; and in an inert solvent, reacting compound of formula III' respectively with the aquo-complex of M₁X₁ (or M₁X₁·B), M₂X₂ (or M₂X₂B), thereby obtaining compound of formula I'; wherein the M₁X₁·B or M₂X₂B are respectively the metallic compound formed by M₁X₁ or M₂X₂ and coordinating solvent B.

The B is coordinating solvent, such as DME (ethylene glycol dimethyl ether), THF (tetrahydrofuran), acetonitrile, ethanol, ethylene glycol, methanol, AcAc (acetylacetonyl), DMF;
wherein M is selected from the group consisting of: Fe, Co, Ni, Pd, Pt, and the combinations thereof;

The "Base" is strong alkali selected from the group consisting of: n-butyl lithium, lithium diisopropylamide, potassium diisopropylamide, sodium bis (trimethylsilyl) amine, potassium bis (trimethylsilyl) amine, sodium bis (trimethylsilyl) amine, lithium bis (trimethylsilyl) amine, Et₃N, pyridine, and the combinations thereof.

X is selected from the group consisting of: halogen, C1-C10 alkyl, substituted or unsubstituted C3-C10 cycloalkyl, substituted or unsubstituted phenyl, and substituted or unsubstituted benzyl;
while other groups are defined as above.

In another preferred embodiment, the coordination compound of formula I' can affect with the ligands selected from the group consisting of: triphenylphosphine, acetonitrile, tetrahydrofuran, C4-C16 hetero aromatic hydrocarbon to form stable structures, which exist in solutions or in a solid state, and the presence of these small molecules do not affect the efficiency of the catalyst.

In another preferred embodiment, the C4-C16 hetroarene is pyridine.

In another preferred embodiment, the complexes of compound of formula II' are formed by atom M coordinating to the hetero atom of the ligands.

In another preferred embodiment, the M is selected from the group consisting of: Fe, Co, Ni, Pd, and Pt; preferably selected from the group consisting of: Fe(II), Co(II), Ni(II), Pd(II), and Pt(II).

In another preferred embodiment, the oxidizing reagent is selected from the group: benzoyl peroxide, air, ozone, oxygen, hydrogen peroxide, and the combinations thereof. More preferably, using oxygen or air as the oxidizing reagent can achieve very good effect.

In another preferred embodiment, the inert solvent is low polar solvent.

In another preferred embodiment, the low polar solvent is selected from the group consisting of: tetrahydrofuran, diethyl ether, toluene, hexane, acetonitrile, methylene chloride, and the combinations thereof.

In another preferred embodiment, the reaction is conducted in oxygen or air.

The reaction temperature is not specifically limited, for example, in some of the preferred embodiments, the reaction can be conducted at -78 °C to 150 °C, 0°C to 100 °C, 0°C to 75 °C, or 20°C to 80 °C.

The reaction time is not specifically limited, for example, in some of the preferred embodiments, the reaction time is 2h to 15 days, 3 to 10 days.

In another preferred embodiment, the reaction is conducted under 0.01 to 10MPa, preferably under 0.1 to 5MPa.

In another preferred embodiment, the compound I' can be mixed with cocatalysts so as to prepare catalyst compositions. In another preferred embodiment, the cocatalyst is selected from the group consisting of: alkyl aluminum compounds, alkyl aluminoxane, weakly coordinating anion, and the combinations thereof.

In another preferred embodiment, the molar ratio of the catalyst and the cocatalyst is 1:1-10000.

In another preferred embodiment, the alkyl aluminum compounds is selected from the group consisting of: AlEt₃, AlEt₂Cl, AlEtCl₂, AlMe₂Cl, AlMe₃, Al(i-Bu)₃, and the combinations thereof.

In another preferred embodiment, the alkyl alumoinxane is methylaluminoxane.

In another preferred embodiment, the weakly coordinating anion is selected from the group consisting of: [B(3,5-(CF₃)₂C₆H₃)₄]⁻, ⁻OSO₂CF₃, ((3,5-(CF₃)₂)C₆R₃)₄B⁻, and the combinations thereof.

### Compound of Formula II and the Preparation Thereof

The present invention also provide a compound of formula II, or the complexes thereof: 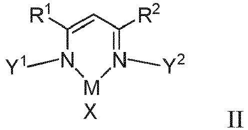
wherein M is selected from the group consisting of: Fe, Co, Ni, Pd, Pt, and the combinations thereof;
X is selected from the group consisting of: halogen, C1-C10 alkyl, substituted or unsubstituted C3-C10 cycloalkyl, substituted or unsubstituted phenyl, substituted or unsubstituted benzyl; wherein the "substituted" means one or more hydrogen atoms on the group are substituted with substituents selected from the group consisting of: OR³, SR⁴, NR⁵R⁶, PR⁷R⁸ or P(O)R⁹R¹⁰, SiR¹², C1-C4 alkyl, halogen, phenyl, -CF₃;
each of R¹ and R² is independently selected from the group consisting of: hydrogen, substituted or unsubstituted C1-C10 alkyl, substituted or unsubstituted aryl, substituted or unsubstituted C3-C10 cycloalkyl; wherein the aryl means phenyl, naphthyl, fluorenyl or anthracenyl;
wherein the "substituted" means one or more hydrogen atoms on the group are substituted with substituents selected from the group consisting of: OR³, SR⁴, NR⁵R⁶, PR⁷R⁸ or P(O)R⁹R¹⁰, SiR¹², C1-C4 alkyl, halogen, and phenyl;
or R¹ and R² together form substituted or unsubstituted C1-C10 alkylene;
each of Y¹ and Y² is independently selected from the group consisting of: substituted or unsubstituted C1-C30 alkyl, substituted or unsubstituted aryl, substituted or unsubstituted heteroaryl; wherein the aryl refers to phenyl, naphthyl, fluorenyl or anthryl; the heteroaryl refers to furyl, thienyl, pyrrolyl, pyridinyl or pyranyl; the "substituted" means one or more hydrogen atoms on the group are substituted with substituents selected from the group consisting of: OR³, SR⁴, NR⁵R⁶, PR⁷R⁸ or P(O)R⁹R¹⁰, SiR¹², C1-C4 alkyl, halogen, phenyl; and
when R¹ and R² are both -CH₃ or -CF₃, then Y¹ and Y² are not simultaneously 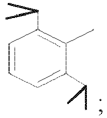 the complexes of compound of formula II are the complex formed by compound of formula II and ligands selected from the group consisting of: triphenylphosphine, acetonitrile, tetrahydrofuran, C4-C16 hetero arene; or ligands which comprises groups selected from the group: OR³, SR⁴, NR⁵R⁶, PR⁷R⁸, and the combinations thereof.

In another preferred embodiment, the C4-C16 hetroarene is pyridine.

In the above formulas, the definition of R³, R⁴, R⁵, R⁶, R⁷, R⁸, R⁹, R¹⁰, R¹², R¹³ are described as above.

In another preferred embodiment, in the compound of formula II, the hetero atoms of Y¹, Y² can optionally form coordination bond with M.

In another preferred embodiment, the M can optionally form coordination compounds with free ligands in the reaction system selected from the group consisting of: pyridine, triphenylphosphine, tetrahydrofuran, acetonitrile.

The compound II can be prepared by the following routes: 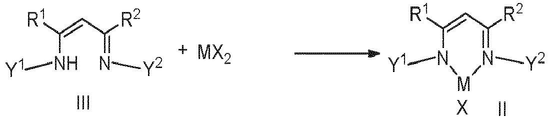
in an inert solvent, reacting compound of formula III with MX₂ or MX₂-B, thereby obtaining compound of formula II or a complex thereof;
wherein MX₂·B is a metallic compound formed by MX₂ and coordinating solvent B;
the B is coordinating solvent;
the complex of compound of formula II is the complex formed by compound of formula II and ligands selected from the group consisting of: triphenylphosphine, acetonitrile, tetrahydrofuran, C4-C16 hetero arene; or ligands which comprises groups selected from the group: OR³, SR⁴, NR⁵R⁶, PR⁷R⁸, and the combinations thereof;

In the above formulas, other groups are defined as above.

In another preferred embodiment, the ligand is selected from the group consisting of: pyridine, triphenylphosphine, tetrahydrofuran, acetonitrile.

In another preferred embodiment, the coordinating solvent is selected from the group consisting of: ethanol, methanol, ethylene glycol dimethyl ether, pyridine, acetonitrile, tetrahydrofuran.

In another preferred embodiment, the MX₂·B is NiX₂·DME

In another preferred embodiment, the inert solvent is low polar solvent.

In another preferred embodiment, the low polar solvent is selected from the group consisting of: tetrahydrofuran, diethyl ether, toluene, hexane, acetonitrile, methylene chloride, and the combinations thereof.

In another preferred embodiment, the reaction is conducted with the existence of alkali.

In another preferred embodiment, the alkali is selected from the group consisting of: KH, NaH, BuLi, Et₃N, pyridine, and the combinations thereof.

### Compound of Formula II' and the Preparation Thereof

The present invention further provides a compound of formula II': 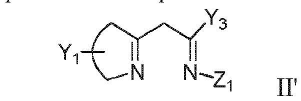
Y¹, Y², Y³ are independently selected from the group consisting of: hydrogen, substituted or unsubstituted C1-C10 alkyl, substituted or unsubstituted aryl, substituted or unsubstituted C3-C10 cycloalkyl; wherein the aryl means phenyl, naphthyl, fluorenyl or anthracenyl;
Y₃, Z₁ can also be cyclizated so as to form bi-cyclic structure compound 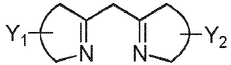
Wherein the Y² is selected from the group consisting of: hydrogen, substituted or unsubstituted C1-C10 alkyl, substituted or unsubstituted aryl, substituted or unsubstituted C3-C10 cycloalkyl; wherein the aryl means phenyl, naphthyl, fluorenyl or anthracenyl;
wherein the "substituted" means one or more hydrogen atoms on the group are substituted with substituents selected from the group consisting of: OR³, SR⁴, NR⁵R⁶, PR⁷R⁸ or P(O)R⁹R¹⁰, SiR¹², C1-C4 alkyl, halogen, and phenyl;

In the compound of formula II', 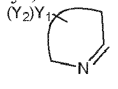 is substituted or unsubstituted five- or six-membered ring, while the five- or six- membered ring comprises 1-3 atoms of N, S, O or Pin which at least one atom is N;

In another preferred embodiment, the substituted or unsubstituted five- or six- membered ring is selected from the group consisting of: 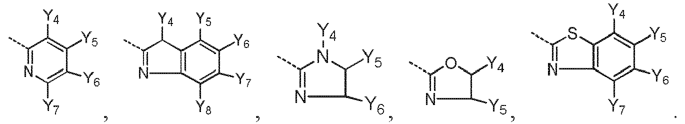
Y₄, Y₅, Y₆, Y₇, Y₈ are independently selected from the group consisting of: hydrogen, substituted or unsubstituted C1-C10 alkyl, substituted or unsubstituted aryl, substituted or unsubstituted C3-C10 cycloalkyl;
wherein the aryl refers to phenyl, naphthyl, fluorenyl or anthryl; while the "substituted" means one or more hydrogen atoms on the group are substituted with substituents selected from the group consisting of: OR³ SR⁴, NR⁵R⁶, PR⁷R⁸ or P(O)R⁹R¹⁰, SiR¹², C1-C4 alkyl, halogen, phenyl;
Z₁ is selected from the group consisting of: substituted or unsubstituted C1-C30 alkyl, substituted or unsubstituted aryl, substituted or unsubstituted heteroaryl; wherein the aryl refers to phenyl, naphthyl, fluorenyl or anthryl; the heteroaryl refers to furyl, thienyl, pyrrolyl, pyridinyl or pyranyl; the "substituted" means one or more hydrogen atoms on the group are substituted with substituents selected from the group consisting of: OR³, SR⁴, NR⁵R⁶, PR⁷R⁸ or P(O)R⁹R¹⁰, SiR¹², C1-C4 alkyl, halogen, phenyl;
wherein the R³ R⁴, R⁵, R⁶, R⁷, R⁸, R⁹, R¹⁰, R¹⁰, R¹² are independently selected from the group consisting of: C1-C10 alkyl, phenyl, C5-C16 heteroaryl; wherein 0 to 5 hydrogen groups on phenyl or heteroaryl can be substituted by the following substituent groups: halogen, -CF₃, OR¹³, C1-C4 alkyl; R¹³ is C1-C4 alkyl.

In another preferred embodiment, Y₄, Y₅, Y₆, Y₇, Y₈ are independently selected from the group consisting of: substituted or unsubstituted C1-C4 alkyl, substituted or unsubstituted C3-C6 cycloalkyl; the "substituted" means being substituted by OR³, SR⁴, NR⁵R⁶, PR⁷R⁸ or P(O)R⁹R¹⁰, SiR¹², C1-C4 alkyl, halogen, and phenyl. Wherein the phenyl means unsubstituted phenyl or phenyl of which one or more hydrogen atoms on the phenyl ring are substituted with substituents selected from the group consisting of: OR¹³, C1-C4 alkyl, halogen, -CF₃.

In another preferred embodiment, Y₄, Y₅, Y₆, Y₇, Y₈ are independently selected from the group consisting of: substituted or unsubstituted aryl; the aryl refers to phenyl, naphthyl, fluorenyl or anthryl; the "substituted" refers to being substituted by OR¹³, C1-C4 alkyl, halogen, CF₃.

In another preferred embodiment, any two of Y₄, Y₅, Y₆, Y₇, Y₈ together form substituted or unsubstituted C1-C4 alkylene.

In another preferred embodiment, Y₄, Y₅, Y₆, Y₇, Y₈ are independently selected from the group consisting of: halogen, substituted or unsubstituted C1-C4 alkyl;

In another preferred embodiment, Z₁ is selected from the group consisting of: substituted or unsubstituted C1-C10 alkyl; the "substituted" means being substituted by OR³, SR⁴, NR⁵R⁶, PR⁷R⁸ orP(O)R⁹R¹⁰, SiR¹², C1-C4 alkyl, halogen.

In another preferred embodiment, Z₁ is selected from the group consisting of: substituted or unsubstituted aryl, substituted or unsubstituted heteroaryl; the aryl refers to phenyl, naphthyl, fluorenyl or anthryl; the heteroaryl refers to furyl, thienyl, pyrrolyl, pyridinyl or pyranyl, and the "substituted" means being substituted by OR³, SR⁴, NR⁵R⁶, PR⁷R⁸ or P(O)R⁹R¹⁰, SiR¹², C1-C4 alkyl, halogen, phenyl. Wherein the phenyl refers to substituted or unsubstituted phenyl, the "substituted" means one or more hydrogen atoms on the phenyl ring are substituted with substituents selected from the group consisting of: OR¹³, C1-C4 alkyl, halogen, CF₃; wherein the R³, R⁴, R⁵, R⁶, R⁷, R⁸, R⁹, R¹⁰, R¹¹, R¹² are independently selected from the group consisting of: C1-C10 alkyl, phenyl, C5-C16 heteroaryl; wherein 0 to 5 hydrogen groups on phenyl or heteroaryl can be substituted by the following substituents: halogen, -CF₃, OR¹³, C1-C4 alkyl; R¹³ is C1-C4 alkyl;
and the halogen refers to fluorine, chlorine, bromine or iodine.

In another preferred embodiment, the hetero atoms of Z₁ can optionally form coordination bonds with M²_{.}

The compound II' of the present invention can be prepared by any method, e.g. by the following method:

The preparation method 1 of symmetric or asymmetric substituted compound precursor is as follows:

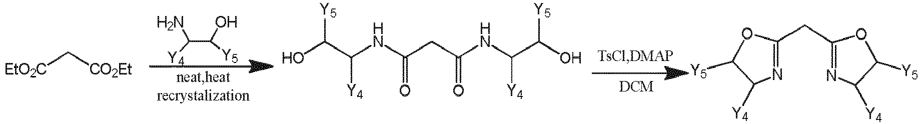

The diethyl malonate and the substituted P- amino alcohol are condensed, and an intermediate is obtained by recrystalization. The intermediate is condensed to form a ring by using TsCl, DMAP and using methylene chloride as solvent to give the final product.

The preparation method 2 of asymmetric substituted compound precursor is as follows:

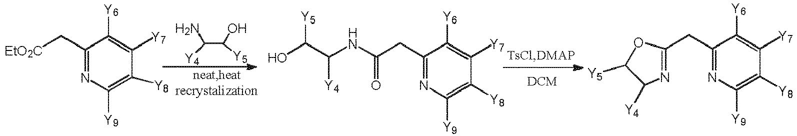

The multi-substituted pyridine and the substituted β- amino alcohol were condensed to give intermediate, while the intermediate is condensed to form a ring by using TsCl, DMAP and using methylene chloride as solvent to give the final product.

The preparation method 3 of symmetric or asymmetric substituted compound precursor II' is as follows:

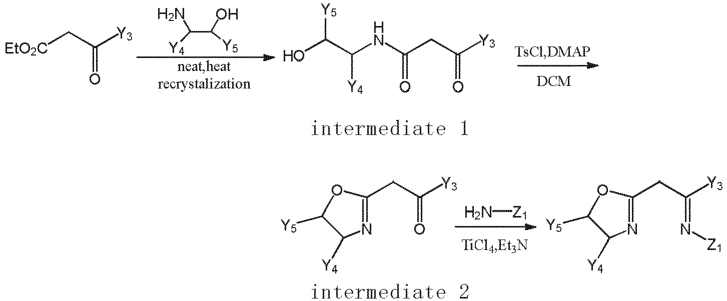

The β-carboxylic ester substituted ketone and the substituted β- amino alcohol were condensed to give the intermediate 1, while the intermediate 1 is condensed to form a ring by using TsCI, DMAP and using methylene chloride as solvent to give the intermediate 2, and the intermediate 2 is condensed with substituted amine catalyzed by Lewis acid to give the final product.

### Compound of Formula III' and the Preparation Thereof

The present invention also provides a compound of formula III' and the preparation method thereof: 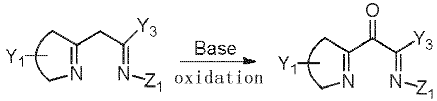 in an inert solvent, reacting the compound of formula II' with a strong base at room temperature, after 2h, then oxidizing reagent is pumped in. The mixture is stirred at room temperature for 24h, and then is post-treated to afford the compound of formula III;
wherein each group is defined as in the first aspect of the present invention.

In another preferred embodiment, the inert solvent is low polar solvent. In another preferred embodiment, the low polar solvent is selected from the group consisting of: tetrahydrofuran, diethyl ether, toluene, hexane, acetonitrile, methylene chloride, and the combinations thereof.

In another preferred embodiment, the reaction is conducted with the existence of alkali. In another preferred embodiment, the alkali is selected from the group consisting of: KH, NaH, BuLi, Et₃N, pyridine, and the combinations thereof.

### Cocatalyst

As used herein, "cocatalyst" refers to substances able to catalyze polymerization reaction of olefins together with the catalyst of the present invention, and can improve the reaction.

In the present invention, preferable cocatalyst can be neutral Lewis acid, which can cramp out X⁻ from metal M so as to form (WX)⁻; when (WX)⁻ is weakly coordinating anions, W can transfer alkyl or hydrogen onto the metal M, such as alkyl aluminum compounds, especially methylaluminoxane (which is abbreviated to MAO in the examples) or modified methylaluminoxane (which is abbreviated to MMAO in the examples); or a combination of two compounds can also be used, in which one can transfer alkyl or hydrogen onto the metal M, such as alkyl aluminum compounds, especially AlEt₃, AlMe₃, Al(i-Bu)₃, while the other can cramp out X- from metal M so as to form weakly coordinating anion, such as sodium salt or silver salt: Na[B(3,5-(CF₃)₂C₆H₃)₄], AgOSO₂CF₃, alkyl aluminum compounds or borane B(C₆F₅)₃.

### Olefin Polymerization Catalyst

The present invention has provided a type of catalyst which can catalyze the polymerization reaction of olefin. The catalyst comprises effective amount of compound of formula I, or the coordination compound thereof; and /or the compound of formula II, or the coordination compound thereof. Or the catalyst is compound of formula I, or the coordination compound thereof, and /or the compound of formula II, or the coordination compound thereof.

In another preferred embodiment, the catalyst can be mixed with effective amount of cocatalyst to prepare catalyst composition for catalysis.

In another preferred embodiment, the cocatalyst is selected from the group consisting of: alkyl aluminum compounds, alkyl aluminoxane, weakly coordinating anion, and the combinations thereof. In another preferred embodiment, the molar ratio of the catalyst and the cocatalyst is 1:1-10000.

In another preferred embodiment, the alkyl aluminum compounds is selected from the group consisting of: AlEt₃, AlEt₂Cl, AlEtCl₂, AlMe₂Cl, AlMe₃, Al(i-Bu)₃, and the combinations thereof.

In another preferred embodiment, the alkyl aluminoxane is methylaluminoxane.

In another preferred embodiment, the weakly coordinating anion is selected from the group consisting of: [B(3,5-(CF₃)₂C₆H₃)₄]⁻, ⁻OSO₂CF₃, ((3,5-(CF₃)₂)C₆H₃)₄B⁻, and the combinations thereof.

### Olefin Polymerization Reaction

The present invention has also provided an olefin polymerization reaction which comprises: using the catalyst of the present invention alone or in combination with cocatalyst in the polymerization of olefin. Wherein the type of olefin is not limited, which can be olefins commonly used in polymerization such as ethylene, propylene, vinyl chloride, or can be sterically hindered olefins, such as higher 1-olefins (e.g. olefin with carbon atom number> 8), etc..

In a preferred embodiment of the present invention, the polymerization reaction comprises: catalyzing the reaction with a compound of formula I or formula II of the present invention, or the catalyst composition of the present invention; or conduct the olefin polymerization reaction with the existence of catalytically effective amount of the catalysts selected from the group consisting of, thereby obtaining an olefin polymer: compound of formula I or compound of formula II, the catalyst compositions of the present invention, and the combinations thereof.

In the present invention, the polymerization reaction can be any olefin polymerization reaction, such as the homopolymerization of single olefin, copolymerization between different olefins, the copolymerization reaction of olefin and polar monomers. In a preferred embodiment of the present invention, the olefin polymerization reaction is the copolymerization reaction between ethylene and the olefins selected from the group consisting of: substituted or unsubstituted C2-C30 α-olefin, substituted or unsubstituted C6-C30 α-olefin, substituted or unsubstituted C5-C30 cycloolefin, etc.; the "substituted" means that there are one or more C1-C4 substituents or polar groups on the carbon chain, while the polar groups refers to derivatives of C2-C50 1-olefin which comprises polar group, or derivatives of C3-C50 cycloolefin which comprises polar group, as shown in the following formula: 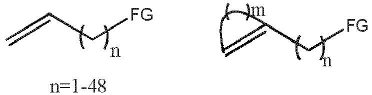 wherein in the 1-olefin derivatives, n=1-48; the cycloolefin derivatives can be monocyclic or a structure with bridged ring.

FG (polar group) refers to organic functional groups comprising oxygen, nitrogen, sulfur or selenium, which comprises carbonyl (C=O), hydroxy (OH), carboxyl (COOH), ester group (COOR³²), alkoxy (OR³³), amido (NR³⁴R³⁵), acylamino (CONR³⁴R³⁵), thioether (SR³⁶), selenide (SeR³⁷), etc.; wherein R³² or R³³ is C₁₋₁₀ hydrocarbyl; R³⁴, R³⁵, R³⁶ or R³⁷ is hydrogen or C₁₋₁₀ hydrocarbyl;

In another preferred embodiment, the polar monomer is of the following formula A: 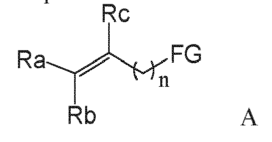
wherein n=0-48;
Ra, Rb, Rc are independently selected from the group consisting of: H, or two or three of Ra, Rb, Rc and the adjacent double bond together form unsaturated C3-C50 monocyclic, polycyclic or bridged ring structures;
FG (polar group) refers to organic functional groups comprising oxygen, nitrogen, sulfur or selenium, which comprises carbonyl (C=O), hydroxy (OH), carboxyl (COOH), ester group (COOR³²), alkoxy (OR³³), amido (NR³⁴R³⁵ ), acylamino (CONR³⁴R³⁵), thioether (SR³⁶), selenide (SeR³⁷), etc.; wherein R³² or R³³ is C₁₋₁₀ hydrocarbyl; R³⁴, R³⁵, R³⁶ or R³⁷ is hydrogen or C₁₋₁₀ hydrocarbyl;
or one, two or three of Ra, Rb and Rc, -(CH₂)ₙ-FG and the adjacent double bond together form unsaturated C3-C50 monocyclic, polycyclic or bridged ring structures.

The polar monomers involved in the preferred embodiments of the present invention mainly comprise (but are not limited to) the following compounds: 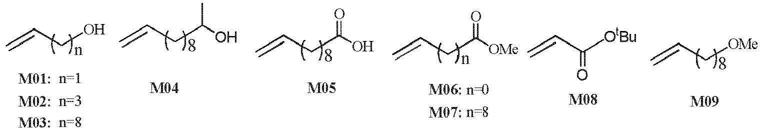 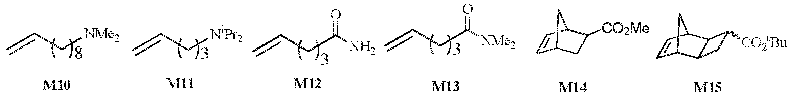 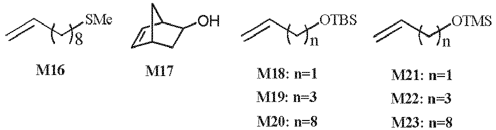 wherein the α- olefin is C3-C30 olefins of which the double bond is at the end of the molecular chain, preferably C3-C18 olefins, such as propylene, 1-butene, 1-pentene, 1-hexene, 1-octene, 4-methyl-1-pentene, 1-decene, 1-dodecene, 1-octadecene and mixtures thereof.

The cycloolefin means that olefins having a cyclic carbon structure, and double bond are located inside the ring, such as cyclopentene, cyclohexene, norbornene and cyclopentadiene dienes, and mixtures thereof.

The polar monomer refers to organic functional groups comprising oxygen, nitrogen, sulfur or selenium, which comprises carbonyl (C=O), hydroxy (OH), carboxyl (COOH), ester group (COOR³²), alkoxy (OR³³), amido (NR³⁴R³⁵), acylamino (CONR³⁴R³⁵), thioether (SR³⁶), selenide (SeR³⁷), etc.; wherein R³² or R³³ is C₁₋₁₀ hydrocarbyl; R³⁴,R³⁵,R³⁶ or R³⁷ is hydrogen or C₁₋₁₀ hydrocarbyl;

In another preferred embodiment, the olefin is substituted or unsubstituted C3-C30 olefin.

In another preferred embodiment, the olefin is substituted or unsubstituted C6-C30 α-olefin.

In another preferred embodiment, the olefin is substituted or unsubstituted C5-C30 cycloolefin.

In another preferred embodiment, the olefin is derivatives of C₃-C₃₀ 1-olefin or cycloolefin comprising polar groups.

The polymerization comprises homopolymerization and copolymerization, including the oligomerization of above-described monomers.

The polymerization process of the present invention is not particularly limited, while any of conventional techniques in the art may be employed, such as slurry polymerization, loop polymerization, gas phase polymerization, or other forms of conventional polymerization processes.

The polymerization reaction is generally carried out in an inert solvent, such as hydrocarbons, cyclic hydrocarbons or aromatic hydrocarbons. In order to be benefit for reactor operation and the product of polymerization, the type of the inert solvent is not particularly limited, and hydrocarbons with less than 12 carbon can be used, e.g. the solvent including (but not limited to) the group consisting of: propane, isobutane, n-pentane, 2-methylbutane, hexane, toluene, chlorobenzene, and the combinations thereof.

The polymerization temperature is not particularly limited, which can be chosen according to the type of the polymerization reaction, equipments, and the target product. Preferably, the temperature of polymerization reaction can be maintained at -50 to 150 °C, in order to achieve good catalytic activity and productivity, it can be maintained at 0 to 120 °C.

The pressure of the polymerization reaction is not particularly limited, which varies from 0.01 to 10MPa according to the type of polymerization reaction, the equipments, and the target product, preferably from 0.1-10MPa. In a preferred embodiment of the present invention, operating within 0.1 to 3MPa would provide better reactor operation parameters as well as polymer.

In the catalytic reaction of the present invention, the cocatalyst can also be added to further improve the efficiency of the catalyst. The cocatalyst may be an alkyl aluminum compound, alkyl aluminoxane or weakly coordinating anion.

In another preferred embodiment, the alkyl aluminum compound is preferably selected from AlEt₃, AlMe₃, Et₂AlCl or Al(i-Bu)₃.

In another preferred embodiment, the alkyl aluminoxane is selected from methylaluminoxane (MAO), MMAO (modified methylaluminoxane), wherein MAO or MMAO are selected from products of AkzoNobel.

In another preferred embodiment, the weakly coordinating anion is preferably [B(3,5-(CF₃)₂C₆H₃)₄]⁻, ⁻OSO₂CF₃ or ((3,5-(CF₃)₂)C₆H₃)₄B⁻.

The catalyst and cocatalyst can be added into the system in any order to enable the polymerization. The ratio between catalyst and co-catalyst used in polymerization is variable. Usually, the molar ratio between catalyst and co-catalyst is 1: 1-10000, and generally is 1: 10-2000 so as to maintain the catalytic activity, polymer properties and production costs in a comparatively good range.

### Olefin Polymer

The present invention also provides an olefin polymer, and the olefin polymer is prepared by the olefin polymerization method according to the present invention.

In another preferred embodiment, the olefin polymer further comprises one or more characters selected from the group consisting of:
(a) the molecular weight is 1000 to 5,000,000 g/mol;
(b) the molecular weight distribution of the olefin polymers is unimodal, bimodal or multimodal.

Preferably, the olefin polymer is the homopolymerization or copolymerization product of the following monomers: substituted or unsubstituted C2-C30 olefin, substituted or unsubstituted C6-C30 α-olefin, substituted or unsubstituted C5-C30 cycloolefin, substituted or unsubstituted derivatives of C₃-C₃₀ 1-olefin or cycloolefin comprising polar group, and the combinations thereof.

In another preferred embodiment, the compound of formula I is used when preparing olefin polymers of which the molecular weight distribution is bimodal.

**The main advantages of the present invention are:**
(1) The present invention has provided a novel olefin polymerization catalyst, and the preparation method thereof. The olefin polymerization catalyst comprises multiple active centres, which can be used in the preparation of olefins of which the molecular weight distribution is bimodal or multimodal.
(2) The catalyst of the present invention can, when alone or together with the co-catalyst, be used in the catalysis of homopolymerization or copolymerization of ethylene, α-olefins, cycloolefins, etc., including oligomerization, in particular in the catalysis of the polymerization of C2-C4 olefins and polar monomers. Compared to the average level of the prior art, the catalytic efficiency can be increased by 2-8 times, and the catalytic activity is high.

The present invention will be further illustrated below with reference to the specific examples. It should be understood that these examples are only to illustrate the invention but not to limit the scope of the invention. The experimental methods with no specific conditions described in the following examples are generally performed under the conventional conditions, or according to the manufacture's instructions. Unless indicated otherwise, parts and percentage are calculated by weight.

### 8 General Methods

All operations (including reactions, preparations and storages) were carried out under a dry inert atmosphere, using standard Schlenk Line.

Polymer molecular weight *M_{w}* was determined by Waters Alliance GPC2000 in 1,2,4 trichlorobenzene (at 1.0mL/min) at 135°C, and polystyrene was used as standard sample.

Polymer melting point was measured by Perkin-Elmer Pyris 1 differential scanning calorimetry (DSC), while the heating rate was 5°C/min and the temperature range was 20 to 200°C, and the melting point data of the second heating curve was recorded.

¹H-NMR spectrum was determined by Varian XL-400 MHz Nuclear Magnetic Resonance at 110°C with D₄-o-dichlorobenzene as solvent. Co-monomer insertion rate was calculated according to the information provided on the ¹H-NMR spectrum.

### Treating method 1 of polar monomer (hereinafter referred to as T1)

In a high temperature baked, evacuated and argon replaced Shlenck bottle, 96 mmol of alkyl aluminum (AlEt₃, AlMe₃, Al¹Bu₃ or AlEt₂Cl, all of them are from Akzo Chemical company) or alkyl aluminoxane (MMAO (1.9M, in toluene) or MAO (1.6M, in toluene), all of them are from Akzo Chemical company) and 20mL of toluene were added. At -78°C, 80 mmol of polar monomer was added into the solution dropwise and slowly. After reacting for 2h, it was heated to room temperature and reacted for 12h. The mixture was refilled with toluene to form a 1.0 mol/L solution of polar monomer in toluene and reserved. The polar monomer **M03** used in the following examples was treated by the above method.

### Treating method 2 of polar monomer (hereinafter referred to as T2)

During the ethylene / polar monomer copolymerization, before adding the main catalyst, the co-catalyst and the polar monomer were *in site* reacted in the polymerization reactor for 3h.

### Method of Copolymerization Reaction

At room temperature, 50mL of toluene, untreated polar monomers or polar monomers treated with the above methods, MMAO (Akzo Chemical company, 1.88mol/L, in heptane, herein after referred as MMAO)) were added into a dry two necked 250mL flask which was high temperature baked, evacuated and ethylene replaced, warmed to polymerization temperature and stirred for 10min. The pressure was maintained at 0.1 MPa, while the main catalyst was added to start the copolymerization reaction. After 10 mins, the ethylene inlet valve was shut off and the mixture was cooled to room temperature, the copolymer was participated in 300 mL 5% (by volume) hydrochloric acidified solvents, which was stirred for 2 hours and filtered. Then it was washed with pure solvent (100mLx3), then filtered and vacuum dried at 50°C to constant weight, and copolymer was obtained.

In examples 1-6, the precursor compounds III were compounds of the following structure:

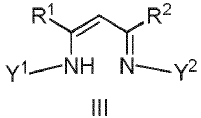

The preparation methods of precursor compounds III were as follows:

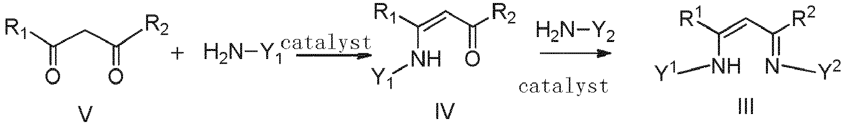

Compound V and H²N-Y₁ were dehydrate condensated under a catalyst to form the intermediate IV, while the intermediate and H₂N-Y₂ were condensated under a catalyst to form compound III.

Wherein the compound V, H₂N-Y₁, H₂N-Y₂ were commercial available.

The catalyst was protonic acid such as p-toluenesulfonic acid, hydrochloric acid, sulfuric acid, acetic acid, etc. or Lewis acid such as titanium tetrachloride, aluminum chloride, ferric chloride, zinc chloride, boron trifluoride, etc..

In the formulas, the definition of the groups are described as above, moreover, in each of the reactions, the definition of R¹, R², Y¹ Y² are the same as the corresponding groups of the products.

### Example 1 the synthesis of compound III

### The preparation method of compound III-1 was as follows:

12.5g (125mmol) of acetylacetone and 44.3g (250mmol) of 2,6-diisopropyl aniline were treated with 10.6mL (125mmol) of concentrated hydrochloric acid to obtain 44.2g of compound III-1 with a yield of 81%.

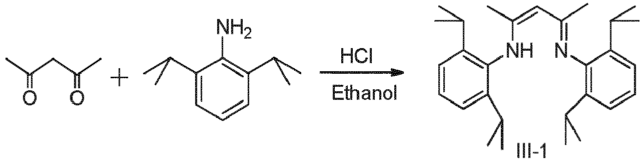

### The preparation method of compound III-11 was as follows:

19.4g (193.8mmol) of acetyl acetone and 33.8g (190.87mmol) of 2,6-diisopropyl aniline were solved in 100mL of toluene, and added with 1.0g (2%) of p-toluenesulfonic acid monohydrate, which was heated to reflux to separate water until there was no water to be separated. The solvent was removed under reduced pressure, the residue was distilled under reduced pressure so as to obtain the yellow oil **IV11 41.3g** with a yield of 83%. Then 3.4g (38.7mmol) of N,N-dimethylethylenediamine and 8g (30.7mmol) of **IV11** were treated with 0.15g of p-toluenesulfonic acid monohydrate to smoothly obtain 8.2g of compound III-11 with a yield of 81%.

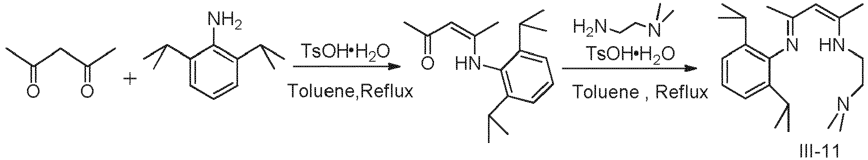

### The synthesis of compound III-2-III-22

Other compounds, **III-2** (yield 64%), **III-3** (yield 75%), **III-4** (yield 51%), **III-5** (yield 61%), **III-13** (yield 87%), **III-15** (yield 82%), **III-20** (yield 67%), **III-22**(yield 68%), were obtained by the same experimental method for preparing III-1 as described above.

Other compounds, **III-6** (yield 41%), **III-7** (yield 45%), **III-8** (yield 56%), **III-9** (yield 61%), **III-10** (yield 51%), **III-12** (yield 75%), **III-14** (yield 52%), **III-16** (yield 43%), **III-17**(yield 63%), **III-18**(yield 55%), **III-19**(yield 61%), **III-21**(yield 57%), were obtained by the same experimental method for preparing III-11 as described above.

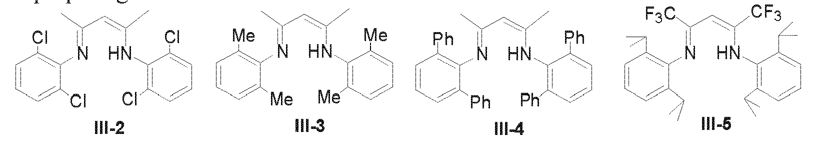

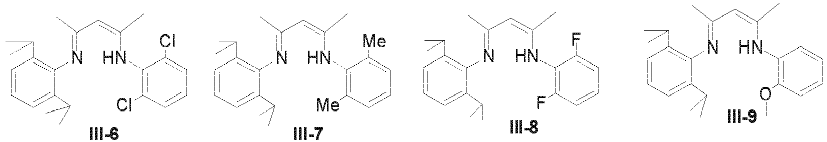

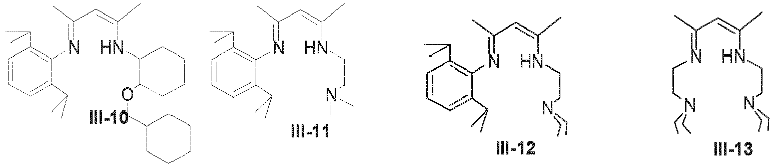

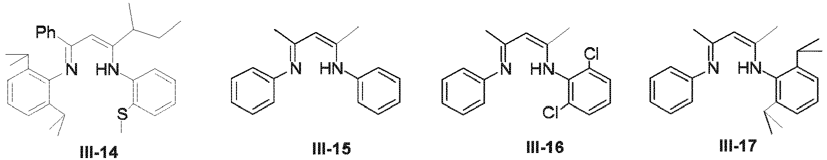

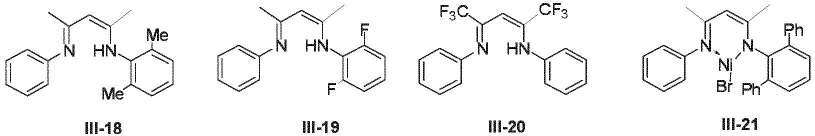

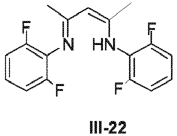

NMR data of some of the compounds (¹H-NMR) were as follows:
**III-1**
   ¹H-NMR (300 MHz, CDCl₃): δ (ppm) = 12.13 (s, 1H, N*H*), 7.12 (s, 6H, Ar-*H*),4.87 (s, 1H, C=C*H*), 3.11 (m, 4H, *J*= 6.9 Hz, C*H*(CH3)2), 1.72 (s, 6H, C*H*3), 1.17 (dd, 24H, *J* = 6.6 Hz, *J* = 6.9 Hz, CH(C*H*3)2).
**III-4**
   ¹H-NMR (300 MHz, CDCl₃): δ(ppm) = 7.53-7.43 (m, 8H, Ar-*H*), 7.38-7.33 (m, 2H, Ar-*H*), 7.14-7.12 (m, 2H, Ar-*H*),6.91-6.66 (m, 1H, Ar-*H*), 3.85 (s, 2H, N*H*2).
**III-5**
   ¹H-NNM (300 MHz, CDCl₃): δ (ppm) = 11.22 (s, 1H, N*H*), 7.25 (m, 6H, Ar-*H*),
   5.81 (s, 1H, C=C*H*), 2.95 (m, 4H, *J* = 6.9 Hz, C*H*(CH3)2), 1.17 (dd, 24H, *J* = 6.6 Hz, *J*=6.9 Hz, CH(C*H*3)2).
**III-6**
   ¹H-NMR (300 MHz, CDCl₃): δ (ppm) = 12.06 (s, 1H, N*H*), 7.28-7.22 (m, 3H, Ar-*H*), 7.15 (d, *J* = 7.5 Hz, 2H, Ar-*H*), 6.85 (t, *J* = 7.8 Hz, 1H, Ar-*H*), 4.95 (s, 1H, C*H*CN), 3.22-3.13 (m, 2H, C*H*(CH₃)₂), 1.82 (s, 3H, CNC*H*₃), 1.72 (s, 3H, CNC*H*₃), 1.24 (d, *J* = 6.9 Hz, 6H, CH(C*H*₃)₂), 1.13 (d, *J* = 6.9 Hz, 6H, CH(C*H*₃)₂).
**III-11**
   ¹H-NMR (300 MHz, CDCl₃): δ (ppm) = 10.81 (s, 1H, N*H*), 7.11-6.99 (m, 3H, Ar-*H*), 4.64 (s, 1H, C*H*CN), 3.35-3.29 (m, 2H, NC*H*₂CH₂N(CH₃)₂), 2.92-2.82 (m, 2H, C*H*(CH₃)₂), 2.39 (t, *J* = 6.6 Hz, 2H, NCH₂C*H*₂N(CH₃)₂), 2.20 (s, 6H, N(C*H*₃)₂), 2.01 (s, 3H, CNC*H*₃), 1.61 (s, 3H, CNC*H*₃), 1.16 (d, *J* = 7.2 Hz, 6H, CH(C*H*₃)₂), 1.11 (d, *J* = 6.6 Hz, 6H, CH(C*H*₃)₂).
**III-12**
   ¹H-NMR (300 MHz, CDCl₃): δ (ppm) = 10.75 (s, 1H, N*H*), 7.11-7.00 (m, 3H, Ar-*H*), 4.63 (s, 1H, C*H*CN), 3.33-3.26 (m, 2H, NC*H*₂CH₂N(CH₃)₂), 2.92-2.82 (m, 2H, C*H*(CH₃)₂), 2.55-2.46 (m, 6H, NCH₂C*H*₂N(CH₃)₂*,* N(C*H*₂CH₃)₂ ), 2.02 (s, 3H, CNC*H*₃), 1.61 (s, 3H, CNC*H*₃)*,* 1.15 (d, *J =* 7.2 Hz, 6H, CH(C*H*₃)₂), 1.11 (d, *J* = 6.9 Hz, 6H, CH(C*H*₃)₂), 0.95 (t, *J* = 6.9 Hz, 6H, N(C*H*₂CH₃)₂).

### Example 2 the synthesis of metal compound II-1

3.6g (8.6 mmol) of compound precursor was added into the 100mL reaction bottle, dissolved with 20 mL of tetrahydrofuran, and added dropwise into 0.41g (10.3mmol) of potassium hydride in tetrahydrofuran (10 mL) at -30 °C. After reacting for 24h at room temperature, the potassium salt was added dropwise into NiBr₂DME in tetrahydrofuran (10 mL) and reacted for 24h at room temperature. The reaction was stopped and the solvent was removed under reduced pressure. To the residue 20 mL of toluene was added, heated to dissolve, and filtered. The mother solution was cooled at -30 °C so as to obtain the product. After it was dried, a dark green crystal **II-1** was obtained, 3.2g (yield 66%). 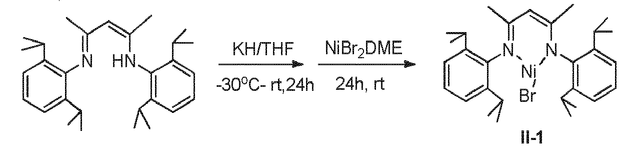
Elemental analysis: found (calculated): C: 62.67(62.62); H: 7.30(7.43); N: 5.06(5.04).

### Example 3 the synthesis of metal compounds II-2-II-22

Other compounds, **II-2**(yield 65%), **II-3**(yield 71%), **II-4**(yield 59%), **II-5**(yield 63%), **II-6**(yield 74%), **II-7**(yield 76%), **II-8**(yield 64%), **II-9**(yield 67%), **II-10**(yield 55%), **II-11**(yield 55%), **II-12**(yield 75%), **II-13**(yield 72%), **II-14**(yield 58%), **II-15**(yield 66%), **II-16**(yield 61%), **II-17**(yield 72%), **II-18**(yield 54%), **II-19**(yield 67%), **II-20**(yield 77%), **II-21**(yield 68%), **II-22**(yield 71%), were obtained by the same experimental method as example 2.

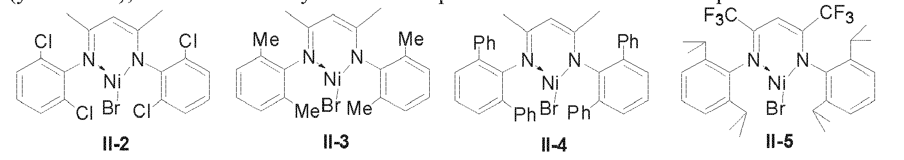

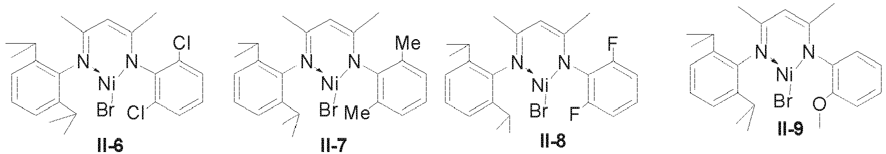

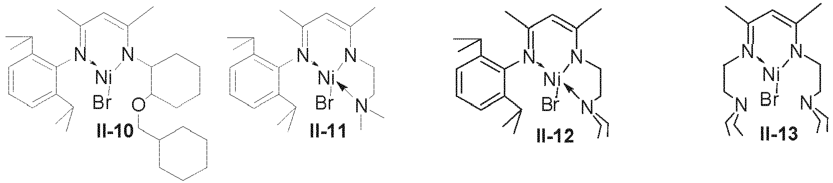

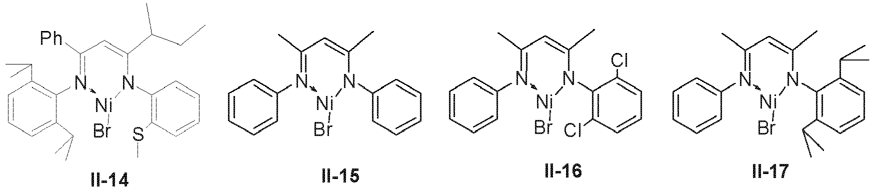

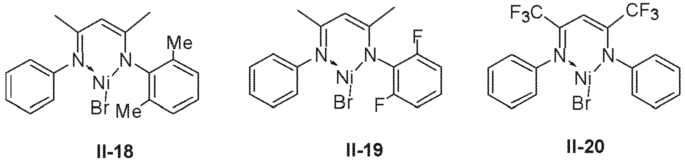

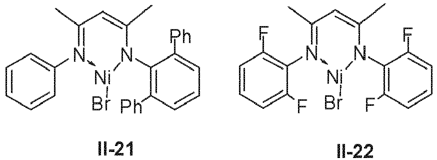

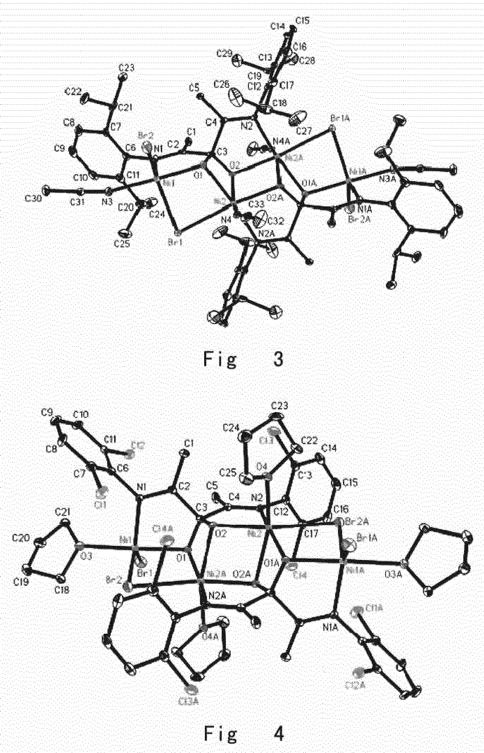

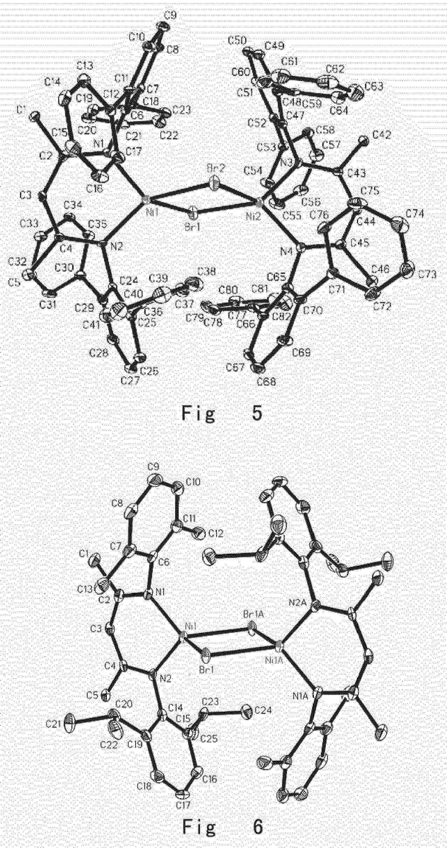

The elemental analysis data of each compound were as follows:
**II-2** elemental analysis: found (calculated) C: 38.84(38.84); H: 2.44(2.49); N: 5.15(5.33).
**II-3** elemental analysis: found (calculated) C: 56.85(56.80); H: 5.75(5.67); N: 6.20(6.31).
**II-4** elemental analysis: found (calculated) C: 71.03(71.13); H: 4.84(4.80); N: 4.05(4.05).
**II-5** elemental analysis: found (calculated) C: 52.30(52.44); H: 5.38(5.31); N: 4.36(4.22).
**II-6** elemental analysis: found (calculated) C: 51.01(51.06); H: 4.98(5.03); N: 5.14(5.18).
**II-7** elemental analysis: found (calculated) C: 60.04(60.04); H: 6.75(6.65); N: 5.53(5.60).
**II-8** elemental analysis: found (calculated) C: 54.32(54.37); H: 5.42(5.36); N: 5.61(5.51).
**II-9** elemental analysis: found (calculated) C: 57.18(57.41); H: 6.32(6.22); N: 5.61(5.58).
**II-10** elemental analysis: found (calculated) C: 60.94(61.04); H: 8.25(8.03); N: 4.68(4.75).
**II-11** elemental analysis: found (calculated) C: 54.28(54.00); H:7.41(7.34); N: 9.00(9.00).
**II-12** elemental analysis: found (calculated) C: 55.83(55.79); H:7.94(7.74); N: 8.52(8.49).
**II-13** elemental analysis: found (calculated) C: 47.27(47.04); H:8.28(8.13); N: 12.72(12.91).
**II-14** elemental analysis: found (calculated) C: 61.81(61.76); H:6.43(6.32); N: 4.60(4.50).
**II-15** elemental analysis: found (calculated) C: 52.58(52.63); H:4.50(4.42); N: 7.20(7.22).
**II-16** elemental analysis: found (calculated) C: 44.55(44.70); H:3.62(3.31); N: 5.99(6.13).
**II-17** elemental analysis: found (calculated) C: 58.72(58.52); H:6.22(6.19); N: 5.74(5.93).
**II-18** elemental analysis: found (calculated) C: 54.72(54.86); H:5.22(5.09); N: 6.55(6.73).
**II-19** elemental analysis: found (calculated) C: 48.31(48.17); H:3.29(3.57); N: 6.66(6.61).
**II-20** elemental analysis: found (calculated) C: 41.30(41.18); H:2.55(2.24); N: 5.60(5.65).
**II-21** elemental analysis: found (calculated) C: 64.70(64.49); H:4.78(4.67); N: 5.30(5.19).
**II-22** elemental analysis: found (calculated) C: 44.33(44.40); H:2.90(2.85); N: 6.13(6.09). The monocrystalline graphs of some of the compounds were shown in figures 4 to 7.

### Example 4 the synthesis of metal compound II-23

4.2g (10.0 mmol) of compound precursor was added into the 100mL reaction bottle, dissolved with 20 mL of tetrahydrofuran, and added dropwise into 0.29g (12.0 mmol) of sodium hydride in tetrahydrofuran (10 mL) at -30 °C. After reacting for 24h at room temperature, the sodium salt was added dropwise into PdCl₂ in tetrahydrofuran (10 mL) and reacted for 24h at room temperature. The reaction was stopped and the solvent was removed under reduced pressure. To the residue 20 mL of toluene was added, heated to dissolve, and filtered. The mother solution was cooled at -30 °C so as to obtain the product. After it was dried, a dark green crystal **II-23** was obtained, 4.2g (yield 75%). 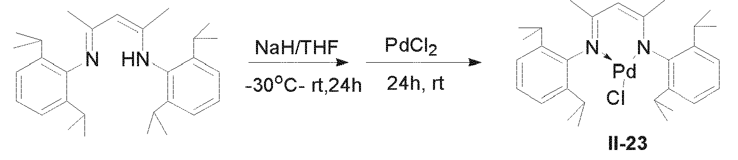
Elemental analysis: found (calculated): C: 62.67(62.62); H: 7.30(7.43); N: 5.06(5.04).

### Example 5 the synthesis of metal compound II-24-II-25

Other compounds, **II 24** (yield 59%), **II 25** (yield 55%), were obtained by the same experimental method as that of example 1. 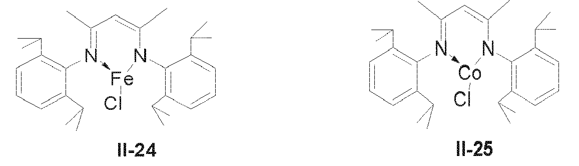
**II-24** Elemental analysis: found (calculated): C: 68.67(68.44); H: 8.30(8.12); N: 5.16(5.50);
**II-25** Elemental analysis: found (calculated): C: 68.37(68.02); H: 8.32(8.07); N: 5.26(5.47).

### Example 6 the synthesis of metal compound II-26

4.2g (10.0 mmol) of compound precursor was added into the 100mL reaction bottle, dissolved with 20 mL of tetrahydrofuran, and added dropwise into NiMe₂Py₄ in tetrahydrofuran (10 mL) at -30 °C. After reacting for 24h at room temperature, the reaction was stopped and the solvent was removed under reduced pressure. To the residue 20 mL of toluene was added, heated to dissolve, and filtered. The mother solution was cooled at -30 °C so as to obtain the product. After it was dried, a green crystal **II-26** was obtained, 4.1g (yield 80%). 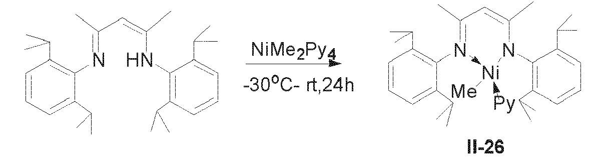
Elemental analysis: found (calculated): C: 73.59(73.69); H: 8.35(8.66); N: 7.58(7.37).

### Example 7 the synthesis of metal compound II-27

4.2g (10.0 mmol) of compound precursor was added into the 100mL reaction bottle, dissolved with 20 mL of tetrahydrofuran, and added dropwise into (7.4 g, 10.0 mmol)NiPh₂(PPh₃)₂ in tetrahydrofuran (10 mL) at -30 °C. After reacting for 24h at room temperature, the reaction was stopped and the solvent was removed under reduced pressure. To the residue 20 mL of toluene was added, heated to dissolve, and filtered. The mother solution was cooled at -30 °C so as to obtain the product. After it was dried, a green crystal **II-27** was obtained, 5.4g (yield 72%). 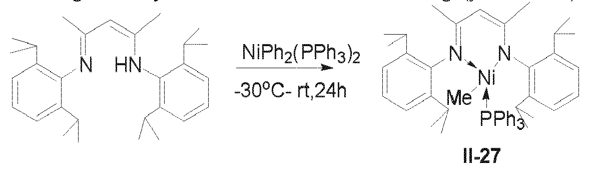
Elemental analysis: found (calculated): C: 76.67(76.49); H: 8.00(7.89); N: 3.85(3.72).

### Example 8 the synthesis of coordination compound I-1

Oxygen was introduced into 1.1 g (2.0mmol) of metal compound **III** in 50ml of toluene, which was stirred for one week at room temperature. The solvent was removed under vacuum. To the residue 10ml of hexane was added and filtered to obtain crude product, which was recrystalized with the mixed solvent of tetrahydrofuran and hexane so as to obtain the coordination compound **I-1** 167mg (yield 46%). 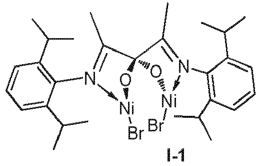
Elemental analysis: found (calculated): C: 47.99(47.99); H:5.70(5.55); N: 3.63(3.86)

### Example 9 the synthesis of coordination compound I-2 to I-16

The experimental method was basically the same as that of example 7, with the difference that compounds **II-2** to **II-22** were used to replace **I-1** so as to obtain other coordination compounds, **I-2** (yield 51%), **I-3** (yield 53%), **I-4** (yield 61%), **I-5** (yield 45%), **I-6** (yield 58%), **I-7** (yield 45%), **I-8** (yield 58%), **I-9** (yield 50%), **I-10** (yield 63%), **I-11** (yield 59%), **I-12** (yield 52%), **I-13** (yield 50%), **I-14** (yield 46%), **I-15** (yield 51%), **I-16** (yield 47%), **I-17** (yield 42%), **I-18** (yield 50%), **I-19** (yield 58%), **I-20** (yield 57%), **I-21** (yield 49%), **I-22** (yield 61%), **I-23** (yield 52%), **I-24** (yield 48%), **I-25** (yield 56%), **I-26** (yield 47%).

The respective correspondence between the raw materials and products were as shown in the table below.

| Raw material | Product | Yield % |
|---|---|---|
| II-2 | I-2 | 51 |
| II-3 | I-3 | 53 |
| II-4 | I-4 | 61 |
| II-5 | I-5 | 45 |
| II-6 | I-6 | 58 |
| II-7 | I-7 | 45 |
| II-8 | I-8 | 58 |
| II-9 | I-9 | 50 |
| II-10 | I-10 | 63 |
| II-11 | 1-11 | 59 |
| II-12 | I-12 | 52 |
| II-13 | I-13 | 50 |
| II-14 | I-14 | 46 |
| II-15 | I-15 | 51 |
| II-16 | I-16 | 47 |
| II-17 | I-17 | 42 |
| II-18 | I-18 | 50 |
| II-19 | I-19 | 58 |
| II-20 | I-20 | 57 |
| II-21 | I-21 | 49 |
| II-22 | I-22 | 61 |
| II-23 | I-23 | 52 |
| II-24 | I-24 | 48 |
| II-25 | I-25 | 56 |
| II-26 | I-26 | 47 |

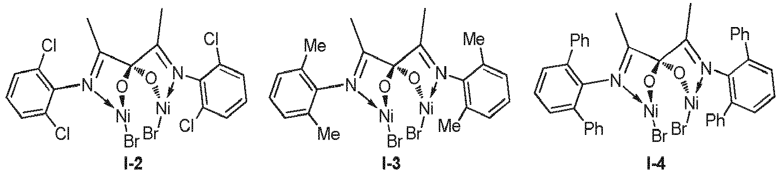

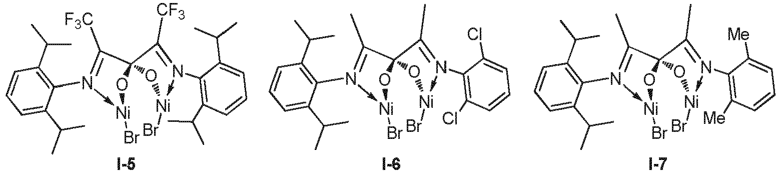

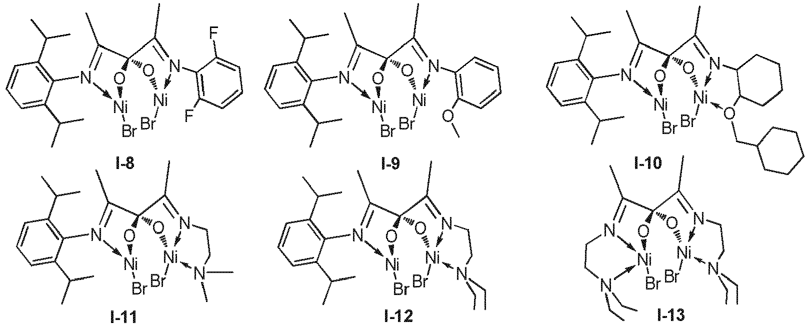

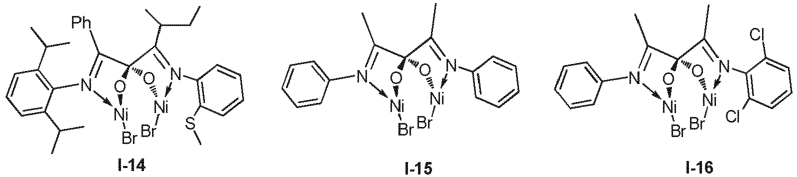

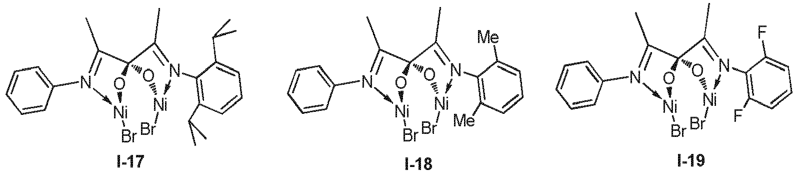

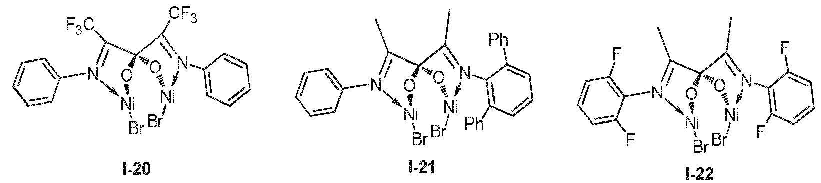

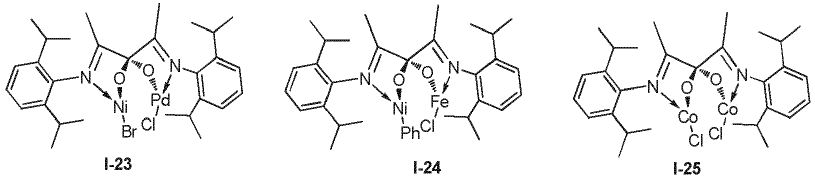

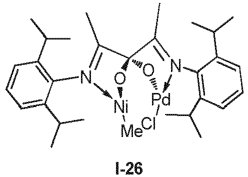

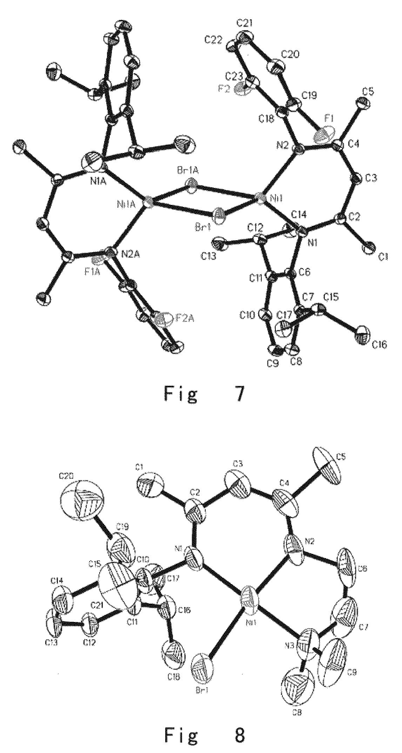

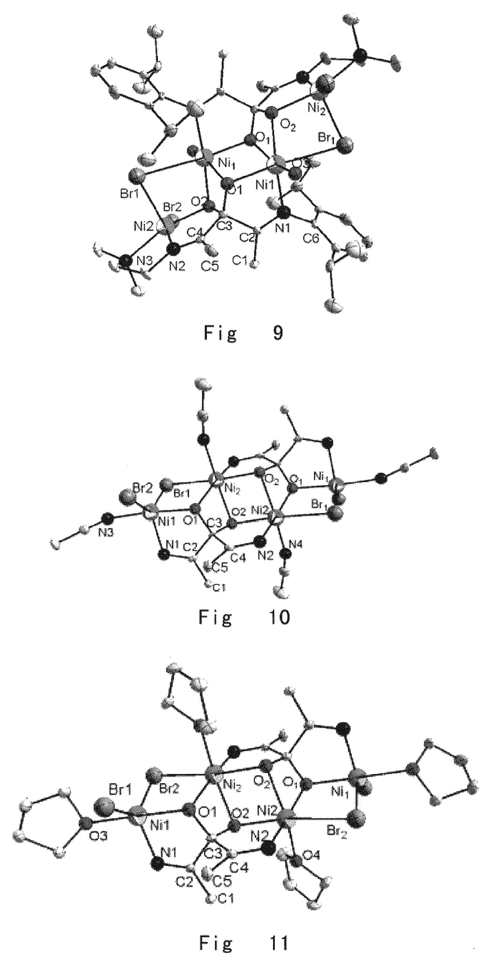

Some of the analysis data were as follows:
**I-2** Elemental analysis: found (calculated): C: 29.78(29.37); H: 1.98(1.74); N:4.20(4.03).
**I-3** Elemental analysis: found (calculated): C: 41.49(41.10); H: 4.36(3.94); N: 4.56(4.57).
**I-4** Elemental analysis: found (calculated): C: 57.00(57.13); H: 3.78(3.74); N: 3.01(3.25).
**I-5** Elemental analysis: found (calculated): C: 41.68(41.77); H: 4.32(4.11); N: 3.45(3.36).
**I-6** Elemental analysis: found (calculated): C: 38.90(38.88); H: 3.90(3.69); N: 4.00(3.94).
**I-7** Elemental analysis: found (calculated): C: 44.81(44.83); H: 5.09(4.82); N: 4.04(4.18).
**I-8** Elemental analysis: found (calculated): C: 40.59(40.76); H: 3.97(3.87); N: 4.19(4.13).
**I-9** Elemental analysis: found (calculated): C: 43.11(42.91); H: 4.72(4.50); N:4.03(4.17).
**I-10** Elemental analysis: found (calculated): C: 47.45(47.42); H: 6.18(6.10); N: 3.38(3.69).
**1-11** Elemental analysis: found (calculated): C: 40.21(39.61); H: 5.33(5.22); N: 6.61(6.60).
**I-12** Elemental analysis: found (calculated): C: 40.21(39.61); H: 5.33(5.22); N: 6.61(6.60).
**I-13** Elemental analysis: found (calculated): C: 40.21(39.61); H: 5.33(5.22); N: 6.61(6.60).
**I-14** Elemental analysis: found (calculated): C: 48.08(48.53); H: 4.78(4.84); N: 3.60(3.54).
**I-15** Elemental analysis: found (calculated): C: 40.21(39.61); H: 5.33(5.22); N: 6.61(6.60).
**I-16** Elemental analysis: found (calculated): C: 40.21(39.61); H: 5.33(5.22); N: 6.61(6.60).
**I-17** Elemental analysis: found (calculated): C: 40.21(39.61); H: 5.33(5.22); N: 6.61(6.60).
**I-18** Elemental analysis: found (calculated): C: 40.21(39.61); H: 5.33(5.22); N: 6.61(6.60).
**I-19** Elemental analysis: found (calculated): C: 40.21(39.61); H: 5.33(5.22); N: 6.61(6.60).
**I-20** Elemental analysis: found (calculated): C: 40.21(39.61); H: 5.33(5.22); N: 6.61(6.60).
**I-21** Elemental analysis: found (calculated): C: 40.21(39.61); H: 5.33(5.22); N: 6.61(6.60).
**I-22** Elemental analysis: found (calculated): C: 40.21(39.61); H: 5.33(5.22); N: 6.61(6.60).
**I-23** Elemental analysis: found (calculated): C: 47.38(47.77); H: 5.18(5.53); N: 3.59(3.84).
**I-24** Elemental analysis: found (calculated): C: 62.62(62.21); H: 6.75(6.71); N: 4.05(4.15).
**I-25** Elemental analysis: found (calculated): C: 54.29(54.64); H: 6.59(6.33); N: 4.59(4.39).
**I-26** Elemental analysis: found (calculated): C: 53.96(54.25); H: 6.78(6.52); N: 4.56(4.22). The monocrystal data of some of the compounds were shown in figures 8-10.

### Example 10 Catalyst I-1 to 1-11 in catalyzing ethylene polymerization experiments

Under 0.1Mpa of ethylene atmosphere, 100ml of toluene and MMAO (molar ratio of MMAO to catalyst was 1500) were sequentially added into the evacuated and baked 250ml polymerization flask. The reaction was vigorously stirred, and then placed in an oil bath at 30 °C, and maintained for a certain time, then a solution of catalyst **I-1-I-11** (5µmol) in toluene was added. After reacting for 10 minutes, the reaction was quenched with 5% of hydrochloric acid in ethanol. After the polymer was precipitated, filtered and washed, it was dried under vacuum at 50 °C to constant weight so as to obtain polyethylene. The homopolymerization results of ethylene were shown in the following table.

**Table 1 the results of catalyst I-1 to I-11 in catalyzing ethylene polymerization**

| Cat | W (g) | Activity (10⁵ g/mol·h·atm) | *M*_{w}/ 10⁴ g/mol | *M*_{w}/*M*ₙ | Branched/ 1000C |
|---|---|---|---|---|---|
| **I-1** | 0.8253 | 10.0 | 66.8 | 2.2 | 14.4 |
| **I-2** | 0.3066 | 3.7 | 59.9 | 2.9 | 15.1 |
| **I-3** | 0.3916 | 4.7 | 56.2 | 2.3 | 21.1 |
| **I-4** | 0.4215 | 5.1 | 39.5 | 2.1 | 13.5 |
| **I-5** | 0.3756 | 4.5 | 47.5 | 2.6 | 14.5 |
| **I-6** | 0.4766 | 5.7 | 49.5 | 3.8 | 15.5 |
| **I-7** | 0.3788 | 4.5 | 62.7 | 3.6 | 12.6 |
| **I-8** | 0.3521 | 4.2 | 45.9 | 3.1 | 13.1 |
| **I-9** | 0.2985 | 3.6 | 55.6 | 2.5 | 15.2 |
| **I-10** | 0.1256 | 1.5 | 58.6 | 3.2 | 13.2 |
| **I-11** | trace | - | - | - | - |

| | | | | | |
|---|---|---|---|---|---|
| Notes: Cat: catalyst; W: the weight of polyethylene; Activity: the activity; M_{w}: The weight average molecular weight; *M*_{w}/*M*ₙ: molecular weight distribution. | | | | | |

The results had shown that the catalyst system could catalyze the polymerization of ethylene under ordinary pressures, of which the activities could be up to 10⁵ g/mol· h, the molecular weight was 10⁵ g/mol, and the degree of branching was 12-21/1000 C.

### Example 11 some of the catalysts in catalyzing ethylene polymerization experiment under high pressure

Under 1.0Mpa of ethylene atmosphere, 100ml of toluene and MMAO (molar ratio of MMAO to catalyst was 1500) were sequentially added into the evacuated and baked 350ml autoclave. The reaction was vigorously stirred, and then placed in an oil bath at 30 °C, and kept for a certain time, then a solution of catalyst **I-1, I-2, I-3, I-4, I-5** or **II-1** (5µmol for each) in toluene was added. After reacting for 10 minutes, the reaction was quenched with 5% of hydrochloric acid in ethanol. After the polymer was precipitated, filtered and washed, it was dried under vacuum at 50°C to constant weight so as to obtain polyvinyl. The polymerization results of ethylene were shown in the following table.

**Table 2 results of some catalysts in catalyzing ethylene polymerization under high pressure**

| Cat | W (g) | Activity (10⁵ g/mol·h·atm) | *M*_{w}/ 10⁴ g/mol | *M*_{w}/*M*ₙ | Branched/ 1000C |
|---|---|---|---|---|---|
| **I-1** | 1.2021 | 1.4 | 201.8 | 2.1 | 9.5 |
| **I-2** | 1.0357 | 1.2 | 135.2 | 2.4 | 5.6 |
| **I-3** | 0.2018 | 0.24 | 79.8 | 8.0 | 6.2 |
| **I-4** | 0.8536 | 1.0 | 132.2 | 2.3 | 7.1 |
| **I-5** | 0.6532 | 0.78 | 145.3 | 2.8 | 2.8 |
| **II-1** | 0.1650 | 0.20 | 61.6 | 2.2 | 19 |

| | | | | | |
|---|---|---|---|---|---|
| Notes: Cat: catalyst; W: the weight of copolymer; Activity: the activity; *M*_{w}: The weight average molecular weight; *M*_{w}/*M*ₙ: molecular weight distribution. | | | | | |

The results had shown that compared to the polymerization under ordinary pressure, the molecular weight of obtained polymer could reach up to 10⁶ g/mol, while the degree of branching was reduced. Comparing to catalyst C-1 with one single active centre, the activity of A-1 was increased by one order of magnitudes.

### Example 12 some of the catalysts in catalyzing propylene polymerization experiment

Under 0.1Mpa of propylene atmosphere, 100ml of toluene and MMAO (molar ratio of MMAO to catalyst was 1500) were sequentially added into the evacuated and baked 250 m polymerization flask. The reaction was vigorously stirred, and then placed in an oil bath at 30 °C, and kept for a certain time, then a solution of catalyst **I-1** to **I-5** (5µmol) in toluene was added. After reacting for 10 minutes, the reaction was quenched with 5% of hydrochloric acid in ethanol. After the polymer was precipitated, filtered and washed, it was dried under vacuum at 50 °C to constant weight so as to obtain polypropylene. The homopolymerization results of propylene were shown in the following table.

**Table 3 results of some catalysts in catalyzing propylene polymerization**

| Cat | W (g) | Activity (10⁵ g/mol·h·atm) | *M*_{w}/10⁴g/mol | *M*_{w}/*M*ₙ |
|---|---|---|---|---|
| **I-1** | 0.5623 | 6.7 | 56.3 | 2.5 |
| **I-2** | 0.4562 | 5.5 | 53.2 | 2.2 |
| **I-3** | 0.2564 | 3.1 | 56.3 | 2.4 |
| **I-4** | 0.4326 | 5.2 | 49.5 | 2.7 |
| **I-5** | 0.2856 | 3.4 | 65.2 | 2.1 |

| | | | | |
|---|---|---|---|---|
| Notes: Cat: catalyst; W: the weight of copolymer; Activity: the activity; M_{w}: The weight average molecular weight; *M*_{w}/*M*ₙ: molecular weight distribution. | | | | |

The results had shown that the catalyzing system could catalyze the polymerization of propylene in comparatively high activity.

### Example 13 some of the catalysts in catalyzing hexylene polymerization

100ml of toluene, 10 mL of 1-hexylene, MMAO (molar ratio of MMAO to catalyst was 1500) were sequentially added into the evacuated and baked 250ml polymerization flask. The reaction was vigorously stirred, and then placed in an oil bath at 30 °C, and kept for a certain time, then a solution of catalyst **I-1** to **I-5** (5µmol) in toluene was added. After reacting for 10 minutes, the reaction was quenched with 5% of hydrochloric acid in ethanol. After the polymer was washed, it was vacuum dried at 50°C to constant weight so as to obtain polyhexylene. The homopolymerization results of hexylene were shown in the following table.

**Table 4 results of some catalysts in catalyzing hexylene polymerization**

| Cat | W (g) | Activity (10⁻⁵ g/mol·h·atm) | *M*_{w}/ 10⁴ g/mol | *M*_{w}/*M*ₙ |
|---|---|---|---|---|
| **I-1** | 0.2356 | 2.8 | 23.2 | 2.1 |
| **I-2** | 0.1956 | 2.3 | 19.5 | 1.9 |
| **I-3** | 0.1231 | 1.5 | 18.5 | 2.5 |
| **I-4** | 0.1185 | 1.4 | 13.2 | 2.6 |
| **I-5** | 0.0985 | 1.2 | 18.5 | 2.4 |

| | | | | |
|---|---|---|---|---|
| Notes: Cat: catalyst; W: the weight of copolymer; Activity: the activity; *M*_{w}: The weight average molecular weight; *M*_{w}/*M*ₙ: molecular weight distribution. | | | | |

The results had shown that the catalyzing system could well catalyze the polymerization of hexylene.

### Example 14 I-1 catalyst in catalyzing the copolymerization of ethylene and olefin

Under 0.1Mpa of ethylene atmosphere, 100ml of toluene, 10 ml of olefin and MMAO (molar ratio of MMAO to catalyst was 1500) were sequentially added into the evacuated and baked 250ml polymerization flask. The reaction was vigorously stirred, and then placed in an oil bath at 30 °C, and kept for a certain time, then a solution of catalyst I-1 (5µmol) in toluene was added. After reacting for 10 minutes, the reaction was quenched with 5% of hydrochloric acid in ethanol. After the polymer was washed, it was vacuum dried at 50°C to constant weight so as to obtain the polymer. The copolymerization results of ethylene and olefin were shown in the following table.

**Table 5 the results of I-1 catalyst in catalyzing the copolymerization of ethylene and olefin**

| Olefin | W (g) | Activity (10⁵ g/mol·h·atm) | Incorp.(mol%) | *M*_{w}/ 10⁴ g/mol | *M*_{w}/*M*ₙ |
|---|---|---|---|---|---|
| 1-octadecene | 0.4356 | 5.2 | 15.3 | 22.2 | 2.2 |
| Norbornene | 0.3956 | 4.7 | 13.1 | 20.5 | 2.8 |
| Cyclopentene | 0.4432 | 5.3 | 15.3 | 23.0 | 2.4 |
| Cyclohexene | 0.4612 | 5.5 | 17.3 | 25.1 | 2.6 |
| Cyclopentadiene dimer | 0.4220 | 5.1 | 14.2 | 22.1 | 2.1 |

The results had shown that the catalyzing system could well catalyze the copolymerization of olefin.

### Example 15 some of the catalyst in catalyzing the copolymerization of ethylene and

### AlⁱBu₃ protected polar monomer

The copolymerization monomer used was polar monomer **M03**, which was treated with AlⁱBu₃ in accordance with the method T1 as described in the general methods.

The copolymerization reactions were conducted in accordance with the method as described in general method, after polymerization was completed, 5% (by volume) hydrochloric acidified ethanol was used to precipitate the copolymer, which was washed by (100mLx3) of ethanol.

Other polymerization conditions, polymerization results and the representation results of the copolymer were listed in table 6 in detail.

**Table 6 ethylene/M03 copolymerization data**

| example | catalyst | Amount of catalyst (µmol) | Amount of MMAO (mmol) | Amount of **M03** (mmol) | Polymerization temperature (°C) | Polymerization activity (10⁵g/mol·h·atm) | Molecular weight of Copolymer *M_{w}* (10⁵ g/mol) | Polar monomer insertion rate (mmol-%) |
|---|---|---|---|---|---|---|---|---|
| 1 | **I-1** | 5.0 | 5.0 | 2 | 30 | 12.0 | 7.2 | 1.7 |
| 2 | **I-1** | 5.0 | 7.5 | 2 | 50 | 8.4 | 6.9 | 0.9 |
| 3 | **I-1** | 5.0 | 5.0 | 10 | 30 | 5.1 | 6.1 | 2.4 |
| 4 | **I-1** | 5.0 | 5.0 | 10 | 50 | 4.3 | 5.9 | 2.8 |
| 5 | **I-1** | 10.0 | 10.0 | 10 | 30 | 3.9 | 6.2 | 3.1 |
| 6 | **I-1** | 5.0 | 15.0 | 20 | 30 | 2.5 | 6.5 | 3.4 |
| 7 | **I-2** | 5.0 | 7.5 | 10 | 30 | 2.1 | 5.9 | 2.2 |
| 8 | **I-3** | 5.0 | 7.5 | 10 | 30 | 3.3 | 6.4 | 2.4 |
| 9 | **I-4** | 5.0 | 7.5 | 10 | 30 | 5.8 | 6.6 | 2.4 |
| 10 | **I-5** | 5.0 | 7.5 | 10 | 40 | 5.1 | 6.0 | 2.0 |
| 11 | **I-6** | 5.0 | 7.5 | 10 | 40 | 4.3 | 6.4 | 2.1 |
| 12 | **I-7** | 5.0 | 7.5 | 10 | 40 | 3.9 | 5.8 | 2.2 |
| 13 | **I-8** | 5.0 | 7.5 | 10 | 40 | 3.2 | 6.4 | 2.1 |
| 14 | **I-9** | 5.0 | 7.5 | 10 | 30 | 4.0 | 5.6 | 1.8 |
| 15 | **I-10** | 5.0 | 7.5 | 10 | 30 | 4.2 | 6.1 | 1.9 |
| 16 | **I-11** | 15.0 | 15.0 | 5 | 30 | 0.5 | 5.3 | 1.4 |
| 17 | **I-12** | 15.0 | 15.0 | 5 | 30 | 0.6 | 5.7 | 1.6 |
| 18 | **I-13** | 20.0 | 30.0 | 5 | 30 | 0.4 | 5.5 | 1.1 |
| 19 | **I-14** | 5.0 | 7.5 | 5 | 30 | 6.2 | 6.2 | 1.7 |
| 20 | **I-15** | 10.0 | 15.0 | 10 | 30 | 1.2 | 7.2 | 2.1 |
| 21 | **I-16** | 5.0 | 7.5 | 5 | 30 | 2.8 | 6.7 | 1.3 |
| 22 | **I-17** | 5.0 | 7.5 | 5 | 30 | 3.0 | 7.4 | 1.5 |
| 23 | **I-18** | 5.0 | 7.5 | 5 | 30 | 2.7 | 6.9 | 1.6 |
| 24 | **I-19** | 5.0 | 7.5 | 5 | 40 | 2.4 | 7.1 | 1.2 |
| 25 | **I-20** | 5.0 | 7.5 | 5 | 40 | 2.3 | 6.7 | 1.5 |
| 26 | **I-21** | 5.0 | 7.5 | 10 | 40 | 1.9 | 5.6 | 2.4 |
| 27 | **I-22** | 20.0 | 30.0 | 10 | 40 | 0.5 | 4.8 | 3.1 |

The conclusion could be drawn from the data in above table that: all the catalysts I-1 to I-22 could well catalyze the copolymerization of ethylene and the AlⁱBu₃ protected monomer **M03** under ordinary pressure, of which the activity could reach up to 10⁵ g/mol·h, the molecular weight was 10⁵ g/mol, and the polar monomer insertion rate was 0.9mol-3.4mol%.

### Example 16 some of the catalysts in catalyzing the copolymerization of ethylene and TBS or TMS protected polar monomer

The copolymerization monomers as used were polar monomer **M18, 19, 20, 21, 22, and 23**.

The copolymerization reactions were conducted in accordance with the method as described in general method, the amount of catalyst used was 5umol, and the co-catalyst was MMAO, and the polymerization time was 10 min. After the polymerization was completed, 5% of hydrochloric acid (by volume) in ethanol was used to precipitate the copolymer, which was washed with (100mL×3) of ethanol.

Other polymerization conditions, polymerization results and the representation results of the copolymer were listed in table 7 in detail.

**Table 7 ethylene/polar monomer copolymerization data**

| Example | catalyst | polar monomer | The amount of polar monomer (mmol) | temperature (°C) | The amount of MMAO (mmol) | Activity (10⁵g/mol ·h·atm) | Polar monomer insertion rate (mol %) |
|---|---|---|---|---|---|---|---|
| 28 | **I-1** | **M20** | 2 | 40 | 5.0 | 14.0 | trace |
| 29 | **I-1** | **M20** | 5 | 40 | 5.0 | 8.2 | 2.1 |
| 30 | **I-1** | **M20** | 10 | 40 | 5.0 | 7.4 | 2.9 |
| 31 | **I-1** | **M18** | 10 | 30 | 5.0 | 5.6 | 1.1 |
| 32 | **I-1** | **M19** | 10 | 30 | 5.0 | 6.7 | 2.1 |
| 33 | **I-1** | **M21** | 10 | 30 | 5.0 | 4.3 | 1.4 |
| 34 | **I-1** | **M22** | 10 | 30 | 5.0 | 5.0 | 2.4 |
| 35 | **I-1** | **M23** | 10 | 30 | 5.0 | 6.8 | 3.3 |
| 36 | **I-2** | **M20** | 10 | 30 | 5.0 | 5.4 | 1.9 |
| 37 | **I-3** | **M20** | 10 | 30 | 5.0 | 5.9 | 2.1 |
| 38 | **I-4** | **M20** | 10 | 30 | 5.0 | 6.1 | 2.3 |
| 39 | **I-5** | **M20** | 20 | 30 | 5.0 | 2.1 | 4.4 |
| 40 | **I-6** | **M20** | 20 | 40 | 5.0 | 3.2 | 4.9 |
| 41 | **I-7** | **M20** | 20 | 40 | 5.0 | 1.2 | 5.1 |
| 42 | **I-8** | **M20** | 10 | 40 | 5.0 | 1.6 | 2.8 |
| 43 | **I-9** | **M20** | 10 | 40 | 5.0 | 3.1 | 2.6 |

The conclusion could be drawn from the data in above table that: all the catalysts could well catalyze the copolymerization of ethylene and the TBS or TMS protected monomer under ordinary pressure, of which the activity could reach up to 10⁵ g/mol·h, the molecular weight was 10⁵ g/mol, and the polar monomer insertion rate was 1.1mol-4.9mol%.

### Example 17 some of the catalysts in catalyzing the copolymerization of ethylene and co-catalyst protected polar monomer

The polar monomer was treated in accordance with the method T2 as described in the general methods.

The copolymerization reactions were conducted in accordance with the method as described in general method, the amount of main catalyst used was 5µmol, and the co-catalyst was MMAO, the amount of the polar monomer used was 10mmol. Before the polymerization, the co-catalyst and the polar monomer was mixed and stirred to react for 3h, the polymerization temperature was 30°C, and the polymerization time was 10 min. After the polymerization was completed, 5%(by volume) of hydrochloric acid in ethanol was used to precipitate the copolymer, which was washed with ethanol (100mLx3).

Other polymerization conditions, polymerization results and the representation results of the copolymer were listed in table 8 in detail.

**Table 8 ethylene/polar monomer copolymerization data**

| Example | catalyst | polar monomer | The amount of co-catalyst (mmol) | Polymerization activity (10⁵g/mol·h·atm) | Molar weight of copolymer *M_{w}* (10⁵g/mol) | Polar monomer insertion rate (mol%) |
|---|---|---|---|---|---|---|
| 44 | **I-1** | **M03** | 15.0 | 6.2 | 8.5 | 7.4 |
| 45 | **I-1** | **M05** | 15.0 | 5.9 | 7.9 | 6.9 |

The conclusion could be drawn from the data in above table that: the catalysts could well catalyze the copolymerization of ethylene and different monomers under ordinary pressure after the polar monomers were pre-treated with MMAO.

### Example 18 some of the catalysts in catalyzing the copolymerization of ethylene and unprotected polar monomer

The copolymerization monomers were used directly to copolymerize with ethylene without being protected.

The copolymerization reactions were conducted in accordance with the method as described in general method, the co-catalyst was MMAO, and the polymerization temperature was 30°C. MMAO was added into 50mL of solvent, and the polar monomer was added after 5 min. The mixture was stirred together for 10min, after that, the pre-dissolved catalyst solution was added. The polymerization was conducted for 10 min. After the polymerization was completed, 5%(by volume) of hydrochloric acid in ethanol was used to precipitate the copolymer, which was washed with ethanol (100mLx3).

Other polymerization conditions, polymerization results and the representation results of the copolymer were listed in table 9 in detail.

**Table 9 ethylene/polar monomer copolymerization data**

| Example | catalyst | The amount of catalyst (µmol) | The amount of MMAO (mmol) | polar monomer | The amount of monomer (mmol) | Polymerization temperature (°C) | Polymerization activity (10⁵g/mol·h·atm) | Polar monomer insertion rate (mol-%) |
|---|---|---|---|---|---|---|---|---|
| 1 | **I-1** | 5.0 | 15.0 | **M03** | 2 | 30 | 9.7 | 2.5 |
| 2 | **I-1** | 5.0 | 15.0 | **M03** | 5 | 30 | 14.4 | 3.7 |
| 3 | **I-1** | 5.0 | 25.0 | **M03** | 10 | 30 | 7.1 | 7.4 |
| 4 | **I-1** | 5.0 | 10.0 | **M02** | 2 | 30 | 14.0 | 0.5 |
| 5 | **I-1** | 5.0 | 25.0 | **M02** | 5 | 40 | 7.4 | 1.3 |
| 6 | **I-1** | 5.0 | 25.0 | **M02** | 10 | 40 | 4.0 | 2.2 |
| 7 | **I-1** | 30.0 | 120.0 | **M01** | 30 | 40 | 0.1 | 1.2 |
| 8 | **I-1** | 5.0 | 25.0 | **M04** | 10 | 40 | 8.6 | 9.6 |
| 9 | **I-1** | 5.0 | 25.0 | **M05** | 10 | 50 | 5.8 | 7.8 |
| 10 | **I-1** | 10.0 | 40.0 | **M06** | 20 | 50 | 1.1 | 1.4 |
| 11 | **I-1** | 5.0 | 25.0 | **M07** | 10 | 30 | 6.5 | 8.1 |
| 12 | **I-1** | 10.0 | 40.0 | **M08** | 20 | 30 | 2.3 | 1.5 |
| 13 | **I-1** | 5.0 | 25.5 | **M09** | 10 | 30 | 9.1 | 5.6 |
| 14 | **I-1** | 5.0 | 25.0 | **M10** | 10 | 30 | 7.5 | 6.9 |
| 15 | **I-1** | 10.0 | 50.0 | **M11** | 10 | 30 | 1.2 | 2.4 |
| 16 | **I-1** | 10.0 | 50.0 | **M12** | 10 | 30 | 2.3 | 5.3 |
| 17 | **I-1** | 10.0 | 50.0 | **M13** | 10 | 30 | 3.2 | 1.8 |
| 18 | **I-2** | 5.0 | 15.0 | **M03** | 5 | 30 | 1.0 | 3.0 |
| 19 | **I-3** | 5.0 | 25.0 | **M03** | 10 | 30 | 3.2 | 6.7 |
| 20 | **I-4** | 5.0 | 25.0 | **M05** | 10 | 30 | 2.1 | 4.9 |
| 21 | **I-5** | 5.0 | 25.0 | **M11** | 10 | 30 | 0.8 | 1.2 |
| 22 | **I-6** | 5.0 | 25.0 | **M07** | 10 | 30 | 4.5 | 7.2 |
| 23 | **I-7** | 5.0 | 25.0 | **M07** | 10 | 30 | 1.2 | 2.1 |
| 24 | **I-8** | 5.0 | 25.0 | **M03** | 10 | 30 | 1.6 | 5.5 |
| 25 | **I-9** | 5.0 | 15.0 | **M03** | 5 | 40 | 3.4 | 3.1 |
| 26 | **I-10** | 5.0 | 15.0 | **M04** | 5 | 40 | 2.7 | 2.6 |
| 27 | **I-15** | 5.0 | 15.0 | **M03** | 5 | 40 | 1.1 | 1.7 |
| 28 | **I-17** | 5.0 | 15.0 | **M17** | 5 | 40 | 2.0 | 2.1 |
| 29 | **I-21** | 5.0 | 15.0 | **M17** | 5 | 30 | 2.9 | 2.8 |
| 30 | **I-22** | 5.0 | 15.0 | **M03** | 5 | 30 | 0.9 | 1.4 |

The conclusion could be drawn from the data in above table that: the catalysts could well catalyze the copolymerization of ethylene and unprotected monomers under ordinary pressure, while compared with the monomers protected the activity was slightly lower; for some of the monomers, compared with the monomers protected, the insertion rate was higher when it was unprotected, such as M03; the insertion of some monomers that were usually difficult to copolymerize could be achieved, such as M01, M06 and M08.

### Example 19 some of the catalysts in catalyzing the copolymerization of propylene and polar monomer

The copolymerization monomers were used directly to copolymerize with propylene without being protected.

The copolymerization reactions were conducted in accordance with the method as described in general method, the co-catalyst was MMAO, and the polymerization temperature was 30°C. MMAO was added into 50mL of solvent, and the polar monomer was added after 5 min. The mixture was stirred together for 10min, after that, the pre-dissolved catalyst solution was added. The polymerization was conducted for 10 min. After the polymerization was completed, 5% (by volume) of hydrochloric acid in ethanol was used to precipitate the copolymer, which was washed with ethanol (100mLx3).

Other polymerization conditions, polymerization results and the representation results of the copolymer were listed in table 10 in detail.

**Table 10 propylene/polar monomer copolymerization data**

| Example | catalyst | The amount of catalyst (µmol) | The amount of MMAO (mmol) | polar monomer | The amount of monomer (mmol) | Polymerization temperature (°C) | Polymerization activity (10⁵g/mol·h·atm) | Polar monomer insertion rate (mol-%) |
|---|---|---|---|---|---|---|---|---|
| 3 | **I-1** | 5.0 | 25.0 | **M03** | 10 | 30 | 8.2 | 8.9 |
| 6 | **I-1** | 5.0 | 25.0 | **M02** | 10 | 40 | 5.2 | 2.5 |
| 7 | **I-1** | 30.0 | 120.0 | **M01** | 30 | 40 | 0.3 | 1.4 |
| 9 | **I-1** | 5.0 | 25.0 | **M05** | 10 | 40 | 4.7 | 9.1 |
| 10 | **I-1** | 10.0 | 40.0 | **M06** | 20 | 30 | 1.8 | 2.1 |
| 11 | **I-1** | 5.0 | 25.0 | **M07** | 10 | 30 | 8.7 | 9.4 |
| 12 | **I-1** | 10.0 | 40.0 | **M08** | 20 | 50 | 3.4 | 2.1 |
| 14 | **I-1** | 5.0 | 25.0 | **M10** | 10 | 50 | 7.9 | 7.3 |
| 15 | **I-1** | 10.0 | 50.0 | **M11** | 10 | 30 | 1.9 | 3.2 |
| 16 | **I-1** | 10.0 | 50.0 | **M12** | 10 | 30 | 2.0 | 5.9 |
| 18 | **I-2** | 5.0 | 15.0 | **M03** | 5 | 30 | 1.9 | 4.1 |
| 19 | **I-3** | 5.0 | 25.0 | **M03** | 10 | 30 | 2.9 | 7.3 |
| 20 | **1-4** | 5.0 | 25.0 | **M05** | 10 | 40 | 3.1 | 5.8 |
| 21 | **I-5** | 5.0 | 25.0 | **M11** | 10 | 40 | 1.8 | 1.0 |
| 22 | **I-6** | 5.0 | 25.0 | **M07** | 10 | 45 | 5.6 | 9.1 |
| 23 | **I-7** | 5.0 | 25.0 | **M07** | 10 | 45 | 0.9 | 1.9 |
| 24 | **I-8** | 5.0 | 25.0 | **M03** | 10 | 45 | 1.2 | 6.7 |
| 25 | **I-9** | 5.0 | 15.0 | **M03** | 5 | 45 | 4.5 | 4.3 |
| 26 | **I-10** | 5.0 | 15.0 | **M04** | 5 | 30 | 3.2 | 3.0 |
| 27 | **I-15** | 5.0 | 15.0 | **M03** | 5 | 30 | 2.1 | 1.5 |

The results had shown that the catalysts could well catalyze the copolymerization of propylene and unprotected monomers under ordinary pressure, and the insertion of some monomers that were usually difficult to copolymerize could be achieved, such as M01, M06 and M08.

### Example 20 some of the catalysts in catalyzing the copolymerization of 1-butylene and polar monomer

The copolymerization monomers were used directly to copolymerize with 1-butylene without being protected.

The copolymerization reactions were conducted in accordance with the method as described in general method, the co-catalyst was MMAO, and the polymerization temperature was 30°C. MMAO was added into 50mL of solvent, and the polar monomer was added after 5 min. The mixture was stirred together for 10min, after that, the pre-dissolved catalyst solution was added. The polymerization was conducted for 10 min. After the polymerization was completed, 5% (by volume) of hydrochloric acid in ethanol was used to precipitate the copolymer, which was washed with ethanol (100mLx3).

Other polymerization conditions, polymerization results and the representation results of the copolymer were listed in table 11 in detail.

**Table 11 1-butylene/polar monomer copolymerization data**

| Example | catalyst | The amount of catalyst (µmol) | The amount of MMAO (mmol) | polar monomer | The amount of the monomer (mmol) | Polymerization temperature monomer (°C) | Polymerization activity (10⁵g/mol·h·atm) | Polar monomer insertion rate (mol%) |
|---|---|---|---|---|---|---|---|---|
| 1 | **I-1** | 5.0 | 25.0 | **M03** | 10 | 30 | 4.2 | 5.2 |
| 2 | **I-1** | 5.0 | 25.0 | **M02** | 10 | 30 | 2.3 | 1.2 |
| 3 | **I-1** | 30.0 | 120.0 | **M01** | 30 | 30 | 0.1 | 0.5 |
| 4 | **I-2** | 5.0 | 15.0 | **M03** | 5 | 30 | 1.2 | 2.1 |
| 5 | **I-3** | 5.0 | 25.0 | **M03** | 10 | 30 | 2.3 | 5.6 |
| 6 | **I-4** | 5.0 | 25.0 | **M05** | 10 | 30 | 1.7 | 3.2 |
| 7 | **I-5** | 5.0 | 25.0 | **M11** | 10 | 30 | 0.4 | 0.8 |
| 8 | **I-6** | 5.0 | 25.0 | **M07** | 10 | 30 | 3.2 | 5.6 |
| 10 | **I-8** | 5.0 | 25.0 | **M03** | 10 | 30 | 1.2 | 3.4 |
| 11 | **I-9** | 5.0 | 15.0 | **M03** | 5 | 30 | 2.1 | 2.1 |

The results had shown that the catalysts could well catalyze the copolymerization of 1-butylene and unprotected monomers under ordinary pressure.

### Example 21 some of the catalysts in catalyzing the polymerization of ethylene and polar monomer under high pressure

Under 1.0Mpa of ethylene atmosphere, 100 mL of toluene, MMAO (molar ratio of MMAO to catalyst was 3000) were sequentially added into the evacuated and baked 350mL autoclave. The reaction was vigorously stirred, and then placed in an 30 °C oil bath, and kept for a certain time, then a solution of catalyst **I-1, I-2, I-3, I-4, I-5** or **I-8** (5µmol for each) in toluene was added. After reacting for 10 minutes, the reaction was quenched with 5% of hydrochloric acid in ethanol. After the polymer was precipitated, filtered and washed, it was dried under vacuum at 50°C to constant weight so as to obtain polyvinyl. The polymerization results of ethylene were shown in following table.

**Table 12 the polymerization results of some of the catalysts in catalyzing ethylene polymerization under high pressure**

| Cat | polar monomer | Activity (10⁵ g/mol·h·atm) | *M*_{w}/ 10⁴g/mol | *M*_{w}/*M*ₙ | Polar monomer insertion rate (mol%) |
|---|---|---|---|---|---|
| **I-1** | **M03** | 1.2 | 192.3 | 2.3 | 3.2 |
| **I-2** | **M03** | 1.3 | 123.0 | 2.1 | 2.3 |
| **I-3** | **M04** | 0.5 | 89.1 | 4.5 | 2.1 |
| **I-4** | **M04** | 1.2 | 121.1 | 2.2 | 2.0 |
| **I-5** | **M05** | 0.9 | 132.1 | 2.4 | 1.8 |
| **I-8** | **M05** | 0.5 | 45.1 | 2.6 | 2.1 |

The results had shown that compared to the ordinary pressure polymerization, the molecular weight of the obtained polymer could reach up to 10⁶ g/mol, and the insertion rate was obviously reduced.

### Example 22 the synthesis of compound II'

In example 22, the compound precursor II' was all compounds of the following structure:

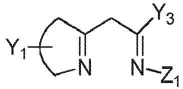

The preparation method 1 of symmetric or asymmetric substituted compound precursor II' was as follows:

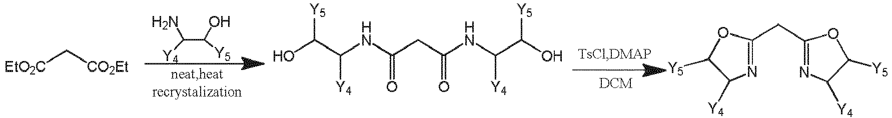

Diethyl malonate and substituted β- amino alcohol were condensed, and a intermediate was obtained by recrystalization. The intermediate was condensed to form a ring by using TsCl, DMAP and using methylene chloride as solvent to give the final product.

The preparation method 2 of asymmetric substituted compound precursor II' was as follows:

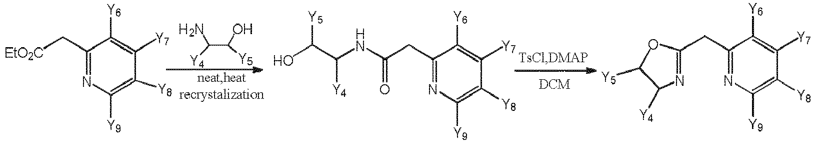

Multi-substituted pyridine and substituted β- amino alcohol were condensed to give intermediate, while the intermediate was condensed to form a ring by using TsCl, DMAP and using methylene chloride as solvent to give the final product.

The preparation method 3 of symmetric or asymmetric substituted compound precursor II' was as follows:

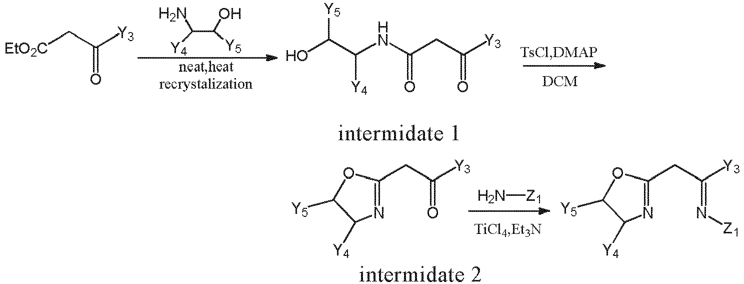

β-carboxylic ester substituted ketone and substituted β- amino alcohol were condensed to give the intermediate 1, while the intermediate 1 was condensed to form a ring by using TsCl, DMAP and using methylene chloride as solvent to give the intermediate 2, and the intermediate 2 was condensed with substituted amine catalyzed by Lewis acid to give the final product.

Other compounds, **II'-1** (yield 62%), **II'-2** (yield 87%), **II'-3** (yield 72%), **II'-4** (yield 89%), **II'-5** (yield 79%), **II'-6** (yield 81%), **II'-7** (yield 91%), **II'-8** (yield 61%), **II'-9** (yield 66%), **II'-10** (yield 54%), **II'-11** (yield 76%), **II'-12** (yield 61%), **II'-13** (yield 57%), **II'-14** (yield 87%), **II'-15** (yield 76%), **II'-16** (yield 68%), **II'-17** (yield 81%), **II'-18** (yield 56%), **II'-19** (yield 64%), **II'-20** (yield 76%), were obtained by the above method.

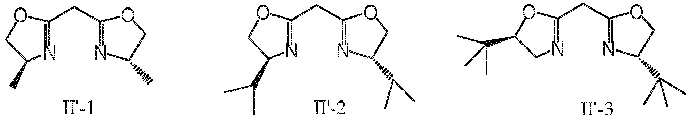

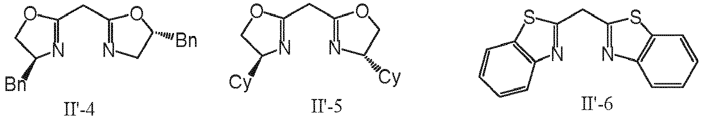

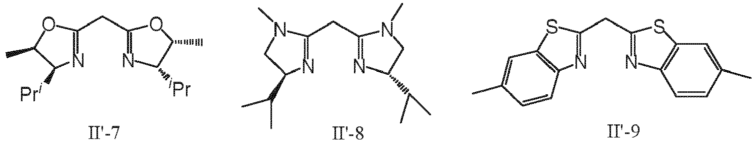

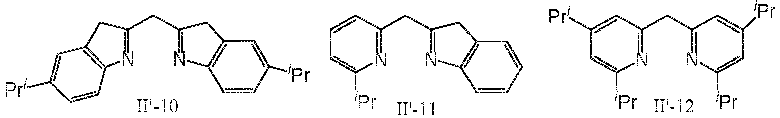

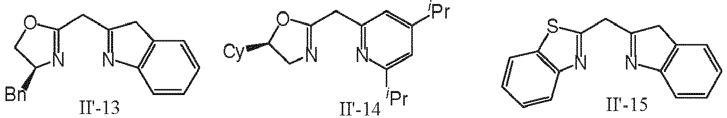

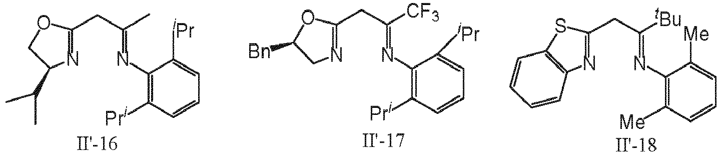

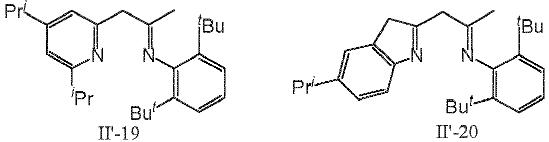

### Example 23 the synthesis of compound III'

The preparation method of compound III'-1:

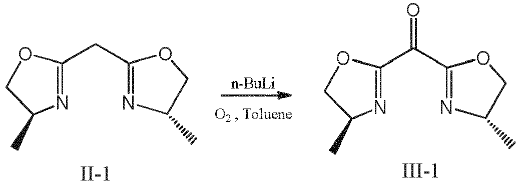

Compound II'-1 0.182g (1mmol) was added into 10mL of toluene. The mixture was stirred together for 10 min. Then it was added dropwise and slowly into 1.2 eq. of n-butyllithium in toluene. After the addition, the reaction was stirred at room temperature for 2h. The oxygen was pumped in after 2h and oxidizing was conducted. The product was yellow oil, which was isolated through column chromatography to obtain compound III'-1.

Other compounds, **III'-2** (yield 54%), **III'-3** (yield 43%), **III'-4** (yield 51%), **III'-5** (yield 36%), **III'-6** (yield 43%), **III'-7** (yield 56%), **III'-8** (yield 60%), **III'-9** (yield 55%), **III'-10** (yield 48%), **III'-11** (yield 56%), **III'-12** (yield 51%), **III'-13** (yield 45%), **III'-14** (yield 51%), **III'-15** (yield 44%), **III'-16** (yield 46%), **III'-17** (yield 41%), **III'-18** (yield 39%), **III'-19** (yield 55%), **III'-20** (yield 57%), were obtained by the above method for preparing compound III'-1.

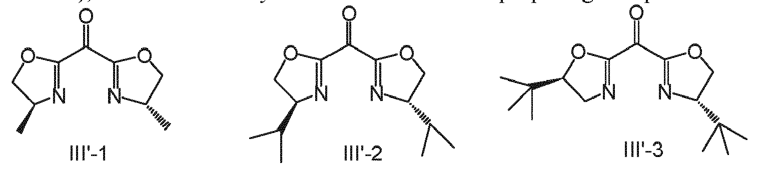

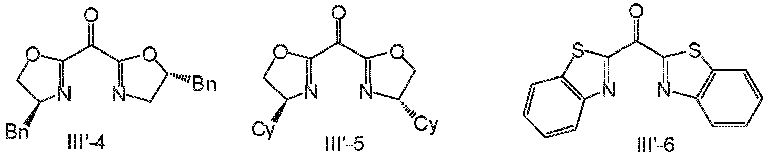

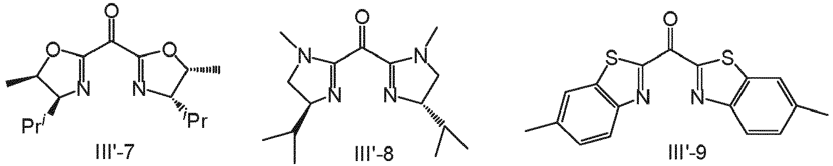

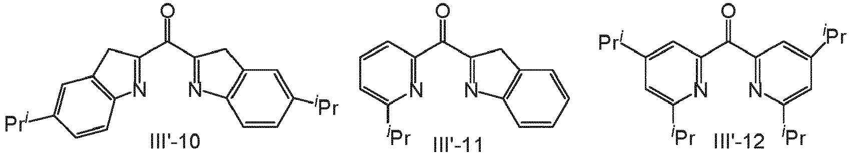

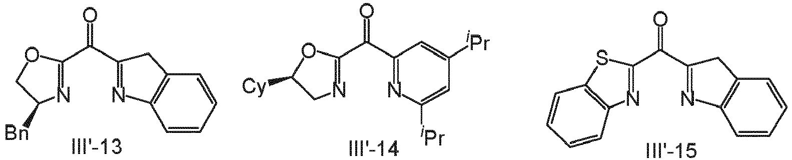

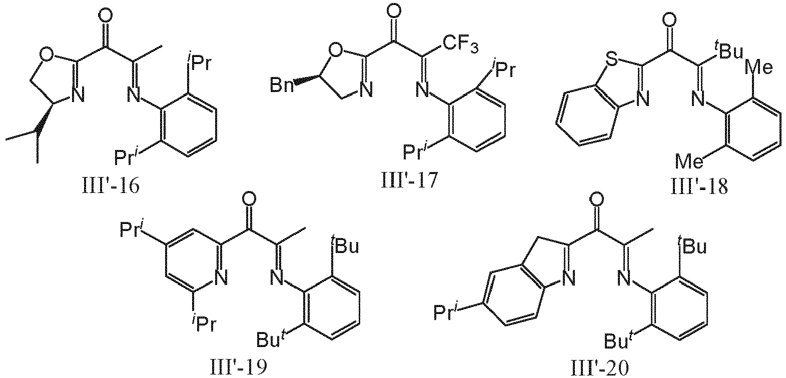

### Example 24 the synthesis of metal compound I'-1

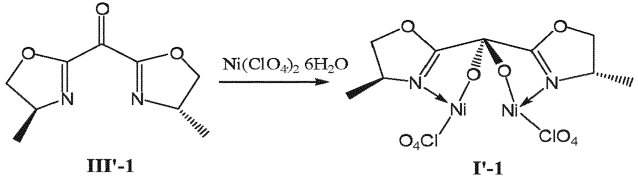

Compound III 0.2g (1mmol) and 1.2 eq. of Ni(ClO₄)₂·6H₂O were added into 20mL of toluene and reacted, thereby obtaining compound I 0.11g, yield 22%;
Elemental analysis: found (calculated): C: 20.25(20.45); H: 2.10(2.29); N:5.43(5.30).

### Example 25 the synthesis of coordination compound I'-2 to I'-20

The experimental method was basically the same as that of example 24, with the difference that compounds **III'-2** to **III'-20** were used to replace **I'-1** so as to obtain other coordination compounds, **I'-2** (yield 33%), **I'-3** (yield 26%), **I'-4** (yield 34%), **I'-5** (yield 45%), **I'-6** (yield 41%), **I'-7** (yield 34%), **I'-8** (yield 57%), **I'-9** (yield 47%), **I'-10** (yield 32%), **I'-11** (yield 50%), **I'-12** (yield 48%), **I'-13** (yield 52%), **I'-14** (yield 46%), **I'-15** (yield 45%), **I'-16** (yield 47%), **I'-17** (yield 41%), **I'-18** (yield 53%), **I'-19** (yield 44%), **I'-20** (yield 51%).

The respective correspondence between the raw materials and products were as shown in table below.

| Raw material | Product | Yield % |
|---|---|---|
| II'-2 | I'-2 | 33 |
| II'-3 | I'-3 | 26 |
| II'-4 | I'-4 | 34 |
| II'-5 | I'-5 | 41 |
| II-6 | I'-6 | 34 |
| II'-7 | I'-7 | 45 |
| II'-8 | I'-8 | 57 |
| II'-9 | I'-9 | 47 |
| II'-10 | I'-10 | 32 |
| II'-11 | I'-11 | 50 |
| II'-12 | I'-12 | 48 |
| II'-13 | I'-13 | 52 |
| II'-14 | I'-14 | 46 |
| II'-15 | I'-15 | 45 |
| II'-16 | I'-16 | 47 |
| II'-17 | I'-17 | 41 |
| II'-18 | I'-18 | 53 |
| II'-19 | I'-19 | 44 |
| II'-20 | I'-20 | 51 |

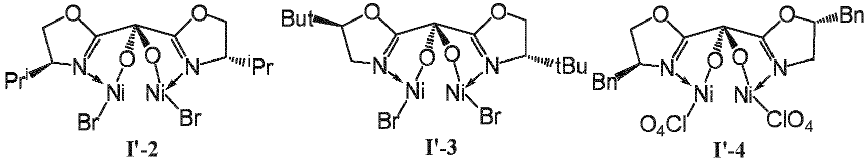

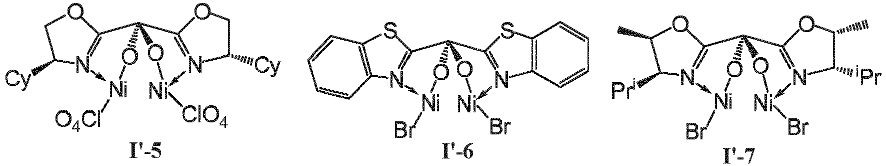

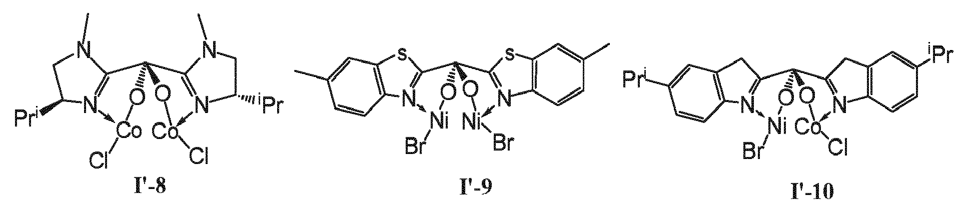

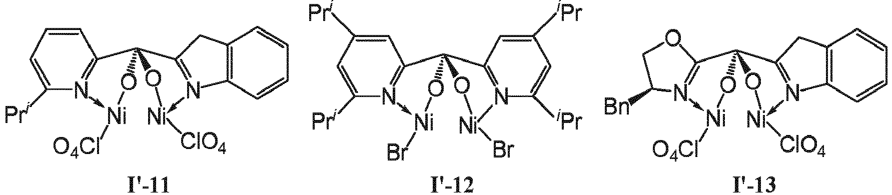

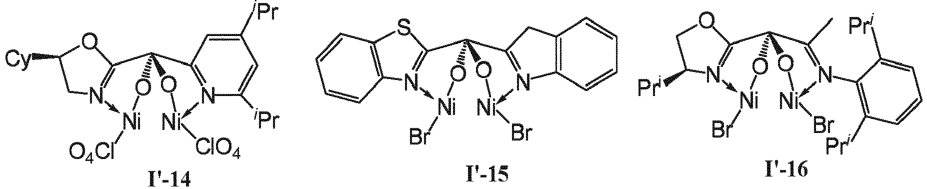

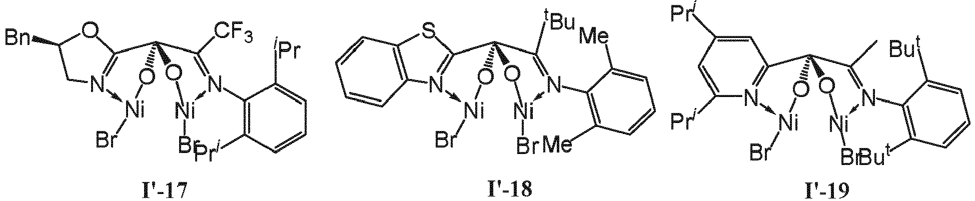

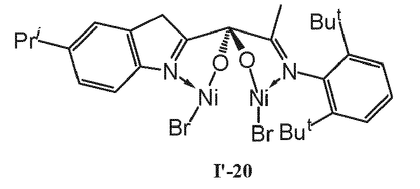

Some of the analysis data were as follows:
**I'-2** Elemental analysis: found (calculated): C: 28.81(28.62); H: 3.55(3.70); N:5.12(5.14).
**I'-3** Elemental analysis: found (calculated): C: 31.20(31.41); H: 4.01(4.22); N: 4.53(4.88).
**I'-4** Elemental analysis: found (calculated): C: 26.91(37.05); H: 2.87(2.96); N: 4.10(4.12).
**I'-5** Elemental analysis: found (calculated): C: 34.30(34.33); H: 4.12(4.25); N: 4.11(4.21).
**I'-6** Elemental analysis: found (calculated): C: 30.55(30.56); H: 1.52(1.37); N: 4.88(4.75).
**I'-7** Elemental analysis: found (calculated): C: 31.53(31.41); H: 4.20(4.22); N: 4.78(4.88).
**I'-8** Elemental analysis: found (calculated): C: 37.30(37.29); H: 5.56(5.42); N: 11.55(11.60).
**I'-9** Elemental analysis: found (calculated): C: 37.00(36.89); H: 2.21(2.19); N:5.24(5.06).
**I'-10** Elemental analysis: found (calculated): C: 46.90(46.55); H: 4.23(4.08); N: 4.67(4.72).
**I'-11** Elemental analysis: found (calculated): C: 34.21(34.22); H:2.56(2.70); N: 4.56(4.70).
**I'-12** Elemental analysis: found (calculated): C: 42.89(42.78); H: 5.12(5.00); N: 4.14(4.34)
**I'-13** Elemental analysis: found (calculated): C: 46.10(35.85); H: 2.34(2.53); N: 4.41(4.40).
**I'-14** Elemental analysis: found (calculated): C: 37.03(37.38); H: 4.55(4.48); N: 4.11(4.15).
**I'-15** Elemental analysis: found (calculated): C: 33.29(33.62); H: 2.00 (1.76); N: 4.89(4.90).
**I'-16** Elemental analysis: found (calculated): C: 39.77(39.68); H: 4.78(4.76); N: 4.55(4.41).
**I'-17** Elemental analysis: found (calculated): C: 40.71(40.70); H: 3.68(3.69); N: 4.00(3.80).
**I'-18** Elemental analysis: found (calculated): C: 39.12(39.19); H: 3.56(3.45); N: 4.55(4.35).
**I'-19** Elemental analysis: found (calculated): C: 47.23(47.11); H: 5.55(5.65); N: 4.12(3.92).
**I'-20** Elemental analysis: found (calculated): C: 47.57(47.38); H: 5.23(5.11); N: 4.10(3.95).

### Example 26 some of the catalysts in catalyzing ethylene polymerization

Under 0.1Mpa of ethylene atmosphere, 100mL of toluene and MMAO (molar ratio of MMAO to catalyst was 1500) were sequentially added into the evacuated and baked 250mL polymerization flask. The reaction was vigorously stirred, and then placed in an 30°C oil bath, and kept for a certain time, then a solution of catalyst **I'-1 to I'-2, I'-6 to I'-7, I'-10 to I'-13, I'-17 to I'-19** (5µmol) in toluene was added. After reacting for 10 minutes, the reaction was quenched with 5% of hydrochloric acid in ethanol. After the polymer was precipitated, filtered and washed, it was dried under vacuum at 50 °C to constant weight so as to obtain polyethylene. The homopolymerization results of ethylene were shown in the following table 13.

**Table 13 the results of catalyst I'-1 to I'-11 in catalyzing ethylene polymerization**

| Cat | W (g) | Activity (10⁵ g/mol·h·atm) | *M*_{w}/ 10⁴ g/mol | *M*_{w}/*M*ₙ | Branched/ 1000C |
|---|---|---|---|---|---|
| **I'-1** | 0.2134 | 2.6 | 10.1 | 2.1 | 21.1 |
| **I'-2** | 0.3112 | 3.7 | 13.1 | 2.3 | 23.1 |
| **I'-6** | 0.2910 | 3.5 | 11.4 | 2.5 | 21.2 |
| **I'-7** | 0.3511 | 4.2 | 12.1 | 2.7 | 19.1 |
| **I'-10** | 0.3791 | 4.5 | 9.9 | 2.5 | 23.0 |
| **I'-11** | 0.4113 | 4.9 | 14.2 | 2.4 | 15.9 |
| **I'-12** | 0.3121 | 3.7 | 21.1 | 2.0 | 28.1 |
| **I'-13** | 0.4512 | 5.4 | 18.2 | 3.1 | 23.1 |
| **I'-17** | 0.2511 | 3.0 | 21.1 | 3.5 | 21.3 |
| **I'-18** | 0.1811 | 2.2 | 18.1 | 4.1 | 24.1 |
| **I'-19** | 0.3512 | 4.2 | 23.1 | 2.3 | 19.1 |

| | | | | | |
|---|---|---|---|---|---|
| Notes: Cat: catalyst; W: the weight of polyethylene; Activity: the activity; *M*_{w}: The weight average molecular weight; *M*_{w}/*M*ₙ: molecular weight distribution. | | | | | |

The results had shown that the catalyst system could catalyze the polymerization of ethylene under ordinary pressures, of which the activities could reach up to 10⁵ g/mol·h, the molecular weight was 10⁵ g/mol, and the degree of branching was 19-28/1000 C.

### Example 27 some of the catalysts in catalyzing ethylene polymerization under high pressure

Under 1.0Mpa of ethylene atmosphere, 100mL of toluene and MMAO (molar ratio of MMAO to catalyst was 1500) were sequentially added into the evacuated and baked 350mL autoclave. The reaction was vigorously stirred, and then placed in an 30 °C oil bath, and kept for a certain time, then a solution of catalyst **I'-1**, **I'-2, I'-7, I'-10, I'-18** (5µmol for each) in toluene was added. After reacting for 10 minutes, the reaction was quenched with 5% of hydrochloric acid in ethanol. After the polymer was precipitated, filtered and washed; it was dried under vacuum at 50°C to constant weight so as to obtain polyvinyl. The polymerization results of ethylene were shown in the following table 14.

**Table 14 results of some catalysts in catalyzing ethylene polymerization under high pressure**

| Cat | W (g) | Activity (10⁵ g/mol·h·atm) | *M*_{w}/ 10⁴ g/mol | *M*_{w}/*M*ₙ | Branched/ 1000C |
|---|---|---|---|---|---|
| **I'-1** | 0.6712 | 0.81 | 88.9 | 2.7 | 12.4 |
| **I'-2** | 0.9712 | 1.2 | 98.2 | 2.5 | 10.1 |
| **I'-7** | 0.1011 | 0.12 | 67.8 | 2.7 | 12.1 |
| **I'-10** | 0.1231 | 0.15 | 101.2 | 2.9 | 9.4 |
| **I'-18** | 0.5212 | 0.63 | 112.3 | 4.3 | 8.7 |

| | | | | | |
|---|---|---|---|---|---|
| Notes: Cat: catalyst; W: the weight of copolymer; Activity: the activity; *M*_{w}: The weight average molecular weight; *M*_{w}/*M*ₙ: molecular weight distribution. | | | | | |

The results had shown that compared to the polymerization under ordinary pressure, the molecular weight of the obtained polymer could reach up to 10⁶ g/mol, while the degree of branching was reduced.

### Example 28 some of the catalysts in catalyzing propylene polymerization

Under 0.1Mpa of propylene atmosphere, 100mL of toluene and MMAO (molar ratio of MMAO to catalyst was 1500) were sequentially added into the evacuated and baked 250 m polymerization flask. The reaction was vigorously stirred, and then placed in a 30 °C oil bath, and kept for a certain time, then a solution of catalyst **I'-1-I'-2, I'-10-I'-12, I'-16-I'-20** (5µmol) in toluene was added. After reacting for 10 minutes, the reaction was quenched with 5% of hydrochloric acid in ethanol. After the polymer was precipitated, filtered and washed; it was dried under vacuum at 50 °C to constant weight so as to obtain polypropylene. The homopolymerization results of propylene were shown in the following table 15.

**Table 15 results of some catalysts in catalyzing propylene polymerization**

| Cat | W (g) | Activity (10⁵ g/mol·h·atm) | *M*_{w}/10⁴ g/mol | *M*_{w}/*M*ₙ |
|---|---|---|---|---|
| **I'-1** | 0.2511 | 3.0 | 32.3 | 2.4 |
| **I'-2** | 0.3312 | 4.0 | 31.2 | 2.2 |
| **I'-10** | 0.2912 | 3.5 | 29.3 | 2.5 |
| **I'-11** | 0.3912 | 4.7 | 28.5 | 2.1 |
| **I'-12** | 0.2834 | 3.4 | 26.2 | 2.1 |
| **I'-16** | 0.1923 | 2.3 | 25.1 | 3.4 |
| **I'-17** | 0.2413 | 2.9 | 31.1 | 4.1 |
| **I'-18** | 0.1612 | 1.9 | 33.3 | 3.8 |
| **I'-19** | 0.2918 | 3.5 | 27.1 | 2.4 |
| **I'-20** | 0.1651 | 2.0 | 30.1 | 4.5 |

| | | | | |
|---|---|---|---|---|
| Notes: Cat: catalyst; W: the weight of copolymer; Activity: the activity; *M*_{w}: The weight average molecular weight; *M*_{w}/*M*ₙ: molecular weight distribution. | | | | |

The results had shown that the catalyzing system could catalyze the polymerization of propylene in comparatively high activity.

### Example 29 some of the catalysts in catalyzing hexylene polymerization

100mL of toluene, 1-hexylene 10 mL and MMAO (molar ratio of MMAO to catalyst was 1500) were sequentially added into the evacuated and baked 250ml polymerization flask. The reaction was vigorously stirred, and then placed in a 30 °C oil bath, and kept for a certain time, then a solution of catalyst **I'-2-I'-3, I'-9-12, I'-16-I'-17** (5µmol) in toluene was added. After reacting for 10 minutes, the reaction was quenched with 5% of hydrochloric acid in ethanol. After the polymer was washed, it was vacuum dried at 50°C to constant weight so as to obtain polyhexylene. The homopolymerization results of hexylene were shown in the following table 16.

**Table 16 results of some catalysts in catalyzing hexylene polymerization**

| Cat | W (g) | Activity (10⁵ g/mol·h·atm) | *M*_{w}/ 10⁴ g/mol | *M*_{w}/*M*ₙ |
|---|---|---|---|---|
| **I'-2** | 0.098 | 1.2 | 12.2 | 2.1 |
| **I'-3** | 0.112 | 1.3 | 11.5 | 2.3 |
| **I'-9** | 0.123 | 1.5 | 9.9 | 2.6 |
| **I'-10** | 0.131 | 1.6 | 8.9 | 2.3 |
| **I'-11** | 0.087 | 1.0 | 10.5 | 2.1 |
| **I'-12** | 0.076 | 0.9 | 12.3 | 2.5 |
| **I'-16** | 0.121 | 1.5 | 8.2 | 4.1 |
| **I'-17** | 0.112 | 1.3 | 7.8 | 4.2 |

| | | | | |
|---|---|---|---|---|
| Notes: Cat: catalyst; W: the weight of copolymer; Activity: the activity; *M*_{w}: The weight average molecular weight; *M*_{w}/*M*ₙ: molecular weight distribution. | | | | |

The results had shown that the catalyzing system could well catalyze the polymerization of hexylene.

### Example 30 I'-2 catalyst in catalyzing the copolymerization of ethylene and olefin

Under 0.1Mpa of ethylene atmosphere, 100mL of toluene, 10 mmol of olefin and MMAO (molar ratio of MMAO to catalyst was 1500) were sequentially added into the evacuated and baked 250mL polymerization flask. The reaction was vigorously stirred, and then placed in an oil bath at 30 °C, and kept for a certain time, then a solution of catalyst **I'-2** (5µmol) in toluene was added. After reacting for 10 minutes, the reaction was quenched with 5% of hydrochloric acid in ethanol. After the polymer was washed, it was vacuum dried at 50°C to constant weight so as to obtain the polymer. The copolymerization results of ethylene and olefin were shown in the following table 17.

**Table 17 the results of I'-2 catalysts in catalyzing the copolymerization of ethylene and olefin**

| Olefin | W (g) | Activity (10⁵ g/mol·h·atm) | Incorp.(mol%) | *M*_{w}/ 10⁴ g/mol | *M*_{w}/*M*ₙ |
|---|---|---|---|---|---|
| 1-octadecene | 0.2341 | 2.8 | 14.3 | 24.2 | 2.0 |
| Norbornene | 0.2812 | 3.4 | 16.1 | 19.5 | 2.1 |
| Cyclopentene | 0.2311 | 2.8 | 18.1 | 24.0 | 2.6 |
| Cyclohexene | 0.3411 | 4.1 | 19.3 | 17.2 | 2.5 |
| Cyclopentadiene dimer | 0.3633 | 4.4 | 21.2 | 20.1 | 2.8 |

The results had shown that the catalyzing system could well catalyze the copolymerization of olefin.

### Example 31 some of the catalysts in catalyzing the copolymerization of ethylene and

### AlⁱBu₃ protected polar monomer

The copolymerization monomer used was polar monomer M03, which was treated with AlⁱBu₃ in accordance with the method T1 as described in the general methods.

The copolymerization reactions were conducted in accordance with the method described in general method, after polymerization was completed, 5% (by volume) hydrochloric acidified ethanol was used to precipitate the copolymer, which was washed by (100mLx3) of ethanol.

Other polymerization conditions, polymerization results and the representation results of the copolymers were listed in table 18 in detail.

**Table 18 ethvlene/M03 copolvmerization data**

| catalyst | The amount of catalyst (µmol) | The amount of MMAO (mmol) | The amount of **M03** (mmol) | Polymerization temperature (°C) | Polymerization activity (10⁵ g/mol·h·atm) | molar weight of copolymer *M_{w}* (10⁵g/mol) | Polar monomer insertion rate (mol-%) |
|---|---|---|---|---|---|---|---|
| **I'-2** | 5.0 | 5.0 | 10 | 30 | 3.4 | 3.4 | 3.2 |
| **I'-2** | 5.0 | 5.0 | 10 | 50 | 2.3 | 4.3 | 3.5 |
| **I'-2** | 10.0 | 10.0 | 12 | 10 | 3.1 | 4.2 | 4.1 |
| **I'-3** | 5.0 | 10.0 | 10 | 40 | 2.3 | 3.1 | 2.5 |
| **I'-5** | 6.0 | 12.0 | 10 | 30 | 2.1 | 3.0 | 2.2 |
| **I'-6** | 5.0 | 7.5 | 10 | 30 | 1.2 | 4.1 | 1.9 |
| **I'-10** | 5.0 | 7.5 | 10 | 40 | 5.5 | 6.1 | 2.9 |
| **I'-12** | 10 | 15.0 | 10 | 30 | 2.1 | 5.7 | 3.1 |
| **I'-14** | 5.0 | 10.0 | 10 | 40 | 2.2 | 3.9 | 2.1 |
| **I'-15** | 5.0 | 10.0 | 10 | 40 | 1.3 | 3.8 | 1.9 |
| **I'-18** | 5.0 | 7.5 | 10 | 30 | 1.9 | 4.2 | 2.3 |
| **I'-19** | 5.0 | 7.5 | 10 | 30 | 3.2 | 3.2 | 1.8 |

The conclusion could be drawn from the data in above table that: the catalysts could well catalyze the copolymerization of ethylene and the AlⁱBu₃ protected monomer **M03** under ordinary pressure, of which the activity could reach up to 10⁵ g/mol·h, the molecular weight was 10⁵ g/mol, and the polar monomer insertion rate was 1.8mol-4.1mol%.

### Example 32 some of the catalysts in catalyzing the copolymerization of ethylene and TBS or TMS protected polar monomer

The copolymerization monomers as used were polar monomer **M18, 19, 20, 21, 22, and** 23.

The copolymerization reactions were conducted in accordance with the method as described in general method, the amount of catalyst used was 5umol, and the co-catalyst was MMAO, the polymerization time was 10 min. After the polymerization was completed, 5%(by volume) of hydrochloric acid in ethanol was used to precipitate the copolymer, which was washed by (100mL×3) of ethanol.

Other polymerization conditions, polymerization results and the representation results of the copolymer were listed in table 19 in detail

**Table 19 ethylene/polar monomer copolymerization data**

| catalyst | polar monomer | Amount of polar monomer (mmol) | temperat ure (°C) | Amount of MMAO (mmol) | Activity (10⁵ g/ mol·h·atm) | Polar monomer insertion rate (mol%) |
|---|---|---|---|---|---|---|
| **I'-2** | **M20** | 10 | 30 | 5.0 | 6.5 | 3.4 |
| **I'-2** | **M18** | 10 | 40 | 7.5 | 5.2 | 1.5 |
| **I'-2** | **M19** | 10 | 40 | 7.5 | 5.9 | 2.3 |
| **I'-2** | **M21** | 10 | 40 | 7.5 | 3.2 | 1.3 |
| **I'-2** | **M22** | 10 | 30 | 7.5 | 4.5 | 2.3 |
| **I'-2** | **M23** | 10 | 30 | 5.0 | 7.1 | 3.4 |
| **I'-6** | **M20** | 15 | 30 | 5.0 | 7.9 | 2.3 |
| **I'-12** | **M20** | 15 | 30 | 5.0 | 5.8 | 2.7 |
| **I'-16** | **M20** | 20 | 40 | 5.0 | 6.8 | 4.5 |
| **I'-20** | **M20** | 20 | 40 | 5.0 | 3.4 | 5.1 |

The conclusion could be drawn from the data in above table that: this type of catalysts could well catalyze the copolymerization of ethylene and the TBS or TMS protected monomer under ordinary pressure, of which the activity could reach up to 10⁵ g/mol·h, the molecular weight was 10⁵ g/mol, and the polar monomer insertion rate was 1.3mol-5.1mol%.

### Example 33 some of the catalysts in catalyzing the copolymerization of ethylene and co-catalyst protected polar monomer

The polar monomer was treated in accordance with the method T2 as described in the general methods.

The copolymerization reactions were conducted in accordance with the method described in general method, the amount of main catalyst used was 5µmol, and the co-catalyst was MMAO, the amount of the polar monomer used was 10mmol. Before the polymerization, the co-catalyst and the polar monomer was mixed and stirred to react for 3h, the polymerization temperature was 30°C, and the polymerization time was 10 min. After the polymerization was completed, 5%(by volume) of hydrochloric acid in ethanol was used to precipitate the copolymer, which was washed with ethanol (100mL×3).

Other polymerization conditions, polymerization results and the representation results of the copolymer were listed in table 20 in detail.

**Table 20 ethylene/polar monomer copolymerization data**

| catalyst | polar monomer | The amount of co-catalyst (mmol) | Polymerization activity (10⁵g/mol·h·atm) | Molar weight of copolymer *M_{w}* (10⁵g/mol) | Polar monomer insertion rate (mol%) |
|---|---|---|---|---|---|
| **I'-2** | **M03** | 15.0 | 5.2 | 3.4 | 8.1 |
| **I'-2** | **M05** | 15.0 | 4.7 | 3.2 | 6.7 |

The conclusion could be drawn from the data in above table that: the catalysts could well catalyze the copolymerization of ethylene and various monomers under ordinary pressure after the monomers were pre-treated with MMAO.

### Example 34 some of the catalysts in catalyzing the copolymerization of ethylene and unprotected polar monomer

The copolymerization monomers were used directly to copolymerize with ethylene without being protected.

The copolymerization reactions were conducted in accordance with the method as described in general method, the co-catalyst was MMAO, and the polymerization temperature was 30°C. The MMAO was added into 50mL of solvent, and the polar monomer was added after 5 min. The mixture was stirred together for 10min, after that, the pre-dissolved catalyst solution was added. The polymerization was conducted for 10 min. After the polymerization was completed, 5%(by volume) of hydrochloric acid in ethanol was used to precipitate the copolymer, which was washed with ethanol (100mLx3).

Other polymerization conditions, polymerization results and the representation results of the copolymer were listed in table 21 in detail.

**Table 21 ethylene/polar monomer copolymerization data**

| catalyst | Amount of catalyst (µmol) | Amount of MMAO (mmol) | polar monomer | Amount of monomer (mmol) | Polymerization temperature (°C) | Polymerization activity (10⁵g/mol·h·atm) | Polar monomer insertion rate (mol-%) |
|---|---|---|---|---|---|---|---|
| **I'-2** | 5.0 | 25.0 | **M03** | 20 | 40 | 3.4 | 11.9 |
| **I'-2** | 5.0 | 25.0 | **M02** | 20 | 40 | 5.2 | 12.9 |
| **I'-2** | 40.0 | 120.0 | **M01** | 30 | 40 | 0.2 | 1.2 |
| **I'-2** | 10.0 | 30.0 | **M07** | 10 | 30 | 7.9 | 5.6 |
| **I'-3** | 10.0 | 30.0 | **M03** | 10 | 30 | 6.7 | 7.8 |
| **I'-7** | 10.0 | 30.0 | **M07** | 10 | 30 | 1.3 | 3.4 |
| **I'-10** | 5.0 | 15.0 | **M04** | 20 | 40 | 1.9 | 7.8 |
| **I'-11** | 5.0 | 15.0 | **M03** | 20 | 40 | 2.3 | 5.6 |
| **I'-15** | 5.0 | 15.0 | **M17** | 5 | 40 | 2.5 | 2.3 |
| **I'-16** | 5.0 | 15.0 | **M17** | 5 | 30 | 3.2 | 2.2 |
| **I'-20** | 5.0 | 15.0 | **M03** | 5 | 30 | 0.4 | 1.9 |

The conclusion could be drawn from the data in above table that: the catalysts could well catalyze the copolymerization of ethylene and unprotected monomers under ordinary pressure, while compared with the monomers protected the activity was slightly lower; the insertion of some monomers that were usually difficult to copolymerize could be achieved, such as M01.

### Example 35 some of the catalysts in catalyzing the copolymerization of propylene and polar monomer

The copolymerization monomers were used directly to copolymerize with propylene without being protected.

The copolymerization reactions were conducted in accordance with the method as described in general method, the co-catalyst was MMAO, and the polymerization temperature was 30°C. MMAO was added into 50mL of solvent, and the polar monomer was added after 5 min. The mixture was stirred together for 10min. After that, the pre-dissolved catalyst solution was added. The polymerization was conducted for 10 min. After the polymerization was completed, 5% (by volume) of hydrochloric acid in ethanol was used to precipitate the copolymer, which was washed with ethanol (100mLx3).

Other polymerization conditions, polymerization results and the representation results of the copolymer were listed in table 22 in detail.

**Table 22 propylene/polar monomer copolymerization data**

| catalyst | Amount of catalyst (µmol) | Amount of MMAO (mmol) | polar monomer | Amount of monomer (mmol) | Polymerization temperature (°C) | Polymerization activity (10⁵g/mol·h·atm) | Polar monomer insertion rate (mol-%) |
|---|---|---|---|---|---|---|---|
| **I'-2** | 5.0 | 25.0 | **M03** | 10 | 30 | 7.8 | 7.1 |
| **I'-2** | 5.0 | 25.0 | **M05** | 10 | 40 | 4.7 | 7.3 |
| **I'-2** | 10.0 | 50.0 | **M11** | 10 | 30 | 2.7 | 4.2 |
| **I'-3** | 5.0 | 15.0 | **M03** | 20 | 30 | 2.4 | 4.2 |
| **I'-6** | 5.0 | 25.0 | **M03** | 10 | 30 | 2.3 | 6.2 |
| **I'-12** | 5.0 | 25.0 | **M05** | 10 | 40 | 3.3 | 4.5 |
| **I'-13** | 5.0 | 25.0 | **M11** | 10 | 40 | 2.4 | 0.8 |
| **I'-16** | 5.0 | 25.0 | **M07** | 10 | 45 | 4.5 | 6.8 |
| **I'-20** | 5.0 | 25.0 | **M07** | 10 | 45 | 1.1 | 2.3 |

The results had shown that this type of catalysts could well catalyze the copolymerization of propylene and unprotected monomers under ordinary pressure.

### Example 36 some of the catalysts in catalyzing the copolymerization of 1-butylene and polar monomer

The copolymerization monomers were used directly to copolymerize with 1-butylene without being protected.

The copolymerization reactions were conducted in accordance with the method as described in general method, the co-catalyst was MMAO, and the polymerization temperature was 30°C. MMAO was added into 50mL of solvent, and the polar monomer was added after 5 min. The mixture was stirred together for 10min, after that, the pre-dissolved catalyst solution was added. The polymerization was conducted for 10 min. After the polymerization was completed, 5% (by volume) of hydrochloric acid in ethanol was used to precipitate the copolymer, which was washed with ethanol (100mL×3).

Other polymerization conditions, polymerization results and the representation results of the copolymer were listed in table 23 in detail.

**Table 23 1-butylene/polar monomer copolymerization data**

| catalyst | Amount of catalyst (µmol) | Amount of MMAO (mmol) | polar monomer | Amount of monomer (mmol) | Polymerization temperature (°C) | Polymerization activity (10⁵g/mol·h·atm) | Polar monomer insertion rate (mol%) |
|---|---|---|---|---|---|---|---|
| **I'-2** | 5.0 | 25.0 | **M03** | 10 | 40 | 2.3 | 4.8 |
| **I'-2** | 5.0 | 25.0 | **M02** | 10 | 40 | 1.3 | 1.5 |
| **I'-2** | 20.0 | 80.0 | **M01** | 30 | 30 | 0.1 | 0.9 |
| **I'-3** | 5.0 | 15.0 | **M03** | 5 | 30 | 1.6 | 2.4 |
| **I'-6** | 5.0 | 25.0 | **M03** | 10 | 30 | 2.1 | 4.3 |
| **I'-10** | 5.0 | 25.0 | **M05** | 10 | 30 | 0.9 | 2.3 |
| **I'-12** | 5.0 | 25.0 | **M11** | 10 | 30 | 0.4 | 0.8 |
| **I'-20** | 5.0 | 15.0 | **M03** | 15 | 40 | 1.9 | 5.1 |

The results had shown that this type of catalysts could well catalyze the copolymerization of 1-butylene and unprotected monomers under ordinary pressure.

### Example 37 some of the catalysts in catalyzing the polymerization of ethylene and polar monomer under high pressure

Under 1.0Mpa of ethylene atmosphere, 100 mL of toluene, MMAO (molar ratio of MMAO to catalyst was 3000) were sequentially added into the evacuated and baked 350mL autoclave. The reaction was vigorously stirred, and then placed in a 30 °C oil bath, and kept for a certain time, then a solution of catalyst **I'-2, I'-3, I'-10, I'-13, I'-20** (5µmol for each) in toluene was added. After reacting for 10 minutes, the reaction was quenched with 5% of hydrochloric acid in ethanol. After the polymer was precipitated, filtered and washed, it was dried under vacuum at 50°C to constant weight so as to obtain polyvinyl. The polymerization results of ethylene were shown in the following table.

**Table 24 the results of some of the catalysts in catalyzing ethylene polymerization under high pressure**

| Cat | polar monomer | Activity (10⁵ g/mol·h·atm) | *M*_{w}/ 10⁴ g/mol | *M*_{w}/*M*ₙ | Polar monomer insertion rate (mol%) |
|---|---|---|---|---|---|
| **I'-2** | **M03** | 1.2 | 103.0 | 2.2 | 2.4 |
| **I'-3** | **M04** | 0.7 | 78.1 | 2.9 | 1.9 |
| **I'-10** | **M05** | 1.1 | 98.9 | 3.0 | 2.3 |
| **I'-13** | **M07** | 1.3 | 101.0 | 2.8 | 1.2 |
| **I'-20** | **M11** | 0.9 | 89.9 | 4.5 | 2.3 |

The results had shown that compared to the ordinary pressure polymerization, the molecular weight of the obtained polymer could reach up to 10⁶ g/mol, while the insertion rate was obviously reduced.

All literatures mentioned in the present application are incorporated herein by reference, as though each one is individually incorporated by reference. Additionally, it should be understood that after reading the above teachings, those skilled in the art can make various changes and modifications to the present invention. These equivalents also fall within the scope defined by the appended claims.

## Claims

1. A compound of formula I: 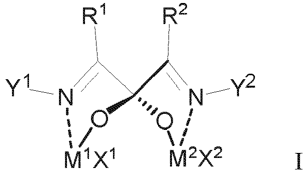 wherein:
------ is a coordination bond;
each of R¹ and R² is independently selected from the group consisting of: hydrogen, substituted or unsubstituted C1-C10 alkyl, substituted or unsubstituted aryl, substituted or unsubstituted C3-C10 cycloalkyl;
wherein the aryl refers to phenyl, naphthyl, fluorenyl or anthryl; wherein the "substituted" means one or more hydrogen atoms on the group are substituted with substituents selected from the group consisting of: OR³, SR⁴, NR⁵R⁶, PR⁷R⁸ or P(O)R⁹R¹⁰, SiR¹², C1-C4 alkyl, halogen, and phenyl;
or R¹ and R² together form substituted or unsubstituted C1-C10 alkylene;
each of M¹ and M² is independently selected from the group consisting of: Fe, Co, Ni, Pd, Pt, and the combinations thereof;
each of X¹ and X² is independently selected from the group consisting of: halogen, CIO₄, substituted or unsubstituted C1-C10 alkyl, substituted or unsubstituted C3-C10 cycloalkyl, substituted or unsubstituted phenyl, substituted or unsubstituted benzyl; wherein the "substituted" means one or more hydrogen atoms on the group are substituted with substituents selected from the group consisting of: OR³, SR⁴, NR⁵R⁶, PR⁷R⁸ or P(O)R⁹R¹⁰, SiR¹², C1-C4 alkyl, halogen, phenyl, and -CF₃;
each of Y¹ and Y² is independently selected from the group consisting of: substituted or unsubstituted C1-C30 alkyl, substituted or unsubstituted aryl, substituted or unsubstituted heteroaryl; wherein the aryl refers to phenyl, naphthyl, fluorenyl or anthryl; the heteroaryl refers to furyl, thienyl, pyrrolyl, pyridinyl or pyranyl; the "substituted" means one or more hydrogen atoms on the group are substituted with substituents selected from the group consisting of: OR³, SR⁴, NR⁵R⁶, PR⁷R⁸ or P(O)R⁹R¹⁰, SiR¹², C1-C4 alkyl, halogen, and phenyl;
wherein R³, R⁴, R⁵, R⁶, R⁷, R⁸, R⁹, R¹⁰, and R¹² are independently selected from the group consisting of: C1-C10 alkyl, phenyl, C5-C16 heteroaryl; wherein 0 to 5 hydrogen groups on phenyl or heteroaryl can be substituted by the following substituent groups: halogen, -CF₃, OR¹³, C1-C4 alkyl; R¹³ is C1-C4 alkyl;
or Y¹, R¹ and the joint N=C together form substituted or unsubstituted 5-12 member heterocyclic ring which comprises 1-3 heteroatoms; wherein the heteroatoms are selected from the group consisting of: N, S, O and P;
wherein the "substituted" means one or more hydrogen atoms on the group are substituted with substituents selected from the group consisting of: hydrogen, substituted or unsubstituted C1-C10 alkyl, substituted or unsubstituted aryl, and substituted or unsubstituted C3-C10 cycloalkyl; the aryl refers to phenyl, naphthyl, fluorenyl or anthryl; the heteroaryl refers to furyl, thienyl, pyrrolyl, pyridinyl or pyranyl;
and/or Y², R² and the joint N=C together form substituted or unsubstituted 5-12 member heterocyclic ring which comprises 1-3 heteroatoms; wherein the heteroatoms are selected from the group consisting of: N, S, O and P; the "substituted" means one or more hydrogen atoms on the group are substituted with substituents selected from the group consisting of: hydrogen, substituted or unsubstituted C1-C10 alkyl, substituted or unsubstituted aryl, and substituted or unsubstituted C3-C10 cycloalkyl; wherein the aryl refers to phenyl, naphthyl, fluorenyl or anthryl; the heteroaryl refers to furyl, thienyl, pyrrolyl, pyridinyl or pyranyl;
and the compound of formula I is charge balanced.

2. The compound of claim 1, wherein,
each of R¹ and R² is independently selected from the group consisting of: hydrogen, substituted or unsubstituted C1-C10 alkyl, substituted or unsubstituted aryl, and substituted or unsubstituted C3-C10 cycloalkyl; or R¹ and R² together form substituted or unsubstituted C1-C10 alkylene;
each of Y¹ and Y² is independently selected from the group consisting of: substituted or unsubstituted C1-C30 alkyl, substituted or unsubstituted aryl, and substituted or unsubstituted heteroaryl; wherein the aryl refers to phenyl, naphthyl, fluorenyl or anthryl; the heteroaryl refers to furyl, thienyl, pyrrolyl, pyridinyl or pyranyl; the "substituted" means one or more hydrogen atoms on the group are substituted with substituents selected from the group consisting of: OR³, SR⁴, NR⁵R⁶, PR⁷R⁸ or P(O)R⁹R¹⁰, SiR¹², C1-C4 alkyl, halogen, and phenyl.

3. The compound of claim 1, wherein, Y¹, R¹ and the joint N=C together form substituted or unsubstituted 5-12 member heterocyclic ring which comprises 1-3 heteroatoms; and/or Y², R² and the joint N=C together form substituted or unsubstituted 5-12 member heterocyclic ring which comprises 1-3 heteroatoms.

4. A method for preparing a compound of claim 2, wherein, it comprises a step of: 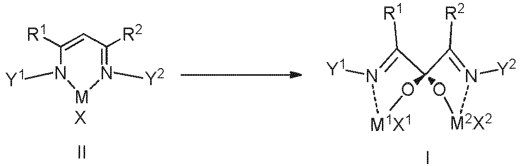 reacting a compound of formula II or a complex thereof with an oxidizing reagent in an inert solvent, thereby obtaining a compound of formula I;
wherein M is selected from the group consisting of: Fe, Co, Ni, Pd, Pt, and the combinations thereof;
X is selected from the group consisting of: halogen, C1-C10 alkyl, substituted or unsubstituted C3-C10 cycloalkyl, substituted or unsubstituted phenyl, and substituted or unsubstituted benzyl;
wherein the "substituted" means one or more hydrogen atoms on the group are substituted with substituents selected from the group consisting of: OR³, SR⁴, NR⁵R⁶, PR⁷R⁸ or P(O)R⁹R¹⁰, SiR¹², C1-C4 alkyl, halogen, phenyl, and -CF₃;
while the other groups are defined as in claim 1.

5. A method for preparing a compound of claim 3, wherein, it comprises a following step: 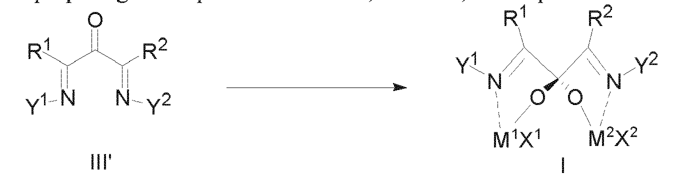
in an inert solvent, reacting a compound of formula III' with a reagent selected from the group consisting of: M¹X¹, M¹X¹·B, M²X², M²X²·B, and the combinations thereof, or the hydrates thereof, thereby obtaining a compound of formula I;
wherein the M₁X₁·B or M₂X₂·B are respectively a metallic compound formed by M₁X₁ or M₂X₂ with a coordinating solvent B; preferably, the coordinating solvent B is selected from the group consisting of: DME(dimethoxyethane), THF(tetrahydrofuran), acetonitrile, ethanol, ethylene glycol, methanol, AcAc(acetylacetonyl), DMF, and the combinations thereof;
while the other groups are defined as in claim 1.

6. A compound of formula II, or the complexes thereof: 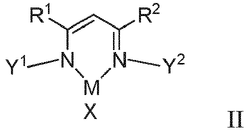 wherein, the groups are defined as in claim 2; and
when R¹ and R² are both -CH₃ or -CF₃, then Y¹ and Y² are not simultaneously 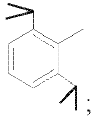 the complexes of compound of formula II are complexes formed by the compound of formula II and a ligand selected from the group consisting of: triphenylphosphine, acetonitrile, tetrahydrofuran, and C4-C16 heteroarene; or ligands which comprises groups selected from the group: OR³, SR⁴, NR⁵R⁶, PR⁷R⁸, and the combinations thereof.

7. A method for preparing a compound of claim 6, wherein, it comprises a following step: 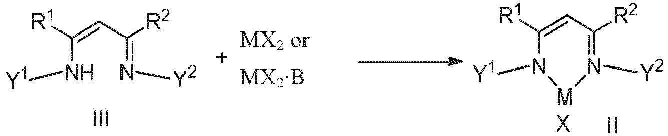
in an inert solvent, reacting a compound of formula III with MX₂ or MX₂-B, thereby obtaining a compound of formula II or a complex thereof;
wherein MX₂·B is a metallic compound formed by MX₂ and a coordinating solvent B;
B is coordinating solvent;
the complex of compound of formula II is a complex formed by compound of formula II and a ligand selected from the group consisting of: triphenylphosphine, acetonitrile, tetrahydrofuran, and C4-C16 heteroarene; or a ligand which comprises groups selected from the group: OR³, SR⁴, NR⁵R⁶, PR⁷R⁸, and the combinations thereof;
wherein the groups are defined as in claim 2.

8. A compound of formula III': 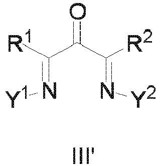 wherein, the groups are defined as in claim 5.

9. A method for preparing a compound of formula III' of claim 8, wherein, it comprises a following step: 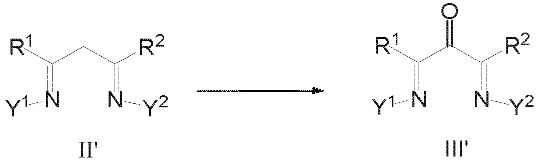 in an inert solvent and in presence of an alkali, reacting a compound of formula II' with an oxidizing reagent, thereby obtaining a compound of formula III'.

10. A compound of formula II': 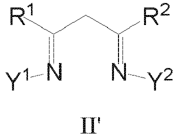 wherein, the groups are defined as in claim 5.

11. A method for preparing a compound of formula II' of claim 10, wherein, it comprises the following steps:
(a1) in an inert solvent, reacting a compound of formula II'c with a compound of formula II'b, thereby obtaining a compound of formula II'a; 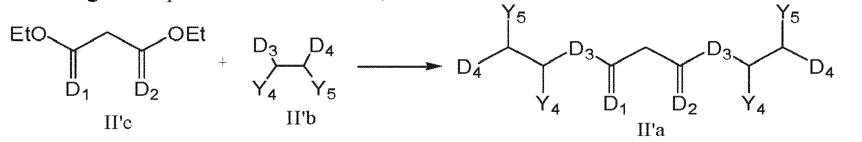
(a2) in an inert solvent, reacting a compound of formula II'a, thereby obtaining a compound of formula II'f; 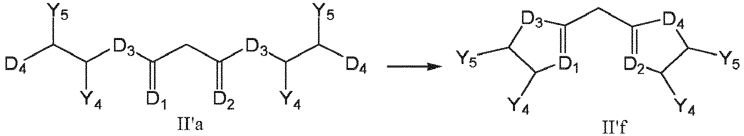
and step (a3) preparing a compound of formula II from a compound of formula II'f;
or it comprises the following steps:
(b1) in an inert solvent, reacting a compound of formula II'd with a compound of formula II'b, thereby obtaining a compound of formula II'e; 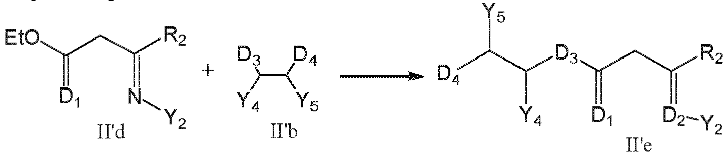
(b2) reacting a compound of formula II'e, thereby obtaining a compound of formula II'g; 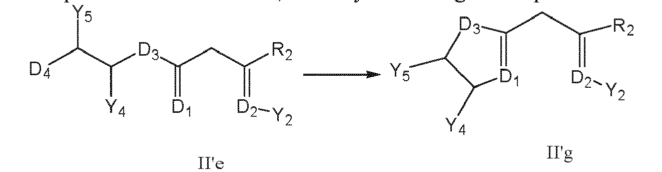
and step (b3) preparing a compound of formula II from a compound of formula II'g;
wherein D₁, D₂, D₃, and D₄ are independently selected from the group consisting of: N, S, O, P, NH, and PH;
Y₄, and Y₅ are independently selected from the group consisting of: substituted or unsubstituted C1-C30 alkyl, substituted or unsubstituted aryl, and substituted or unsubstituted heteroaryl; wherein the aryl refers to phenyl, naphthyl, fluorenyl or anthryl; the heteroaryl refers to furyl, thienyl, pyrrolyl, pyridinyl or pyranyl; the "substituted" means one or more hydrogen atoms on the group are substituted with substituents selected from the group consisting of: OR³, SR⁴, NR⁵R⁶, PR⁷R⁸ or P(O)R⁹R¹⁰, SiR¹², C1-C4 alkyl, halogen, and phenyl;
or Y₄, Y₅ and the joint two carbon atoms together form substituted or unsubstituted phenyl; or together form substituted or unsubstituted 5-7 member heterocyclic aryl; wherein the "substituted" means one or more hydrogen atoms on the group are substituted with substituents selected from the group consisting of: hydrogen, substituted or unsubstituted C1-C10 alkyl, substituted or unsubstituted aryl, and substituted or unsubstituted C3-C10 cycloalkyl;
while the other groups are defined as in claim 3.

12. Use of the compound of any of claims 6, 8 or 10, or the complexes thereof, wherein the compound is used (a) as olefin polymerization catalysts; (b) in preparation of olefin polymerization catalyst composition; or (c) in preparation of a compound of formula I in claim 1.

13. An catalyst composition for olefin polymerization, wherein, it comprises a catalytically effective amount of compound of formula I in claim 1, compound of formula II or the complexes thereof in claim 4, compound of formula II' or the complexes thereof in claim 6, and/or compound of formula III' or the complexes thereof in claim 8.

14. The catalyst composition of claim 13, wherein it further comprises an effective amount of cocatalyst.

15. A method for preparing olefin polymerization, wherein it comprises: polymerizing an olefin in presence of a catalytically effective amount of catalyst thereby obtaining olefin polymers;
wherein the catalyst is selected from the group consisting of: compound of claim 1, compound of formula II or the complexes thereof in claim 6, the catalyst composition of claim 13, and the combinations thereof.

16. An olefin polymer prepared by the method of claim 15.

17. Use of compound of claim 1, wherein the compound is used (a) as a catalyst used in catalyzing olefin polymerization reaction; or (b) in preparation of olefin polymerization catalyst composition.

18. The use of claim 17, wherein the olefin polymerization reaction is ethylene homopolymerization, propylene homopolymerization or the copolymerization between C2-C4 olefins and C2-C18 α-olefin or cycloolefins; wherein the C2-C18 α- olefins or cycloolefins do not comprise any polar groups.

19. The use of claim 17, wherein, the polymerization reaction is the copolymerization reaction between C2-C4 olefins and polar monomers.

20. The use of claim 19, wherein, the polar monomers are C₃-C₅₀ 1-olefin derivatives or cycloolefin derivatives comprising a polar group selected from the group consisting of: carbonyl group, hydroxyl group, carboxyl group, COOR³² ester group, OR³³ alkoxy group, NR³⁴R³⁵ amino group, CONR³⁴R³⁵ acylamino group, SR³⁶ thioether and SeR³⁷ selenide; wherein R³² or R³³ is C₁₋₁₀ hydrocarbyl; R³⁴, R³⁵, R³⁶ or R³⁷ are hydrogen or C₁₋₁₀ hydrocarbyl.
